# EUROPEAN PATENT APPLICATION

(11) **EP 4 825 839 A1**
(43) Date of publication of application: **21.10.2026**
(21) Application number: 24902999.2
(22) Date of filing: 13.12.2024
(51) Int. Cl.: C07C 323/12, C07C 319/22, C07D 295/13, C07D 311/58, A61K 45/00, A61K 47/54, A61K 47/69

(54) **DEGRADABLE CATIONIC LIPID CONTAINING DISULFIDE BOND AND USE THEREOF**

(30) Priority: 15.12.2023 CN 202311726611; 28.03.2024 CN 202410365737
(71) Applicant: Xiamen Sinopeg Biotech Co., Ltd, Xiamen, Fujian 361100 (CN)
(72) Inventor: LIN, Minggui, Xiamen, Fujian 361100 (CN); ZHU, Qi, Xiamen, Fujian 361100 (CN); WANG, Ailan, Xiamen, Fujian 361100 (CN); CHEN, Dandan, Xiamen, Fujian 361100 (CN); WEI, Guohua, Xiamen, Fujian 361100 (CN); WENG, Wengui, Xiamen, Fujian 361100 (CN); LIU, Chao, Xiamen, Fujian 361100 (CN); LIN, Sheng, Xiamen, Fujian 361100 (CN)
(74) Representative: Algemeen Octrooi- en Merkenbureau B.V.
(86) International application number: PCT/CN2024/139417
(87) International publication number: WO 2025/124584

(57) **Abstract**

The present application relates to a cationic lipid containing a disulfide bond of Formula (1), wherein, -S-S- is a disulfide bond; L₁ and L₂ are each independently a degradable divalent linking group, X₁ and X₂ are each independently an acyclic divalent linking group containing a tertiary amine group; G₁ and G₂ are each independently a linking bond or a trivalent branching group; when G₁ is a linking bond, k1 is 1; when G₁ is a trivalent branching group, k1 is 2; when G₂ is a linking bond, k1 is 1; when G₂ is a trivalent branching group, k2 is 2; R₁ and R₂ are each independently a substituted or unsubstituted C₅₋₃₀ hydrocarbon group or C₅₋₃₀ hydrocarbon derivative residue. The lipid composition prepared with the cationic lipid of the present application, under the premise of good ability to complex with nucleic acid drugs, stability in the systemic circulation, and low toxicity, can more effectively exert the therapeutic effect of the LNP-nucleic acid pharmaceutical composition formulation, thereby improving immunological or therapeutic outcomes.

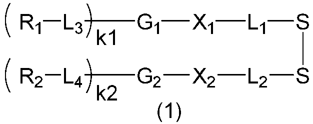

## Description

### TECHNICAL FIELD

The present invention pertains to the field of drug delivery, specifically relates to a cationic lipid as a pharmaceutical carrier, particularly relates to a degradable cationic lipid containing a disulfide bond, and further relates to a lipid composition and a lipid pharmaceutical composition, both comprising the cationic lipid, and formulations and applications thereof.

### BACKGROUND

Lipid nanoparticles (LNPs) are among the most prominent delivery technologies at present. The lipid formulation of LNPs can comprise various lipids, including cationic lipids, zwitterionic lipids, neutral lipids, steroid lipids, and PEGylated lipids. As a key component in LNP-nucleic acid pharmaceutical composition formulations, cationic lipids can form complexes with negatively charged nucleic acid drugs through electrostatic interactions. Cationic lipids include cationizable lipids and permanent cationic lipids. Wherein, cationizable lipids remain electrically neutral under physiological conditions, thereby minimizing interference with biological membranes during systemic circulation and exhibiting lower toxicity compared with permanent cationic lipids. After cellular uptake of LNPs, cationizable lipids are converted into positively charged molecules under the acidic conditions of the endosomal lumen, enhancing membrane permeability and promoting the endosomal escape of lipid pharmaceutical compositions. Additionally, the reorganization of lipid components facilitates the release of drug molecules from LNPs, thereby improving therapeutic efficacy.

The specific structure of cationic lipids has a significant impact on the nucleic acid drug delivery efficiency of LNPs. One of the key factors affecting delivery efficiency is whether the LNPs can effectively release the drug within cells. The concentration of glutathione (GSH) inside cells is significantly higher than that outside cells, which is one of the main contributors to the reductive environment within cells. In this regard, some existing technologies use a disulfide bond to connect two lipid moieties, utilizing the cleavage of disulfide bond in the intracellular reductive environment to destabilize LNP structures and promote the release of encapsulated substances. Cationic lipids with these features, as disclosed in CN107406396A and CN103930398A, improve the intracellular expression efficiency of nucleic acid drugs. However, for LNPs that enter cells via endocytosis, the reductive sensitivity of disulfide bonds may not be fully utilized in practical applications. This is because, compared with the cytoplasm with a high concentration of GSH, the endosomal environment features a relatively lower GSH concentration and a poorer reductive condition, resulting in the disulfide bonds not necessarily undergoing effective cleavage within endosomes.

Therefore, the efficiency of conventional cationic lipids containing a disulfide bond for nucleic acid drug delivery is not fully satisfactory. To address this issue, substantial improvements are needed for cationic lipids containing a disulfide bond.

### SUMMARY

The object of the present invention is to provide a novel cationic lipid containing a disulfide bond, as well as a lipid composition and a lipid pharmaceutical composition, both comprising the cationic lipid, and formulations thereof, for use in the field of drug delivery.

**The above object of the present invention is achieved through the following technical solution:**
A cationic lipid containing a disulfide bond, the structure of which is represented by general formula (1): 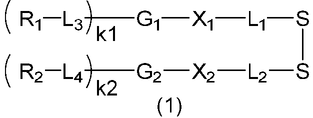
or salt, tautomer, stereoisomer, isotopically substituted derivative, or solvate thereof;
wherein, -S-S- is a disulfide bond;
L₁ and L₂ are each independently -(CH₂)ₜ-L_{d}-(CH₂)ₜ- or -(CH₂)ₜ-L_{d}-(CH₂)ₜ-L_{d}-(CH₂)ₜ-, wherein each t is independently an integer from 1 to 4;
L_{d} is a degradable divalent linking group; any two L_{d} in the same molecule have the same or different structures; preferably, each L_{d} is independently -NHC(=Y₁)Y₂- or -Y₂C(=Y₁)NH-, wherein Y₁ and Y₂ are each independently O, S, or NH;
X₁ and X₂ are each independently an acyclic divalent linking group containing a tertiary amine group;
G₁ and G₂ are each independently a linking bond or a trivalent branching group;
when G₁ is a linking bond, k1 is 1; when G₁ is a trivalent branching group, k1 is 2;
when G₂ is a linking bond, k1 is 1; when G₂ is a trivalent branching group, k2 is 2;
L₃ and L₄ are, at each occurrence, each independently selected from the group consisting of a linking bond, -(CH₂)_{q}-, Z, P₀, 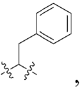 and combinations thereof; wherein, q is an integer from 1 to 10, Z is a heteroatom-containing divalent linking group, P₀ is 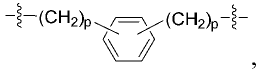 and p is, at each occurrence, independently 0 or 1;
R₁ and R₂ are each independently a substituted or unsubstituted C₅₋₃₀ hydrocarbon group or C₅₋₃₀ hydrocarbon derivative residue.

**Another embodiment of the present invention is provided as follows:**
A lipid composition comprising a disulfide-containing cationic lipid whose structure is represented by formula (1).

**Another embodiment of the present invention is provided as follows:**
A lipid pharmaceutical composition comprising a lipid composition and a drug, wherein the lipid composition contains a disulfide-containing cationic lipid whose structure is represented by formula (1).

**Another embodiment of the present invention is provided as follows:**
A lipid pharmaceutical composition formulation, comprising the aforementioned lipid pharmaceutical composition and a pharmaceutically acceptable diluent or excipient.

**Compared with the prior art, the present invention has the following advantageous effects:**
The cationic lipid structure of the present invention can be considered as two cationic lipid components, each having an acyclic tertiary amine head group and a hydrophobic tail, connected by a degradable segment. The degradable segment comprises a disulfide bond and other degradable divalent linking groups. In the reductive environment inside cells, the cationic lipid of the present invention can undergo initial degradation through the cleavage of the disulfide bond into two independent cationic lipids, destabilizing the LNP structure while retaining the ability of the cationic lipids to disrupt the endosomal membrane structure. This enables effective release of the loaded drug into the cytoplasm. The other degradable divalent linking groups in the degradable segment include, but are not limited to, carbamate groups, thiocarbamate groups, urea groups, thiourea groups, and the like, which have suitable degradability and can maintain sufficient stability of the LNP during the systemic circulation phase of drug delivery while promoting the degradation after cellular uptake (especially in the endosome), ensuring sufficient endosomal escape of the drug and significantly enhancing the efficacy of the LNP-nucleic acid pharmaceutical composition formulation. Optionally, appropriately increasing the number of degradable groups in the hydrophobic tails of the cationic lipid can enhance its intracellular degradability to some extent; or, introducing phenyl rings as part of the hydrocarbon skeleton of the cationic lipid can make the lipid structure more cone-shaped, thereby enhancing its ability to disrupt the endosomal membrane, promoting endosomal escape of the drug, and ultimately improving the drug delivery efficiency of the LNP.

The lipid composition prepared with the cationic lipid of the present invention, under the premise of good ability to complex with nucleic acid drugs, stability in the systemic circulation, and low toxicity, can more effectively exert the therapeutic effect of the LNP-nucleic acid pharmaceutical composition formulation, thereby improving immunological or therapeutic outcomes.

### BRIEF DESCRIPTION OF THE FIGURES

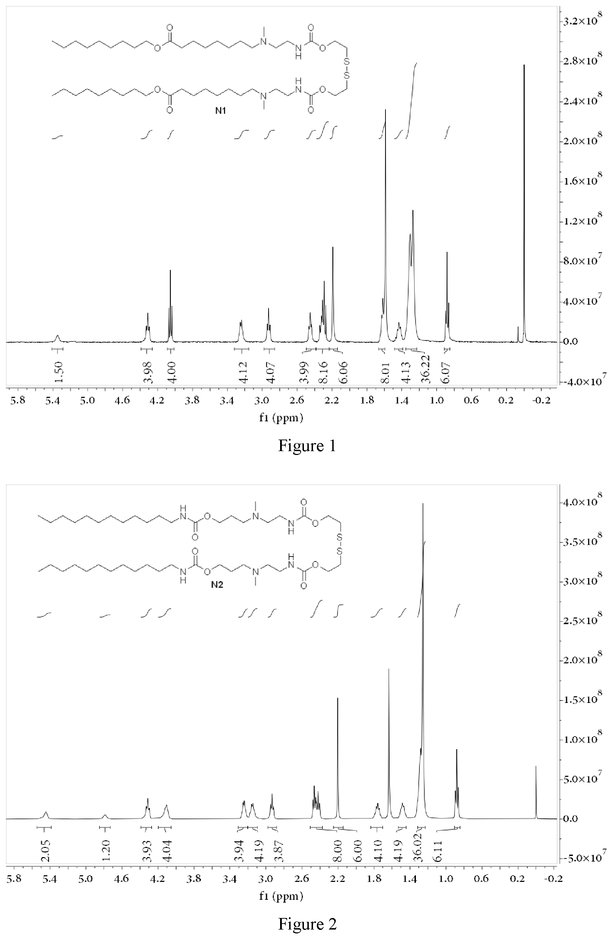

FIG. **1** shows the ¹H NMR spectrum of cationic lipid N1 prepared in Example 1.
FIG. **2** shows the ¹H NMR spectrum of cationic lipid N2 prepared in Example 2.

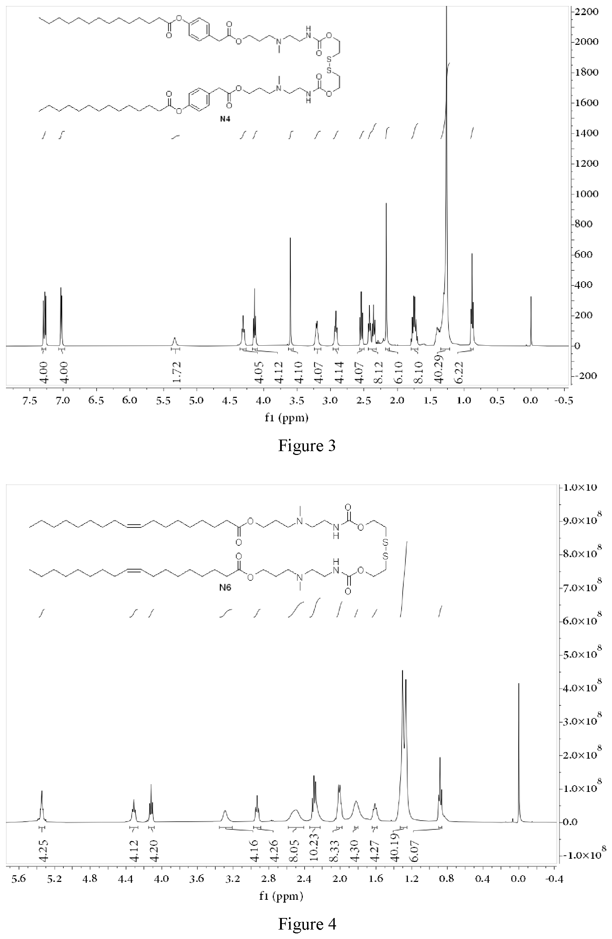

FIG. **3** shows the ¹H NMR spectrum of cationic lipid N4 prepared in Example 4.
FIG. **4** shows the ¹H NMR spectrum of cationic lipid N6 prepared in Example 6.

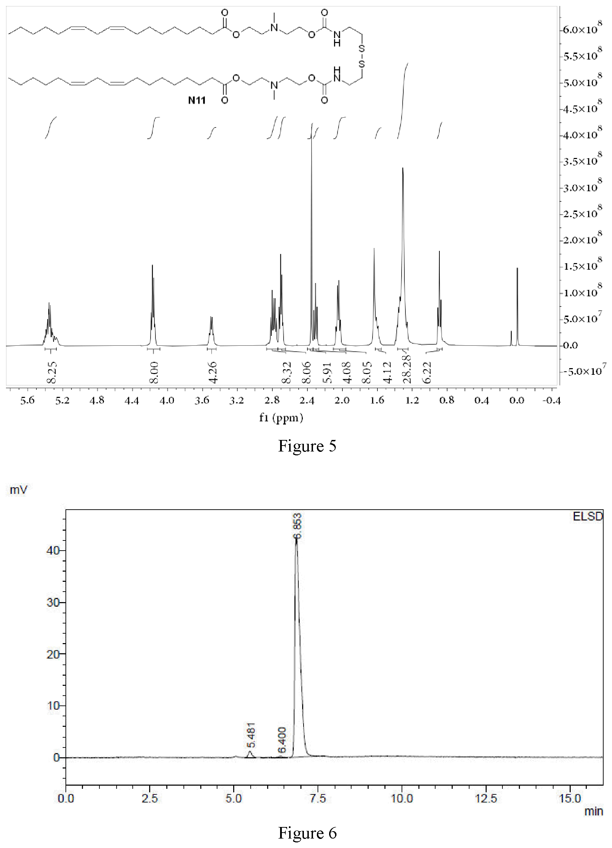

FIG. **5** shows the ¹H NMR spectrum of cationic lipid N11 prepared in Example 11.
FIG. **6** shows the high-performance liquid chromatography (HPLC) test result of cationic lipid N4 prepared in Example 4.
FIG. **7** shows the mass spectrometry (MS) of cationic lipid N4 prepared in Example 4.
FIG. **8** shows the cytotoxicity test results of the LNP-mRNA pharmaceutical composition L4 prepared in Example 22.
FIG. **9** shows the imaging results after mouse injection of the LNP-mRNA pharmaceutical composition L4 prepared in Example 22.

### DETAILED DESCRIPTION

### 1. Terminology Description

Unless otherwise specified, all technical and scientific terms used herein shall have the usual meaning in the field to which the subject matter pertains. If a term has multiple definitions, the definition provided herein shall prevail.

Unless otherwise specified, the terms "include", "comprise", "contain" and similar expressions should be interpreted in an open and inclusive manner, meaning "including but not limited to" or "non-restrictively including".

"Including but not limited to" a range means that the items within the specified range are optional, but the optional items are not limited to those within the range.

The term "independently" can be used not only to describe different objects with different names, but also to describe objects with the same name at different positions. For example, the statement "in -NR_{c}C(=O)O- and -NR_{c}C(=O)NR_{c}-, R_{c} is, at each occurrence, independently a hydrogen atom or a C₁₋₁₂ alkyl group" indicates that in -NR_{c}C(=O)NR_{c}-, the two R_{c} groups can be the same or different (e.g., one R_{c} being a methyl group and the other R_{c} being a hydrogen atom or an ethyl group), and each R_{c} in -NR_{c}C(=O)NR_{c}- can be the same as or different from the R_{c} in -NR_{c}C(=O)O-.

When at least two items are listed, the "combination" of the listed items refers to any combination of two or more of different listed items, or any combination of two or more of the same listed item. In the combination, the quantity of any item may be one or more, and the specific forms of multiple items with the same name may be identical or different from each other. By default, for a combination of linking groups, the linking groups form a new linking group by being interconnected. A combination of a linking bond and any linking group still refers to the linking group itself. For example, the statement "L is selected from the group consisting of a linking bond, a hydrocarbylene group, -O-, -S-, -C(=O)-, -NH-, and combinations thereof" indicates that L may be any one of the listed items, or any combination of two or more of the listed items. Such a combination could be -CH₂-O-CH₂- (i.e., a combination of -O- and hydrocarbylene groups, wherein the hydrocarbylene groups are specifically two methylene groups), -CH₂-NH-CH₂CH₂- (i.e., a combination of -NH- and hydrocarbylene groups, wherein the hydrocarbylene groups are specifically one methylene group and one ethylene group), -S-S- (a combination of two -S-), -C(=O)NH- (a combination of one -C(=O)- and one -NH-), etc.

A "linking bond" does not contain any atoms and serves only as a connecting element. It can be represented by a hyphen (e.g., the "-" at both ends of -C(=O)NH-), or marked by " 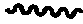 " (e.g., 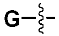 represents the group -G or G-). Particularly, when the definition of a group includes a "linking bond", it indicates that the group may be absent and that the position occupied by the group is taken by the linking bond.

A group can be monovalent, divalent, or multivalent, wherein "multivalent" indicates a valence of at least 3.

Groups with a valence of 2 or greater are collectively referred to as "linking groups", and by default, contain at least one atom. Linking groups that contain only one atom include, but are not limited to, ether group (-O-), thioether group (-S-), etc. The term "group" for a divalent linking group can be replaced with "bond" without altering the meaning. For example, a divalent ether group can also be referred to as an ether bond (-O-), a divalent ester group can also be referred to as an ester bond (-OC(=O)- or -C(=O)O-), and a divalent carbamate group can also be referred to as a carbamate bond (-OC(=O)NH- or -NHC(=O)O-). Unless otherwise specified, there are no particular restrictions on the linking ends of linking groups used to connect other groups. For example, when using -C(=O)NH- as a divalent linking group between group A and group B, either end of the -C(=O)NH- (left or right) can be used to connect A or B. Thus, both A-C(=O)NH-B and A-NHC(=O)-B are optional connected forms.

For a linking bond extended from a cyclic structure, when it is not marked at a specific ring atom but points inward to the ring, it indicates that the linking bond can be extended from any suitable ring atom. When the marked ring is part of a fused ring structure, the linking bond can be extended from any suitable ring atom within the fused ring structure. The linking bond represented in this manner as well as the group attached via such linking bond may be one or more instances; by default, unless otherwise specified, the number of instances is one. For example, 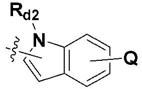 represents a group consisting of structures having any suitable chemical bondings (including but not limited to 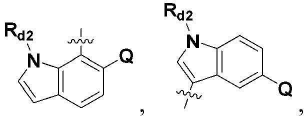 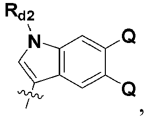 etc.).

The terms "any suitable linking group", "any suitable reactive group", "any suitable chemical bonding", etc., refer to those that conform to the basic principles of chemical structure. Chemical structures described in this manner are considered to have a clear and defined scope.

Numerical ranges can be indicated by a hyphen, a tilde, or the word "to", such as 1-6, 1~6, or "1 to 6", all of which represent the group consisting of all values between 1 and 6. Unless otherwise specified, the values include, but are not limited to, integers, non-integers, percentages, fractions, etc., and the range includes both endpoints. Unless otherwise specified, averages by default include both integers and non-integers. For example, "the average number of EO units is selected from 22 to 100" indicates that both integer 22 and non-integer 22.2 are valid options for the average number of EO units. Additionally, for example, "integers in the range 1-3" refers to the group consisting of 1, 2, and 3. Unless otherwise specified, numerical ranges indicating the number of groups are by default composed of integers. For example, -(CH₂)₁₋₄- refers to the group consisting of -CH₂-, -(CH₂)₂-, -(CH₂)₃-, and -(CH₂)₄-, and similarly, 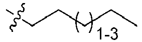 refers to the group consisting of

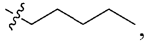

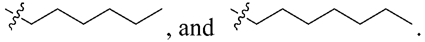

For percentages, when the given value is accurate to the N-th decimal place (excluding the percentage sign), the term "approximately" or "about" generally indicates a range of the base value ± (0.5*0.1^{N})%. For example, "approximately 1%" accurate to the 0th decimal place (excluding the percentage sign) by default refers to 1 ± (0.5*0.1⁰)%, i.e., the range of 0.5% to 1.5%; "approximately 2.2%" accurate to the 1st decimal place (excluding the percentage sign) by default refers to 2.2 ± (0.5*0.1¹)%, i.e., the range of 2.15% to 2.25%.

The numerical range indicated at the subscript position of "C" represents the number of carbon atoms in a group, and unless otherwise specified, the number of carbon atoms does not include contributions from substituents. For example, C₁₋₁₂ means "having 1 to 12 carbon atoms". For example, a C₁₋₁₀ alkylene group represents any alkylene group having 1 to 10 carbon atoms, i.e., any of C₁, C₂, C₃, C₄, C₅, C₆, C₇, C₈, C₉, and C₁₀ alkylene groups, including but not limited to a straight-chain C₁₋₁₀ alkylene group (e.g., -(CH₂)₆-) and a branched C₁₋₁₀ alkylene group (e.g., -(CH₂)₃-CH(CH₃)-(CH₂)₃-). For example, a "substituted C₁₋₃ alkyl group" refers to a C₁, C₂, or C₃ alkyl group that contains at least one substituent, such as -CH₂Cl, -CH₂CH₂OH, or -CH₂CH(OCH₃)CH₃, which represent -Cl substituted methyl group, -OH substituted ethyl group, and -OCH₃ substituted propyl group, respectively; the number of carbon atoms and heteroatoms in the substituents are not particularly limited.

Unless otherwise specified, any stereoisomers of a structure are considered disclosed collectively, including but not limited to *cis*/*trans* isomers, *E*/*Z* isomers, enantiomers (levorotatory/dextrorotatory isomers), etc. For example, for compounds with *cis-trans* isomerism, even if only the structure of the *cis* isomer is provided, the *trans* isomer is considered to be disclosed as well, and vice versa.

Unless otherwise specified, the unit for molecular weight is Dalton (Da). For polymers, the molecular weight generally refers to the average molecular weight, usually the number-average molecular weight (Mn).

The terms "stable" and "degradable" for groups are a pair of relative concepts.

A "degradable" linking group can undergo cleavage of chemical bonds to form two moieties that are not directly connected. If a linking group undergoes a chemical change that alters its structure but still remains a complete linking group, it is still categorized as "stable". There are no particular limitations on the conditions used to assess the degradability of a linking group, which may include physiological conditions in vivo, simulated physiological conditions in vitro, and other conditions, with a preference for physiological conditions in vivo or simulated physiological conditions in vitro. The physiological conditions have no particular restrictions and include, but are not limited to, serum, heart, liver, spleen, lungs, kidneys, bones, muscles, fat, brain, lymph nodes, small intestine, gonads, and other sites, which may refer to physiological conditions inside cells or in extracellular matrices, and may also refer to normal physiological tissues or pathological tissues (such as tumors, inflammatory tissues, etc.). The simulated physiological conditions in vitro have no particular limitations and include, but are not limited to, saline, buffer solutions, and culture media. There is no particular limitation on the rate of "degradation", for example, rapid degradation under enzyme action, slow hydrolysis under physiological conditions, etc. The physiological conditions in vivo include physiological conditions during treatment, such as UV irradiation, hyperthermia, etc. The conditions used to examine the degradability of a linking group include, but are not limited to, light, heat, low temperature, enzymes, redox reactions, acidity, basicity, physiological conditions, simulated physiological conditions in vitro, etc., with a preference for light, heat, enzymes, redox reactions, acidity, basicity, etc. The light conditions include, but are not limited to, visible light, ultraviolet light, infrared light, near-infrared light, mid-infrared light, etc. The heat conditions refer to temperatures above normal physiological temperatures, typically above 37°C and usually below 45°C, preferably below 42°C. The low temperature conditions refer to temperatures below normal body temperature, preferably below 25°C, more preferably ≤10°C, such as refrigeration temperatures, freezing temperatures, liquid nitrogen treatment temperatures, 2-10°C, 4-8°C, 4°C, 0°C, -20±5°C, etc. The enzymes have no particular restrictions and include any enzymes that can be generated under physiological conditions, such as peptidases, proteases, lyases, etc. The redox conditions have no specific restrictions and include, for example, the redox transition between thiol groups and disulfide bonds, hydrogenative reductive transitions, etc. The acidic and basic conditions mainly refer to the pH conditions at in vivo locations including normal tissues, pathological tissues, organs/tissues under treatment, etc.; for example, the stomach has acidic conditions, and tumor sites are often slightly acidic. Degradation can occur via in vivo metabolic processes (such as physiological effects, enzyme-catalyzed reactions, and redox reactions), under microenvironmental stimuli (such as acidity or basicity) at specific in vivo locations, or in response to stimuli during clinical treatment (such as light, heat, or low temperature), etc. It should be noted that certain conditions in organic chemistry, such as strong acids, strong bases, and high temperatures (e.g., above 100°C), etc., are extreme for biological systems, and the bond cleavage under these conditions is not categorized under "degradation". For example, although an ether bond may cleave under strong acid conditions such as hydrogen bromide, it should still be considered a stable linking group.

"Stable" linking groups are capable of maintaining their integrity as complete linking groups (stably covalently bonded with adjacent groups), allowing for chemical changes that do not compromise the integrity. The chemical changes are not particularly limited, including but not limited to isomerization, oxidation, reduction, ionization, protonation, deprotonation, substitution reactions, etc. The conditions used to assess the stability of a linking group are not particularly limited, including but not limited to light, heat, low temperature, enzymes, redox reactions, neutrality, acidity, basicity, physiological conditions, simulated physiological conditions in vitro, etc., with light, heat, enzymes, redox reactions, acidity, and basicity being preferred. The stable existence of a linking group indicates that the linking group can maintain its structural integrity and remain stably connected to other moieties in its environment in actual use (such as in the metabolic cycle in vivo) without special stimuli (such as pH conditions at specific sites, light, heat, low temperatures during treatment, etc.).

A linking group is not absolutely "stable" or "degradable"; its stability or degradability is influenced by the compound's structure and the specific environment in which it is used. For example, amide bonds are much more stable than ester bonds under acidic or basic conditions, and "stable" linking groups include amide bonds. However, peptide bonds, which are a type of amide bond formed by the dehydration condensation of the α-carboxyl group of one amino acid and the α-amino group of another, can be cleaved in the presence of specific enzymes, and thus are also considered "degradable" linking groups. Carbamate groups, thiocarbamate groups, and the like can serve as either stable or degradable linking groups; more generally, carbamate groups and thiocarbamate groups tend to undergo slow degradation, whereas non-peptide amide bonds may remain stable during in vivo circulation. Additionally, common ester bonds can degrade under acidic or basic conditions, while ester bonds within certain structures may also degrade under ultraviolet light. Furthermore, even if certain chemical bonds can degrade under the action of specific enzymes, if the compound's circulation path during clinical use does not pass through or minimally passes through the specific enzymatic environment (e.g., in site-specific drug delivery), these chemical bonds may still be considered stable.

All compounds of general formulas should be understood to include their salts. The term "salt" is selected from any one, or any combination of two or more types of acid addition salts formed with inorganic and/or organic acids, base addition salts formed with inorganic and/or organic bases, of the corresponding compound. When a compound of general formula contains both a basic moiety (such as, but not limited to, pyridine or imidazole) and an acidic moiety (such as, but not limited to, carboxylic acid), a zwitterion ("inner salt") can be formed and included in the term "salt". The "salt" can be pharmaceutically acceptable (i.e., non-toxic, physiologically acceptable) or otherwise. The salt of a compound of general formula can be formed by reacting the compound of general formula with a certain amount (e.g., an equivalent) of acid or base in a medium such as a medium for salt precipitation or an aqueous medium, followed by lyophilization. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecyl sulfates, ethanesulfonates, fumarates, glucoheptanoates, glycerophosphates, hemi sulfates, heptanoates, hexanoates, hydrochlorides, hydrobromides, hydroiodides, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates, sulfonates, tartrates, thiocyanates, toluenesulfonates, undecanoates, etc. Exemplary base addition salts include ammonium salts, alkali metal salts (e.g., sodium, lithium, and potassium salts), alkaline earth metal salts (e.g., calcium and magnesium salts), salts with organic bases (e.g., organic amines), and salts with amino acids (e.g., arginine, lysine). Basic nitrogen-containing groups may be quaternized with agents such as lower alkyl halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, tetradecyl, and stearyl chlorides, bromides, and iodides), aralkyl halides (e.g., benzyl and phenethyl bromides), etc. The acid addition salts and base addition salts are both preferably pharmaceutically acceptable salts.

The term "solvate" refers to an aggregate of a compound with one or more solvent molecules. The solvent may be water, and the corresponding solvate may be referred to as a hydrate, including monohydrate, dihydrate, hemihydrate, sesquihydrate, trihydrate, tetrahydrate, etc. The solvent may also be an organic solvent.

The term "tautomer" refers to isomers that result from tautomerism of a compound. Tautomerism refers to a process in which a hydrogen atom or proton is relocated within the molecule, leading to a change in the compound structure, often involving the interconversion between single bonds and double bonds. Tautomerism includes, but is not limited to, keto-enol tautomerism, amide-imidic acid tautomerism, lactam-lactim tautomerism, enamine-imine tautomerism, enamine-enamine tautomerism (e.g., enzyme reactions catalyzed by pyridoxal phosphate), proton transfer tautomerism, valence tautomerism, etc.

The term "stereoisomer" refers to isomers that have the same sequence of atomic connectivity but possess different three-dimensional structures. Stereoisomerism can be categorized into enantiomerism and diastereomerism, with diastereomerism further encompassing cis-trans isomerism and conformational isomerism. Enantiomers are molecules that are mirror images of each other and cannot be superimposed.

The term "isotopically substituted derivative" refers to the forms derived when one or more atoms in a compound molecule are replaced by their respective isotopes.

Heteroatoms are not particularly limited, including but not limited to O, S, N, P, Si, F, Cl, Br, I, B, etc.

When a group or compound is described as "heteroatom-containing", the location of the heteroatoms is not particularly limited, including but not limited to cases where the heteroatoms are located in the main chain, side chain, and/or substituents.

Relative to a compound, a group formed by the loss of certain atoms or groups is also referred to as a residue.

When a molecular structure (including those in the form of a compound, group, fragment, etc.) is described as "substituted", it indicates that the molecular structure contains one or more substituents. Relative to the "unsubstituted" structure, the "substituted" structure is also referred to as the "substituted form". Unless otherwise specified, a "substituent" can be a monoatomic or polyatomic group, excluding hydrogen atom, and replaces the hydrogen atom at the position originally occupied in the molecular structure. For substituted hydrocarbon groups and heterohydrocarbon groups (including but not limited to alkyl groups, heteroalkyl groups, etc.), the substituents include but are not limited to =O, -OR", =NR", =N-OR", -NR"R", -SR", -F, -Cl, -Br, -I, -SiR"R"R", -C(=O)R", -OC(=O)R", -C(=O)OR", -OC(=O)OR", -C(=O)NR"R", -OC(=O)NR"R", -NR"C(=O)R", -NR"C(=O)NR"R", -NR"C(=O)OR", -CN, -NO₂, etc.; wherein, each R" is independently hydrogen or a C₁₋₁₀ alkyl group containing 0 to 3 heteroatoms; any two R" groups may optionally form a cyclic structure with the nitrogen atom they are both connected to; for example, -NR"R" includes, but is not limited to, 1-pyrrolidinyl group and 4-morpholinyl group.

The term "optionally substituted" indicates that the described group or compound can be either substituted or unsubstituted.

The terms "amine group" and "amino group" both include neutral or cationic forms with valences of one, two, three, or four. The "amino group" includes primary amino group (-NH₂), secondary amino group (-NH-), tertiary amino group (-N<), and quaternary amino group (>N⁺<). Amine compounds refer to hydrocarbon-substituted derivatives of NH₃, including primary amine (mono-hydrocarbon substitution, e.g., CH₃NH₂), secondary amine (di-hydrocarbon substitution, e.g., NH(CH₃)₂), tertiary amine (tri-hydrocarbon substitution, e.g., N(CH₃)₃), and quaternary ammonium (tetra-hydrocarbon substitution, e.g., N⁺(CH₃)₃). The groups formed by the loss of one or more hydrogen atoms from amine compounds are collectively referred to as "amine groups", wherein the hydrogen atoms may come from the amino group, hydrocarbon groups, or other groups. The cationic form of an amino or amine group is also termed a quaternary ammonium group.

The term "amide" refers to compounds with the general formula R^{p}-C(=O)-N(R^{q})₂, wherein R^{p} is an organic group, and each R^{q} is independently H or an organic group. When R^{p} or R^{q} is an organic group, it is connected to the (C=O) or N in the general formula through its own carbon atom. Depending on the nature of R^{q}, amides can be classified into primary amides, secondary amides, and tertiary amides; wherein, in primary amides, both R^{q} groups are H; in secondary amides, one R^{q} is H and the other R^{q} is an organic group; in tertiary amides, both R^{q} groups are organic groups. Secondary amides are also termed N-substituted amides, and tertiary amides are also termed N,N-disubstituted amides.

The term "amide group" refers to a monovalent, divalent, or trivalent group formed by the loss of certain atoms or groups from an amide. For example, -C(=O)NH- is a divalent amide group, and -C(=O)N< is a trivalent amide group. Unless otherwise specified, the term "amide bond" by default refers to -C(=O)NH-.

The term "hydrocarbon" refers to a compound composed of carbon and hydrogen elements. Hydrocarbons that do not contain benzene rings or other aromatic rings are collectively referred to as "aliphatic hydrocarbons". Aliphatic hydrocarbons can be divided into open-chain hydrocarbons and alicyclic hydrocarbons, with open-chain hydrocarbons further classified into straight-chain hydrocarbons and branched-chain hydrocarbons. Based on the degree of unsaturation, aliphatic hydrocarbons can be divided into saturated and unsaturated aliphatic hydrocarbons, which are further categorized into alkanes, alkenes, and alkynes; wherein, alkenes contain at least one carbon-carbon double bond, and alkynes contain at least one carbon-carbon triple bond.

The term "hydrocarbon group" refers to a group formed when a hydrocarbon loses at least one hydrogen atom, and it can be monovalent, divalent, trivalent, quadrivalent, or higher-valent. Unless otherwise specified, a hydrocarbon group is assumed to be monovalent. Unless otherwise specified, a hydrocarbon group is optionally substituted, and the substituents are not particularly limited. When an aliphatic hydrocarbon group is described as "substituted", its substituent groups may or may not contain carbon-carbon double bonds, carbon-carbon triple bonds, and/or aromatic rings.

The term "hydrocarbon derivative" refers to aliphatic hydrocarbon derivatives and aromatic hydrocarbon derivatives, whose structure contains one or more non-hydrocarbon moieties located in side chains, substituent groups, and/or the skeleton. Common non-hydrocarbon moieties are heteroatom-containing groups. For example, ether compounds can be considered as aliphatic hydrocarbon derivatives with an -O- group in the skeleton, and halogenated benzenes can be considered as aromatic hydrocarbon derivatives with halogen substituents. The term "hydrocarbon derivative residue" refers to a group formed when a hydrocarbon derivative loses at least one hydrogen atom.

The term "alkyl group" refers to a hydrocarbon group formed by the loss of a hydrogen atom from any position of an alkane, which can be linear or branched. Specifically, e.g., a propyl group refers to either n-propyl group or isopropyl group. Unless otherwise specified, alkyl groups are optionally substituted.

The term "alkenyl group" refers to a hydrocarbon group formed by the loss of a hydrogen atom from any position of an alkene, containing at least one carbon-carbon double bond, and can be linear or branched. For example, "C₂₋₁₅ alkenyl group" refers to an alkenyl group with 2 to 15 carbon atoms and at least one carbon-carbon double bond, which can be either linear or branched. Alkenyl groups can contain one, two, three, four, or more carbon-carbon double bonds. Unless otherwise specified, alkenyl groups are optionally substituted.

The term "alkynyl group" refers to a hydrocarbon group formed by the loss of a hydrogen atom from any position of an alkyne, containing at least one carbon-carbon triple bond, and can be linear or branched. For example, "C₂₋₁₅ alkynyl group" refers to an alkynyl group with 2 to 15 carbon atoms and at least one carbon-carbon triple bond, which can be either linear or branched. Alkynyl groups can contain one, two, three, four, or more carbon-carbon triple bonds. Unless otherwise specified, alkynyl groups are optionally substituted.

The term "hydrocarbylene group" refers to a divalent hydrocarbon group, the term "alkylene group" refers to a divalent alkyl group, the term "alkenylene group" refers to a divalent alkenyl group, and the term "alkynylene group" refers to a divalent alkynyl group. Unless otherwise specified, hydrocarbylene groups, alkylene groups, alkenylene groups, and alkynylene groups include both open-chain structures and ring-containing structures, and are optionally substituted.

The term "amino acid residue" includes residues formed after amino acids undergo the following modifications: removal of a hydrogen atom from the amino group, removal of a hydroxyl group from the carboxyl group, removal of a hydrogen atom from the thiol group, protection of the amino group, protection of the carboxyl group, protection of the thiol group, and combinations thereof. The amino acids may be of natural origin, non-natural origin, or a mixture of both. The structure of amino acids may be L-type, D-type, or a mixture of both. The divalent amino acid residue is preferably a residue formed by removing a hydrogen atom from the amino group and a hydroxyl group from the carboxyl group of the corresponding amino acid. For example, the structure of divalent glycine residue is preferably

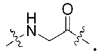

The term "oligopeptide" refers to a peptide molecule containing two or more amino acid residues, preferably with 2 to 20 amino acid residues.

The term "functional group" includes but is not limited to reactive groups, protected reactive groups, precursors of reactive groups, and the like. A polyfunctional compound contains at least two functional groups. For example, a polyol refers to a compound containing at least two hydroxyl groups, and a polythiol refers to a compound containing at least two thiol groups. It should be noted that the functional groups in a polyfunctional compound may include more than one type. For instance, tris(hydroxymethyl)aminomethane is a triol that also contains an amino group, and citric acid is a tricarboxylic acid that also contains a hydroxyl group.

Unless otherwise specified, the reactive groups involved in the preparation method also include their protected forms, which can be deprotected at any suitable step during the actual preparation process to yield the corresponding active forms.

Ring atoms refer to atoms that jointly constitute the ring skeleton.

The term "minor modification" refers to a chemical modification achievable through a simple chemical reaction process. Such simple chemical reaction processes primarily include protection, deprotection, salt complexation, decomplexation, ionization, protonation, deprotonation, and alteration of leaving groups, etc. The alteration of leaving groups, i.e., the transformation of leaving groups, includes but is not limited to the conversion from an ester form to an acyl chloride form. The term "minorly modified form" refers to a structural form capable of forming the target reactive group through minor modification.

The "protection" of a reactive group refers to a strategy in which the reactive group to be protected is reversibly converted into an inert group (a non-reactive group) using specific reagents. In the protected group, the portion that differs from the unprotected form is referred to as the "protecting group". For example, -OTBS is a protected form of a hydroxyl group (-OH), where TBS serves as the protecting group for the hydroxyl group.

The term "deprotection" refers to the process of converting the protected form into the unprotected form.

The term "hydroxyl protecting group" includes groups commonly used in the art for protecting hydroxyl groups, and is not limited to alkanoyl group (e.g., acetyl group, tert-butanoyl group), aralkanoyl group (e.g., benzoyl group), benzyl group, triphenylmethyl group, trimethylsilyl group, tert-butyldimethylsilyl group, allyl group, acetal group, ketal group, etc. Removal of the acetyl group is generally carried out under basic conditions, with the most commonly used systems being NH₃/MeOH and NaOMe/MeOH. The benzyl protecting group can be removed by catalytic hydrogenation or by reduction using metallic sodium or lithium in ethanol or liquid ammonia. The triphenylmethyl group is typically removed by catalytic hydrogenation. The trimethylsilyl group is usually removed using systems containing fluoride ions (e.g., tetrabutylammonium fluoride/anhydrous THF, etc.). The tert-butyldimethylsilyl group can be removed using systems containing fluoride ions or by treatment with wet acetic acid at room temperature. Protection of diols includes, but is not limited to, the formation of dioxolane, dioxane, cyclic carbonate, cyclic borate ester, etc.

The term "amino protecting group" includes groups commonly used in the art for protecting amino groups, such as aryl C₁₋₆ alkyl, C₁₋₆ alkoxy C₁₋₆ alkyl, C₁₋₆ alkoxycarbonyl, aryloxycarbonyl, C₁₋₆ alkylsulfonyl, arylsulfonyl, methylsilyl, and the like. Preferred amino protecting groups include Boc (tert-butoxycarbonyl), Moz (p-methoxybenzyloxycarbonyl), Cbz (benzyloxycarbonyl), or Fmoc (9-fluorenylmethyloxycarbonyl). Reagents for removing amino protecting groups include, but are not limited to, TFA, H₂O, LiOH, MeOH, EtOH, and combinations thereof. TFA is preferred for removing the Boc protection. A 20% piperidine in N,N-dimethylformamide (DMF) solution is preferred for removing the Fmoc protection.

The hydroxyl groups that can be protected are not particularly limited, and include hydroxyl groups in compounds such as alcohols, phenols, and the like. The amino groups that can be protected are not particularly limited, and include amino groups in compounds such as primary amines, secondary amines, diamines, amides, and the like.

The term "lipid" refers to a broad group of organic compounds, including fats, waxes, sterols, fat-soluble vitamins (such as vitamins A, D, E, K), monoglycerides, diglycerides, phospholipids, and other hydrophobic or amphiphilic small molecules. Lipids include simple esters, complex esters, and derived lipids. The simple esters are esters formed from fatty acids and alcohols, which can further be categorized into fats, oils, and waxes. The complex esters, also referred to as "lipoids", include phospholipids, sphingolipids, glycolipids, steroids, sterols, and lipoproteins. The derived lipids include simple lipid derivatives and complex lipid derivatives, with general characteristics of lipids. Lipids can be compounds that are synthetic or derived (isolated or modified) from natural sources.

The term "lipid nanoparticle" (LNP) refers to nanoparticles in the nanometer scale (e.g., 1 nm to 1000 nm) that contain one or more lipids. LNPs may further contain at least one non-lipid active payload molecule (e.g., one or more nucleic acid molecules).

The term "cationic lipid" refers to a lipid that carries a positive charge at any pH or hydrogen ion activity, or a lipid that becomes positively charged in response to the pH or hydrogen ion activity of its intended use environment (i.e., "cationizable lipid"); the latter also includes zwitterionic lipids exhibiting this characteristic. In some cases, the positive charge of the cationic lipid originates from the presence of a quaternary nitrogen atom. For LNPs containing cationizable lipids, it is preferred that at a pH range of about 1 to 9, more preferably at a pH range of 4 to 9, 5 to 8, or 6 to 8, and most preferably at endosomal pH (e.g., about 5.5 to 6.5), approximately 1% to 100% of the cationizable lipids transition to their cationic forms.

The term "PEGylated lipid" refers to a molecule containing both lipid and polyethylene glycol moieties, which can be further classified into linear and non-linear PEGylated lipids based on the structure of the polyethylene glycol moiety.

The term "neutral lipid" refers to a lipid that exists in an overall neutral form at a selected pH, either non-ionic or zwitterionic, including phospholipids.

The term "steroid lipid" refers to a lipid characterized primarily by a fused ring system ( 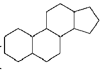 ) consisting of three cyclohexane rings and one cyclopentane ring.

The term "targeting group" refers to a group that provides a strong affinity for a selected target (e.g., cell, tissue, organ, or body regions or compartments, such as cell, tissue, or organ compartments). Exemplary targeting groups include, but are not limited to, residues of the following substances: antibodies, antigens, peptides, vitamins, carbohydrates (including but not limited to monosaccharides such as N-acetylgalactosamine (GalNAc)), folic acid, aptamers, receptor ligands, transferrin, biotin, PSMA, endothelin, GCPII, somatostatin, LDL ligands, and HDL ligands.

The term "N/P ratio" refers to the molar ratio of cationizable groups (typically, such as tertiary amine groups) in the cationic lipid to the phosphate groups in the nucleic acid.

The term "nucleic acid" refers to DNA, RNA, or modified forms thereof, containing purine and pyrimidine bases that constitute DNA or RNA (adenine "A," cytosine "C," guanine "G," thymine "T," uracil "U").

The term "RNA" refers to ribonucleic acid that can occur naturally or non-naturally. RNA may include one or more modified and/or non-naturally occurring nucleobases, nucleosides, nucleotides, or linkers. RNA may include cap structures, chain-terminating nucleosides, stem-loops, polyadenylation sequences, and/or polyadenylation signals. Examples of RNA include, but are not limited to, small interfering RNA (siRNA), asymmetric interfering RNA (aiRNA), microRNA (miRNA), Dicer-substrate RNA (dsRNA), small hairpin RNA (shRNA), messenger RNA (mRNA), single-guide RNA (sgRNA), cas9 mRNA, etc. Antisense oligonucleotides or small interfering RNA (siRNA) may inhibit the expression of target genes and target proteins in vitro or in vivo. RNA may possess a nucleotide sequence encoding a specific polypeptide, such as mRNA, which can be translated to produce the encoded polypeptide. FLuc mRNA expresses luciferase protein, which emits bioluminescence in the presence of a luciferin substrate.

Methods for determining target gene expression levels include, but are not limited to, dot blotting, Northern blotting, in situ hybridization, ELISA, immunoprecipitation, enzymatic assays, and phenotypic assays.

The term "transfection" refers to the process of introducing a species (e.g., RNA) into cells. Transfection may occur in vitro, ex vivo, or in vivo.

The term "antigen" typically refers to a substance that can be recognized by the immune system, preferably by the adaptive immune system, and is capable of triggering an antigen-specific immune response, such as the formation of antibodies and/or antigen-specific T cells as part of the adaptive immune response. Typically, an antigen can be or include peptides or proteins that can be presented to T cells by MHC molecules. An antigen may be the translation product of the provided nucleic acid molecule, preferably mRNA as defined herein. In this context, peptides, as well as fragments, variants, and derivatives of proteins, which contain at least one epitope, are also understood to be antigens.

The term "delivery" refers to the provision of an entity to a target. For example, delivery may involve administering drugs and/or therapeutic agents and/or prophylactic agents to a subject, wherein the subject refers to organs and/or tissues and/or cells of humans and/or other animals.

The term "pharmaceutically acceptable carrier" refers to a diluent, excipient, adjuvant, or medium administered together with a therapeutic agent, which, within the bounds of reasonable medical judgment, is suitable for contact with the tissues of humans and/or other animals without causing excessive toxicity, irritation, allergic response, or other complications or issues inconsistent with a reasonable benefit/risk ratio. Pharmaceutically acceptable carriers usable in pharmaceutical compositions include, but are not limited to, sterile liquids such as water and oils, including oils of petroleum, animal, plant, or synthetic origin, for example, peanut oil, soybean oil, mineral oil, sesame oil, and the like. When the pharmaceutical composition is administered intravenously, water is an exemplary carrier. Saline, glucose and glycerol aqueous solutions can also be used as liquid carriers, particularly for injectable formulations. Suitable pharmaceutical excipients include starch, glucose, lactose, sucrose, gelatin, maltose, chalk, silica, sodium stearate, glyceryl monostearate, talc, sodium chloride, skimmed milk powder, glycerol, propylene glycol, water, ethanol, and others. The pharmaceutical composition may include small amounts of wetting agents, emulsifiers, or pH buffering agents as needed. Oral formulations may contain standard carriers such as pharmaceutical-grade mannitol, lactose, starch, magnesium stearate, sodium saccharin, cellulose, magnesium carbonate, and others. Specifically, excipients include, but are not limited to, antiadherents, antioxidants, binders, coatings, compression aids, disintegrants, dyes (pigments), soothing agents, emulsifiers, fillers (diluents), film formers, flavoring agents, fragrances, flow aids (flow enhancers), lubricants, preservatives, printing inks, adsorbents, suspending agents or dispersants, sweeteners, and water for hydration. More specifically, excipients include, but are not limited to, butylated hydroxytoluene (BHT), calcium carbonate, dicalcium phosphate, calcium stearate, croscarmellose sodium, cross-linked polyvinylpyrrolidone, citric acid, crospovidone, cysteine, ethyl cellulose, gelatin, hydroxypropyl cellulose, hydroxypropyl methylcellulose, lactose, magnesium stearate, maltitol, mannitol, methionine, methylcellulose, methylparaben, microcrystalline cellulose, polyethylene glycol, polyvinylpyrrolidone, povidone, pregelatinized starch, phenylparaben, retinyl palmitate, shellac, silicon dioxide, sodium carboxymethylcellulose, sodium citrate, sodium glycolate starch, sorbitol, starch (corn), stearic acid, sucrose, talc, titanium dioxide, vitamin A, vitamin E (α-tocopherol), vitamin C, and xylitol.

Pharmaceutical compositions may act systemically and/or locally. For this purpose, they can be administered via suitable routes, such as injection (e.g., intravenous, intra-arterial, subcutaneous, intraperitoneal, or intramuscular injection, including infusion), transdermal administration, oral administration, sublingual administration, intranasal administration, transmucosal administration, topical administration, ocular formulations, or inhalation. Pharmaceutical compositions may be administered in suitable dosage forms, including but not limited to tablets, capsules, lozenges, troches, powders, sprays, creams, ointments, suppositories, gels, pastes, lotions, salves, aqueous suspensions, injectable solutions, elixirs, and syrups.

The term "antagomir", also known as "anti-miR", refers to a class of chemically engineered oligonucleotides designed to silence endogenous miRNAs.

The term "vaccine" refers to a prophylactic or therapeutic material that provides at least one antigen or antigenic function. The antigen or antigenic function may stimulate the body's adaptive immune system to elicit an adaptive immune response.

The term "treatment" refers to the management and care of a patient aimed at addressing a disease, disorder, or condition, intended to encompass delaying the progression of the disease, disorder, or condition; alleviating or mitigating symptoms and complications; and/or curing or eliminating the disease, disorder, or condition. The patient to be treated is preferably a mammal, particularly a human.

### 2. Cationic Lipid Containing a Disulfide Bond

### An embodiment of the present invention is as follows:

A cationic lipid containing a disulfide bond, the structure of which is represented by general formula (1): 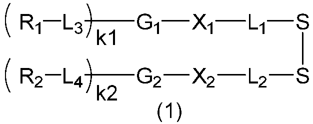
or salt, tautomer, stereoisomer, isotopically substituted derivative, or solvate thereof;
wherein, -S-S- is a disulfide bond;
L₁ and L₂ are each independently -(CH₂)ₜ-L_{d}-(CH₂)ₜ- or -(CH₂)ₜ-L_{d}-(CH₂)ₜ-L_{d}-(CH₂)ₜ-, wherein each t is independently an integer from 1 to 4;
L_{d} is a degradable divalent linking group; any two L_{d} in the same molecule have the same or different structures; preferably, each L_{d} is independently -NHC(=Y₁)Y₂- or -Y₂C(=Y₁)NH-, wherein Y₁ and Y₂ are each independently O, S, or NH;
X₁ and X₂ are each independently an acyclic divalent linking group containing a tertiary amine group;
G₁ and G₂ are each independently a linking bond or a trivalent branching group;
when G₁ is a linking bond, k1 is 1; when G₁ is a trivalent branching group, k1 is 2;
when G₂ is a linking bond, k2 is 1; when G₂ is a trivalent branching group, k2 is 2;
L₃ and L₄ are, at each occurrence, each independently selected from the group consisting of a linking bond, -(CH₂)_{q}-, Z, P₀, 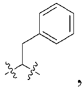 and combinations thereof; wherein, q is an integer from 1 to 10, Z is a heteroatom-containing divalent linking group, P₀ is 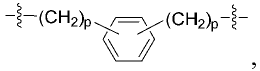 and p is, at each occurrence, independently 0 or 1;
R₁ and R₂ are each independently a substituted or unsubstituted C₅₋₃₀ hydrocarbon group or C₅₋₃₀ hydrocarbon derivative residue.

### 2.1. L_{d}, L₁, and L₂

In a specific embodiment of the present invention, L_{d} is selected from divalent linking groups of the following structures: disulfide ether, ethylene ether, thioester, disulfide ester, carbonate ester, thiocarbonate ester, dithiocarbonate ester, trithiocarbonate ester, carbamate, thiocarbamate, dithiocarbamate, urea, thiourea, acetal, cyclic acetal, thioacetal, aza-acetal, cyclic aza-acetal, aza-thioacetal, dithioacetal, hemiacetal, thiohemiacetal, aza-hemiacetal, ketal, thioketal, azaketal, cyclic azaketal, aza-thioketal, imine, hydrazone, acylhydrazone, oxime, sulfoxime, semicarbazone, thiosemicarbazone, hydrazine, acylhydrazine, thiocarbohydrazide, carbazone, thiocarbazone, carbazate, thiocarbazate, carbazide, thiocarbazide, azo bond, isourea, isothiourea, allophanate, thioallophanate, guanidine, amidine, aminoguanidine, aminoamidine, imidate, thioimidate, sulfonate, sulfinate, sulfonyl hydrazine, sulfonylurea, maleimide, orthoester, phosphate, phosphite, phosphinate, phosphonate, phosphosilyl ester, silyl ester, carbamide, thiocarbamide, sulfonamide, polyamide, phosphamide, phosphorous amide, pyrophosphamide, cyclic phosphamide, ifosfamide, thiophosphamide, aconitine amide, oligopeptide fragment, skeleton of nucleotide and derivative thereof, skeleton of deoxyribonucleotide and derivative thereof; or, L_{d} is a degradable divalent linking group formed by any of the aforementioned divalent linking groups and adjacent heteroatom-containing groups thereof; the adjacent heteroatom-containing groups are selected from -O-, -S-, -C(=O), -NR_{c}-, and any combination thereof, wherein R_{c} is a hydrogen atom or a C₁₋₆ alkyl group; preferably, L_{d} is selected from a divalent linking group of carbamate, thiocarbamate, dithiocarbamate, urea, or thiourea.

In a specific embodiment of the present invention, L₁ and L₂ are identical or different, each independently being -(CH₂)ₜ-L_{d}-(CH₂)ₜ-; preferably, L₁ and L₂ are identical and selected from the group consisting of -(CH₂)ₜ-OC(=O)NH-(CH₂)ₜ-, -(CH₂)ₜ-NHC(=O)O-(CH₂)ₜ-, and -(CH₂)ₜ-NHC(=S)NH-(CH₂)ₜ-; more preferably, L₁ and L₂ are identical and selected from the group consisting of -(CH₂)₂-NHC(=O)O-(CH₂)₂-, -(CH₂)₂-OC(=O)NH-(CH₂)₂-, and -(CH₂)₂-NHC(=S)NH-(CH₂)₂-.

### 2.2. X₁ and X₂

In a specific embodiment of the present invention, X₁ and X₂ are each independently 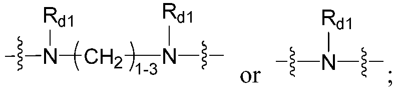 wherein, R_{d1} is, at each occurrence, independently a substituted or unsubstituted C₁₋₆ alkyl group, preferably a hydroxyl-substituted or unsubstituted C₁₋₆ alkyl group;
preferably, X₁ and X₂ are identical and selected from the group consisting of

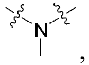

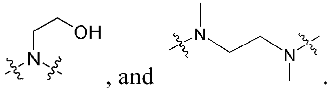

### 2.3. R₁ and R₂

In a specific embodiment of the present invention, R₁ and R₂ are identical or different, each independently being a C₅₋₃₀ aliphatic hydrocarbon group, a C₅₋₃₀ aliphatic hydrocarbon derivative residue, a C₇₋₃₀ aromatic hydrocarbon group, or a C₇₋₃₀ aromatic hydrocarbon derivative residue; preferably, R₁ and R₂ are each independently selected from the group consisting of R_{L}, R_{B}, Rᵣ, and R_{A}; more preferably, R₁ and R₂ are each independently R_{L}, R_{B}, or R_{A};
R_{L} is a linear C₅₋₃₀ aliphatic hydrocarbon group containing 0 to 4 carbon-carbon double bonds or carbon-carbon triple bonds; preferably, R_{L} is any one or a substituted form of 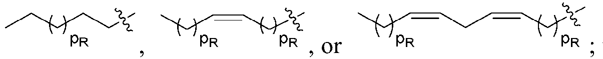 wherein, p_{R} is, at each occurrence, independently an integer from 1 to 15; the substituted form contains 1 to 4 substituents; the substituent is, at each occurrence, a linear or branched C₁₋₄ alkyl group, a hydroxyl group, or a halogen atom, preferably a methyl group or a hydroxyl group; more preferably, R_{L} is selected from the group consisting of the following structures: 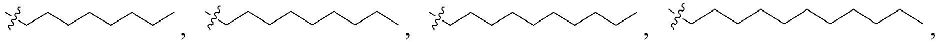 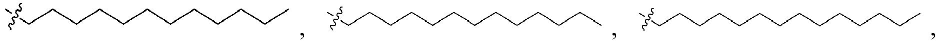 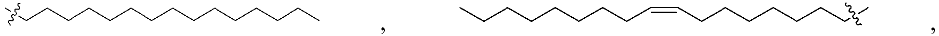 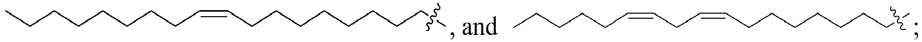
R_{B} is a branched C₅₋₃₀ aliphatic hydrocarbon group or C₅₋₃₀ aliphatic hydrocarbon derivative residue, selected from the group consisting of the following structures: 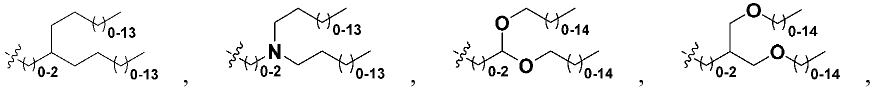 and 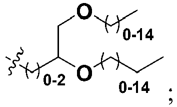
preferably, R_{B} is selected from the group consisting of the following structures: 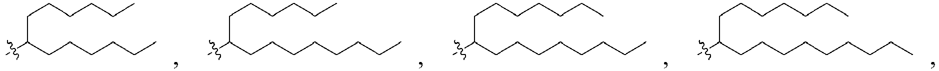 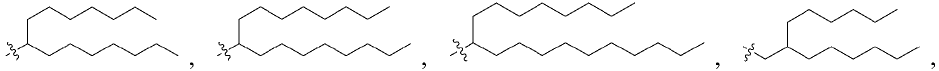 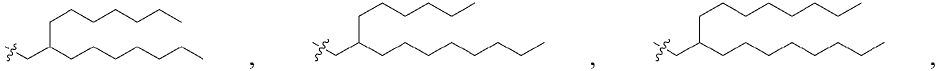 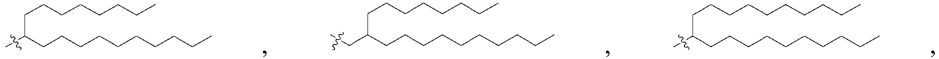 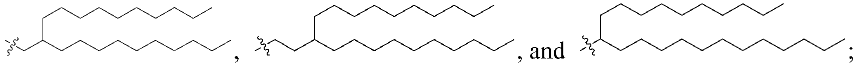
Rᵣ is a ring-containing C₅₋₃₀ aliphatic hydrocarbon group, preferably 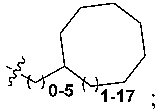
R_{A} is a C₇₋₃₀ aromatic hydrocarbon group or C₇₋₃₀ aromatic hydrocarbon derivative residue, preferably

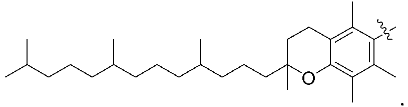

### 2.4. L₃ and L₄

In a specific embodiment of the present invention, Z is, at each occurrence, independently selected from the group consisting of -O-, -S-, -OC(=O)-, -C(=O)O-, -OC(=O)O-, -C(=O)-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, -C(R_{c})=N-NR_{c}-, -NR_{c}-N=C(R_{c})-, and -CH(OH)-; wherein, R_{c} is, at each occurrence, independently H or a methyl group.

In a specific embodiment of the present invention, L₃ and L₄ are each independently selected from the group consisting of a linking bond, -Z-(CH₂)_{q}-, -Z-(CH₂)_{q}-Z-(CH₂)_{q}-, -Z-P₀-(CH₂)_{q}-, -Z-P₀-Z-(CH₂)_{q}-, and -P₀-(CH₂)_{q}-, and the left end is connected to R₁ or R₂; wherein, the structures of any two Z are identical or different from each other.

In a specific embodiment of the present invention, Z is, at each occurrence, independently selected from the group consisting of -O-, -C(=O)-, -OC(=O)-, -C(=O)O-, -NHC(=O)-, -C(=O)NH-, -OC(=O)O-, -OC(=O)NH-, -NHC(=O)O-, and -CH(OH)-.

In a specific embodiment of the present invention, at least one of L₃ and L₄ comprises P₀ and is preferably -Z-P₀-(CH₂)_{q}-*, -Z-P₀-Z-(CH₂)_{q}-*, or -P₀-(CH₂)_{q}-*, wherein the * end is connected to G₁ or G₂; preferably, P₀ is

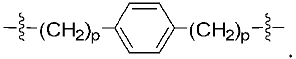

In a specific embodiment of the present invention, L₃ and L₄ are identical.

In a specific embodiment of the present invention, L₃ and L₄ are identical and defined by any of the following cases:
Case (1): L₃ and L₄ are both linking bonds;
Case (2): L₃ and L₄ are both -C(=O)O-(CH₂)_{q}- or -OC(=O)-(CH₂)_{q}-;
Case (3): L₃ and L₄ are both -C(=O)NH-(CH₂)_{q}- or -NHC(=O)-(CH₂)_{q}-;
Case (4): L₃ and L₄ are both -OC(=O)NH-(CH₂)_{q}- or -NHC(=O)O-(CH₂)_{q}-;
Case (5): L₃ and L₄ are both -OC(=O)-(CH₂)_{q}-C(=O)O-(CH₂)_{q}-, -OC(=O)-(CH₂)_{q}-OC(=O)-(CH₂)_{q}-, -C(=O)O-(CH₂)_{q}-C(=O)O-(CH₂)_{q}-, -C(=O)O-(CH₂)_{q}-OC(=O)-(CH₂)_{q}-, -OC(=O)-(CH₂)_{q}-C(=O)NH-(CH₂)_{q}-, -OC(=O)-(CH₂)_{q}-NHC(=O)-(CH₂)_{q}-, -C(=O)O-(CH₂)_{q}-C(=O)NH-(CH₂)_{q}-, -C(=O)O-(CH₂)_{q}-NHC(=O)-(CH₂)_{q}-, -OC(=O)-(CH₂)_{q}-CH(OH)-(CH₂)_{q}-, -C(=O)O-(CH₂)_{q}-CH(OH)-(CH₂)_{q}-, -NHC(=O)-(CH₂)_{q}-CH(OH)-(CH₂)_{q}-, or -C(=O)NH-(CH₂)_{q}-CH(OH)-(CH₂)_{q}-;
Case (6): L₃ and L₄ are both -OC(=O)-P₀-(CH₂)_{q}- or -C(=O)O-P₀-C(=O)O-(CH₂)_{q}-, preferably 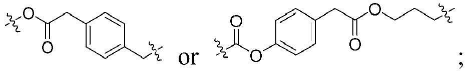
in any of the above cases, the left end of L₃ is connected to R₁, and the left end of L₄ is connected to R₂.

### 2.5. Other General Formulas

In a specific embodiment of the present invention, the structure of the cationic lipid containing a disulfide bond is represented by general formula (2) or (3): 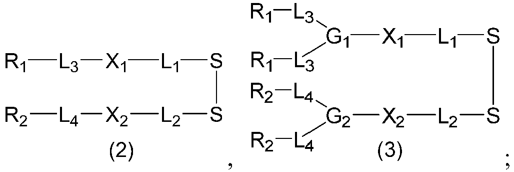
in general formula (3), G₁ and G₂ are both trivalent branching groups, two L₃ are identical, two R₁ are identical, two L₄ are identical, and two R₂ are identical;
in a more specific embodiment, the structure of the cationic lipid is represented by general formula (3), wherein G₁ and G₂ are each independently represented by 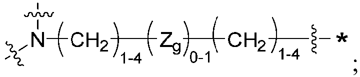 wherein, the * end is connected to X₁ or X₂, the other two ends are connected to L₃ or L₄, and Z_{g} is selected from the group consisting of -O-, -C(=O)-, -OC(=O)-, -C(=O)O-, -NHC(=O)-, -C(=O)NH-, -OC(=O)O-, -OC(=O)NH-, and -NHC(=O)O-;
preferably, G₁ and G₂ are each independently selected from the group consisting of the following structures: 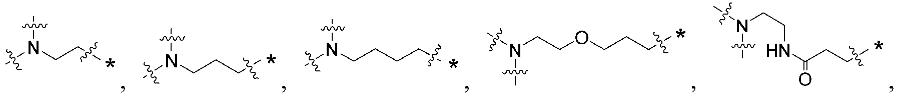 and 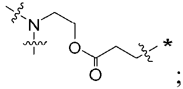 more preferably, G₁ and G₂ are both 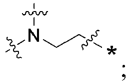
preferably, all L₃ and L₄ are -Z-(CH₂)_{q}-.

In a specific embodiment of the present invention, the structure of the cationic lipid containing a disulfide bond is represented by any of the following general formulas: 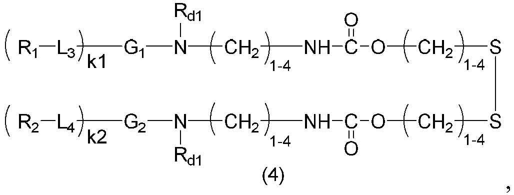 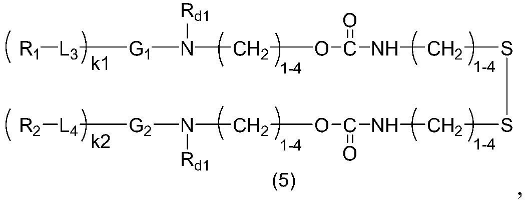 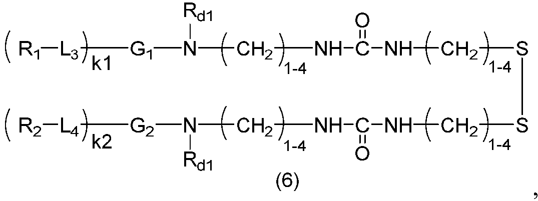 and

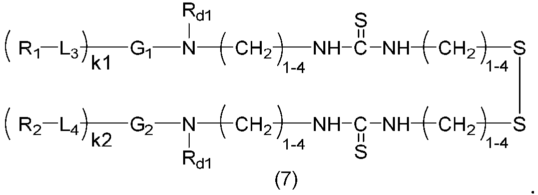

### 2.6. Examples of Specific Structures

In a specific embodiment of the present invention, the structure of the cationic lipid containing a disulfide bond is selected from the group consisting of **N1** to **N21:** 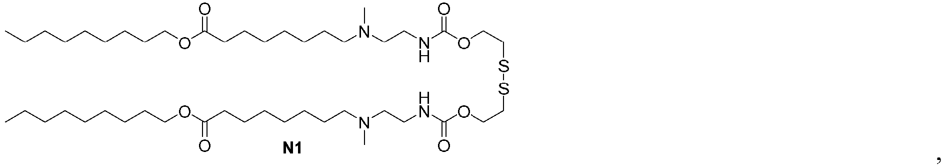 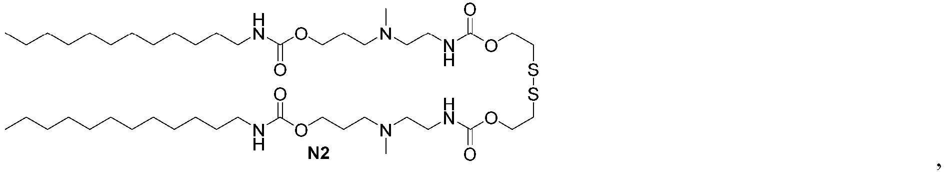 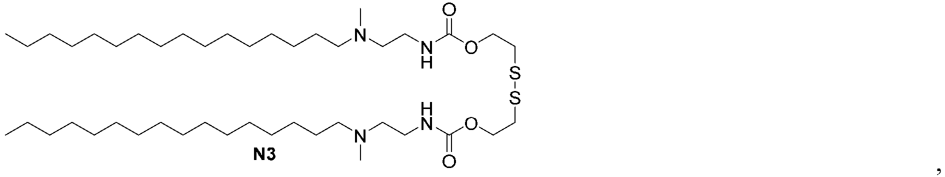 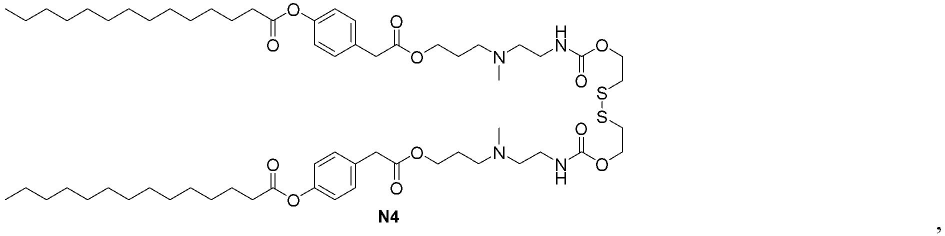 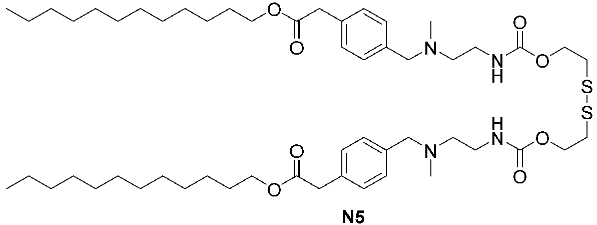 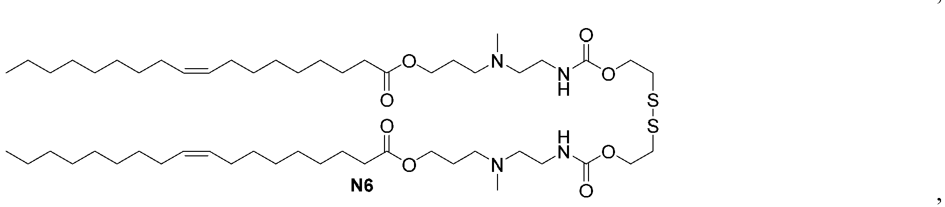 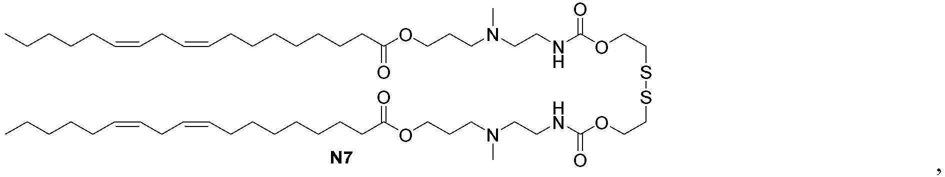 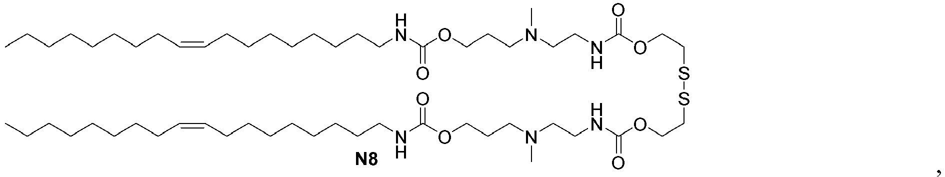 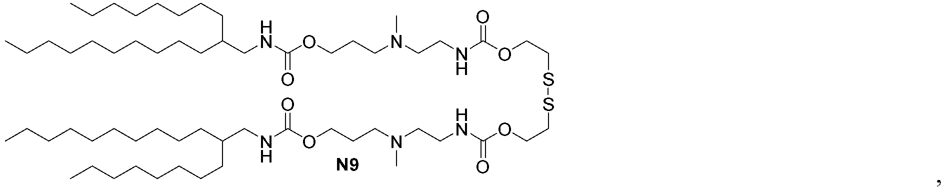 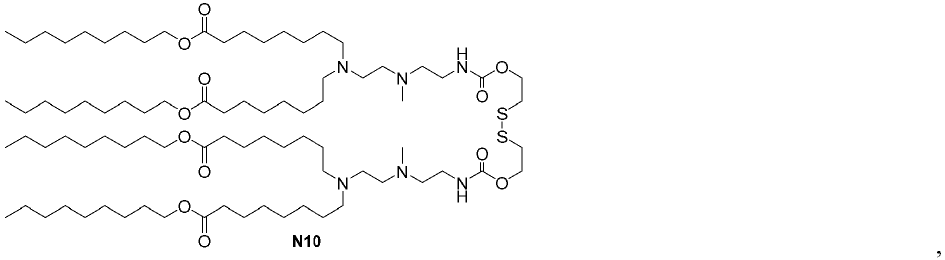 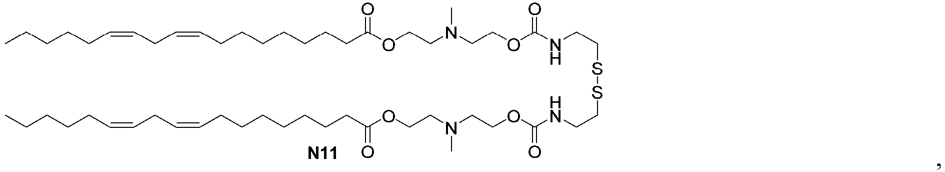 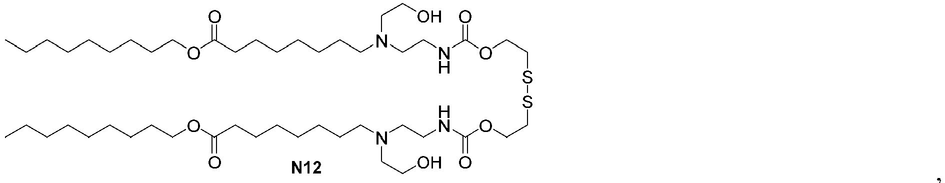 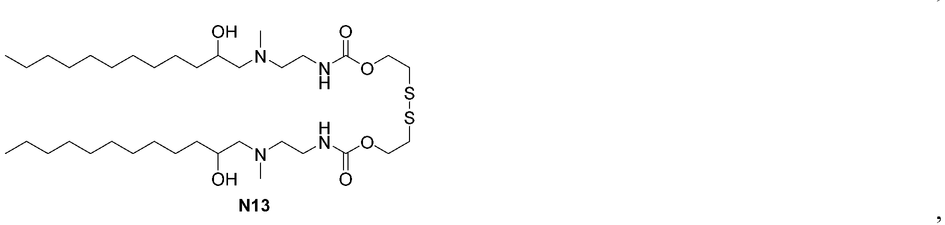 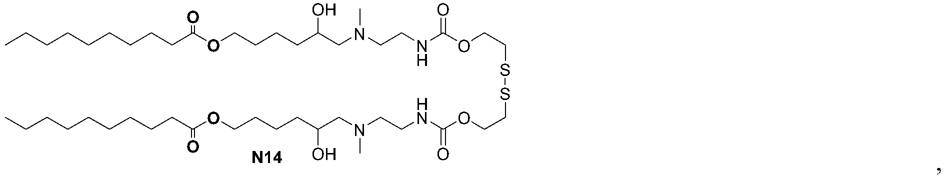 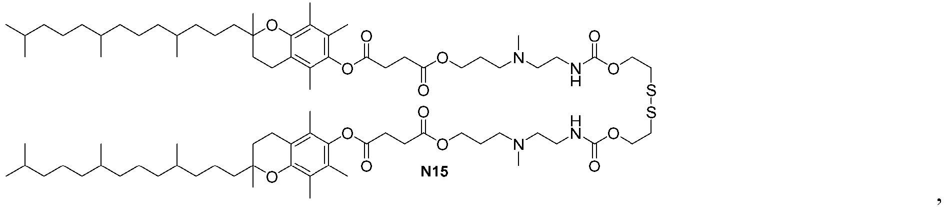 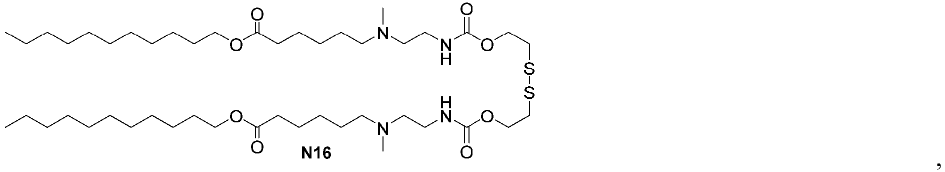 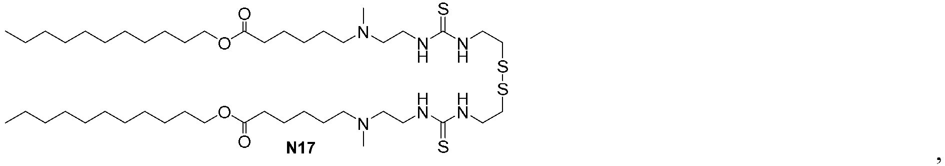 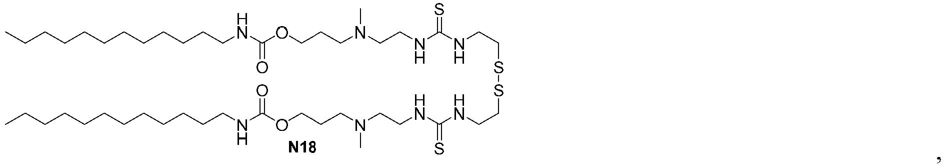 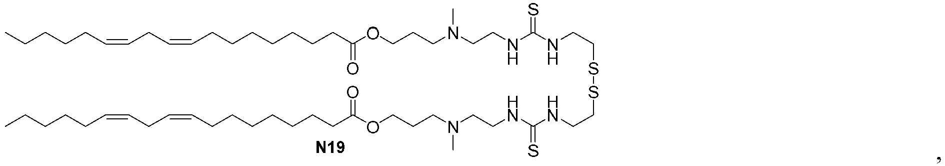 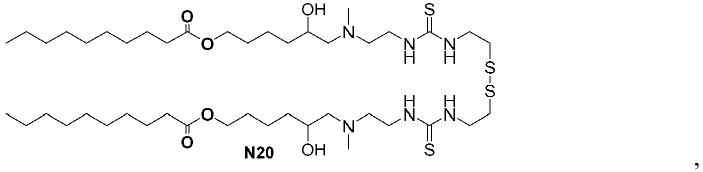 and

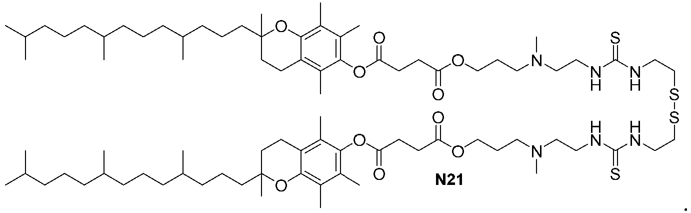

In a specific embodiment of the present invention, the structure of the cationic lipid containing a disulfide bond is selected from the group consisting of the following: 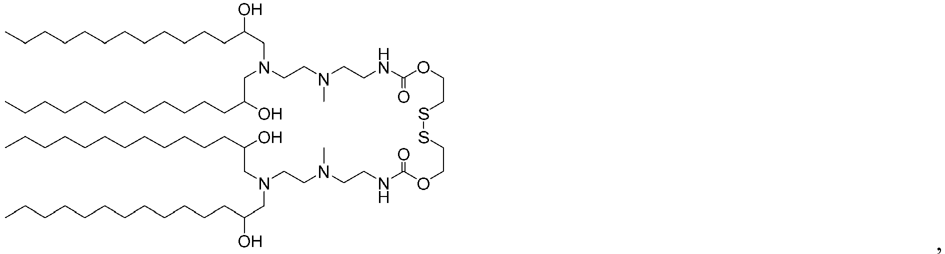 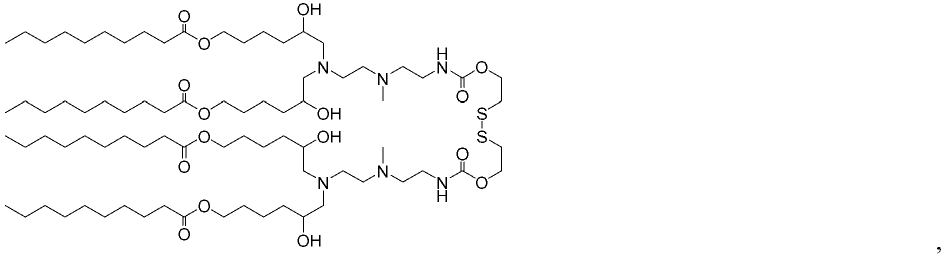 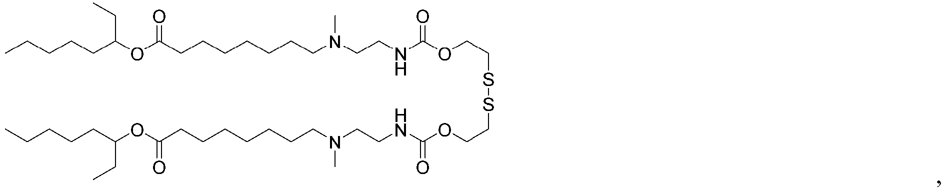 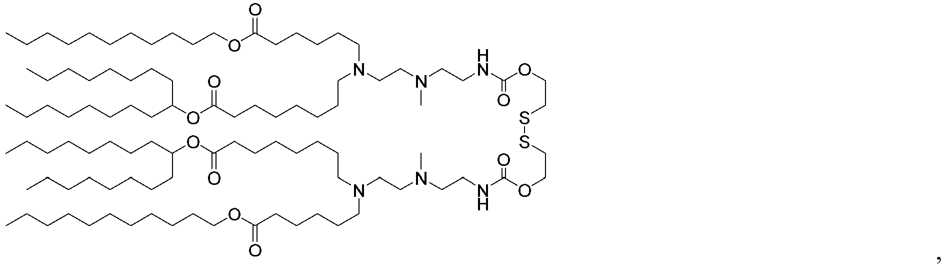 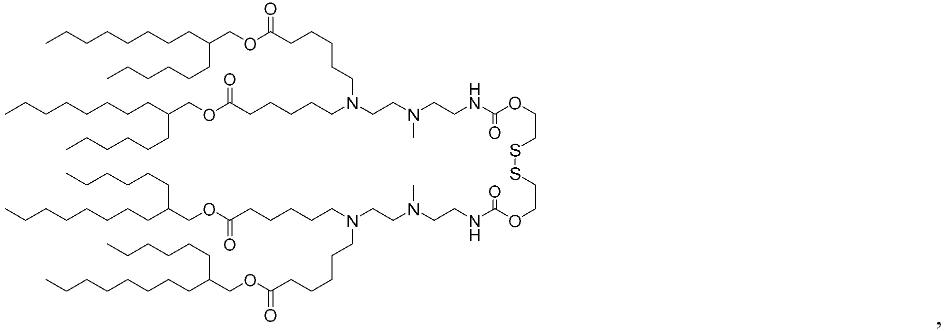 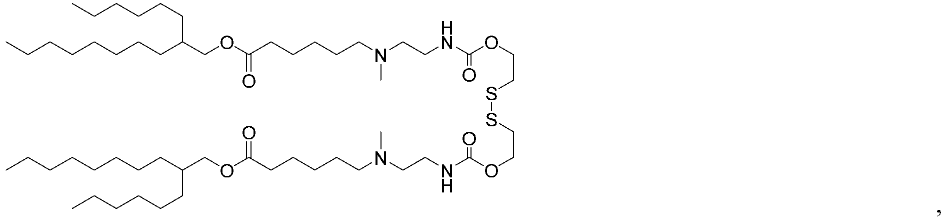 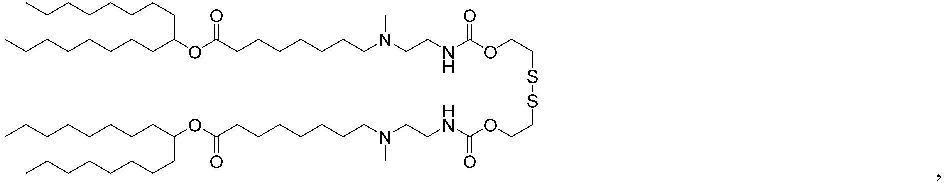 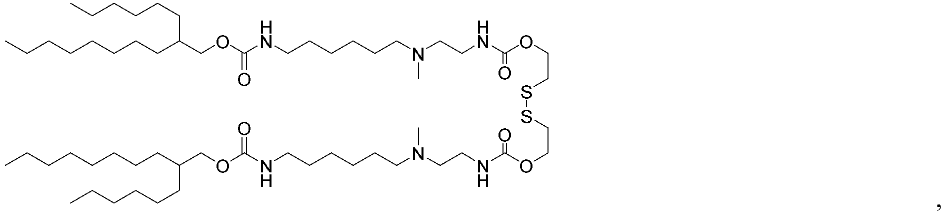 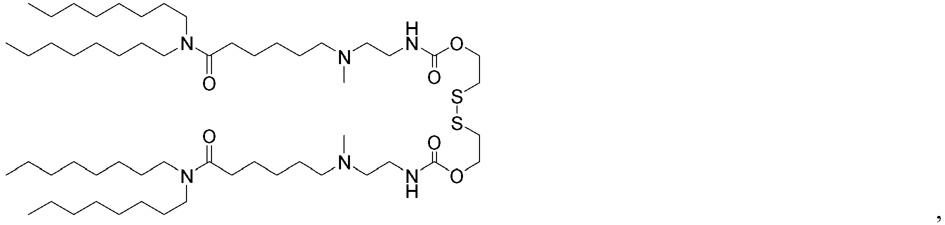 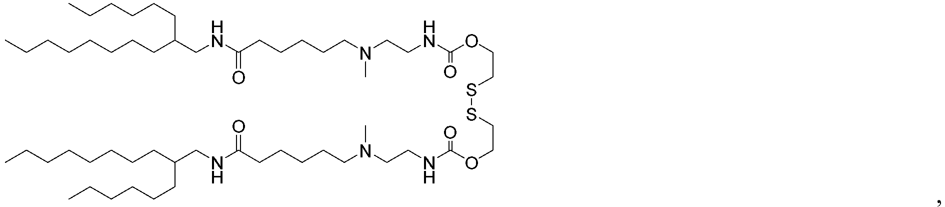 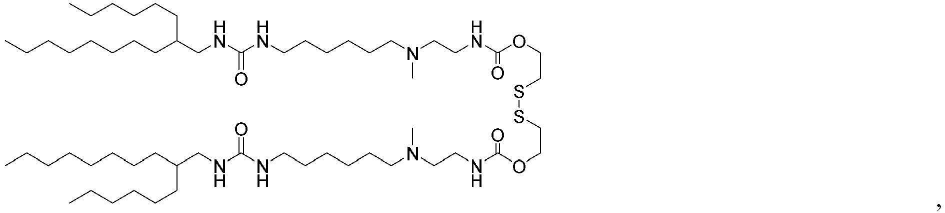 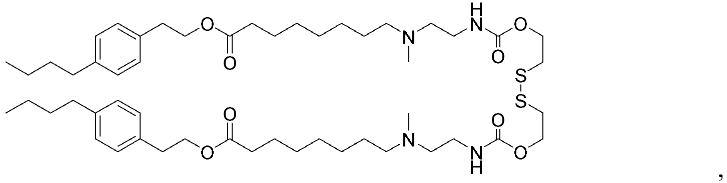 and

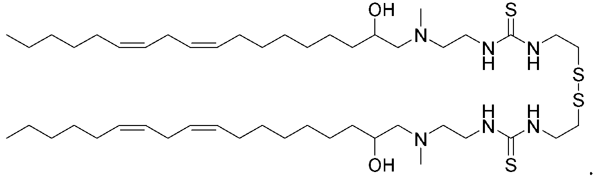

### 3. Methods for Preparing the Cationic Lipid Containing a Disulfide Bond

In the preparation method of the cationic lipid of the present invention, "protection" and "deprotection" of reactive groups are performed as needed. When more than two reactive groups are involved in the reaction process, the reactive group that may interfere with the desired reaction is typically protected, allowing selective reaction of the target reactive group. The protected group remains stable during the course of the target reaction. If necessary, deprotection can be carried out at any suitable stage in the preparation process using conventional techniques in the field.

The preparation method of the cationic lipid of the present invention involves reaction types including coupling reactions. Based on the successful implementation of the present invention, the type of coupling reaction is not particularly limited, as long as the reactive groups can form a covalent linking group through the reaction. The same preparation process may contain one-step or multi-step coupling reactions. Preferably, each step of the coupling reaction is independently selected from alkylation, condensation, amidation, esterification, thioesterification, ring-opening reaction, ring-closing condensation, addition, cycloaddition, α,β-unsaturated bond addition, alkynyl addition, Schiff base reaction combined with reduction, click reaction, azide-alkyne addition, 1,3-dipolar cycloaddition, Diels-Alder addition, thiol-yne reaction, thiol-ene reaction, and thiol-vinyl reaction. The reaction conditions for the coupling reaction are related to the type of covalent linking group formed in the reaction, and conventional techniques may be employed. The valency of the covalent linking group formed by the coupling reaction can be divalent or trivalent, preferably divalent. The coupling reaction may result in a stable group or a degradable group.

The cationic lipid containing a disulfide bond of the present invention can be prepared by methods including, but not limited to, any of the following. Unless otherwise specified, the definitions of the parameters involved in the following methods (e.g., X₁, X₂, L₁, L₂, etc.) are the same as previously described.

**Method 1:** A compound containing a disulfide bond is first coupled with a compound containing X₁/X₂, followed by further coupling with a compound containing R₁/R₂.

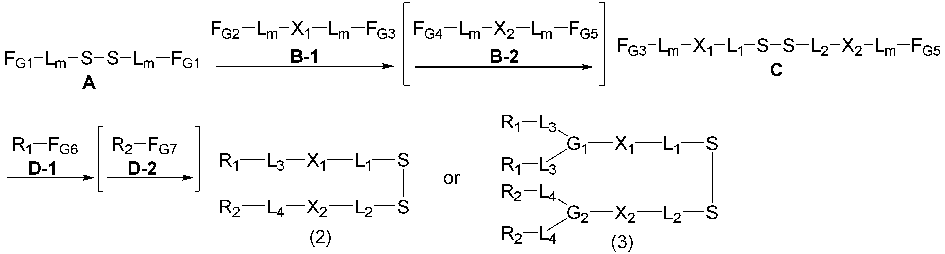

**Step 1:** The bifunctional small molecule **A** containing a disulfide bond is sequentially reacted with the bifunctional small molecule **B-1** containing X₁ and the bifunctional small molecule **B-2** containing X₂ to obtain the bifunctional intermediate **C.** When **B-1** and **B-2** are identical, compounds **A** and **B-1** can react in a single step to yield compound **C.** Wherein, the two F_{G1} groups of compound **A** may be identical or different, with identical groups being preferred. The divalent linking group formed from the reaction of F_{G1} and F_{G2}, together with the adjacent two Lₘ derived respectively from **A** and **B-1,** jointly constitutes L₁. Similarly, the divalent linking group formed from the reaction of F_{G1} and F_{G4}, together with the adjacent two Lₘ derived respectively from **A** and **B-2,** jointly constitutes L₂.

**Step 2:** The bifunctional intermediate C is sequentially reacted with the monofunctional small molecules **D-1** and **D-2** containing R₁/R₂ to yield the cationic lipid containing a disulfide bond represented by general formula (1). Wherein, the divalent linking group formed in the reaction, together with the Lₘ in C, jointly constitutes L₃ or L₄, and the corresponding G₁ or G₂ serves as a linking bond. Alternatively, the trivalent linking group formed in the reaction, together with the Lₘ in C, jointly constitutes (L₃)₂G₁ or (L₄)₂G₂, and the corresponding G₁ or G₂ serves as a trivalent branching group. When **D-1** and **D-2** are identical, one molecule of compound **C** reacts with two molecules of **D-1** to yield the cationic lipid represented by general formula (2), or one molecule of compound **C** reacts with four molecules of **D-1** to yield the cationic lipid represented by general formula (3).

Lₘ is a linking bond or a divalent linking group, and any two Lₘ may have the same or different structures. Preferably, Lₘ is a linking bond, an alkylene group, or an alkylene group interrupted by one to three Lₘ₀. The alkylene group may be substituted or unsubstituted, and each Lₘ₀ is independently selected from the group consisting of -O-, -S-, -C(=O)-, -C(=S)-, -C(=O)NH-, -NHC(=O)-, -C(=O)O-, and -OC(=O)-. Additionally, no Lₘ₀ is directly connected to any other Lₘ₀. F_{G1}, F_{G2}, F_{G3}, F_{G4}, F_{G5}, F_{G6}, and F_{G7} are reactive groups or minorly modified forms thereof, with specific types chosen to ensure the smooth implementation of the present invention. Preferably, F_{G1}, F_{G2}, F_{G3}, F_{G4}, F_{G5}, F_{G6}, and F_{G7} are each selected from the group consisting of -OH, -NH₂, -COOH, active ester groups, -F, -Cl, -Br, -I, -OMs, -OTs, anhydride groups, epoxy groups, -NCO, -NCS, and protected forms or acid/base addition salts thereof. The protected forms of the reactive groups may be deprotected at any appropriate step to perform the necessary subsequent reactions or to obtain the final product. Particularly, F_{G3} in **B-1** or F_{G5} in **B-2** may also be a hydrogen atom or an amino protecting group directly connected to the nitrogen atom of X₁ or X₂, wherein the amino protecting group is preferably -Boc, -Cbz, or Fmoc.

The compound **C** obtained in Step 1 may also first undergo a coupling reaction with other bifunctional small molecules to obtain another compound **C** with the same general formula but a different specific structure, which can then be used for the reaction in Step 2.

**Method 2:** A compound containing X₁/X₂ is first coupled with a compound containing R₁/R₂, followed by further coupling with a compound containing a disulfide bond.

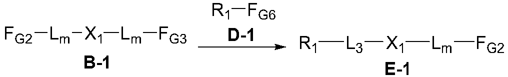

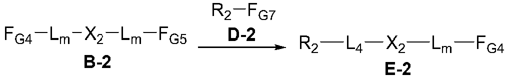

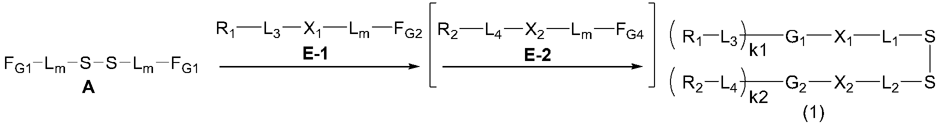

The primary difference between Method 2 and Method 1 lies in that, in Method 2, compound **B-1/B-2** is first reacted with compound **D-1/D-2** to obtain compound **E-1,** which contains both R₁ and X₁, or compound **E-2,** which contains both R₂ and X₂. Subsequently, compound **A** reacts with compound **E-1/E-2** to yield the cationic lipid containing a disulfide bond represented by general formula (1). The definitions of Lₘ, F_{G1}, F_{G2}, F_{G3}, F_{G4}, F_{G5}, F_{G6}, and F_{G7}, as well as other details, are the same as those described in Method 1 and will not be repeated here.

**Method 3:** A trifunctional small molecule compound is involved in the coupling reactions to obtain the cationic lipid with four tail chains.

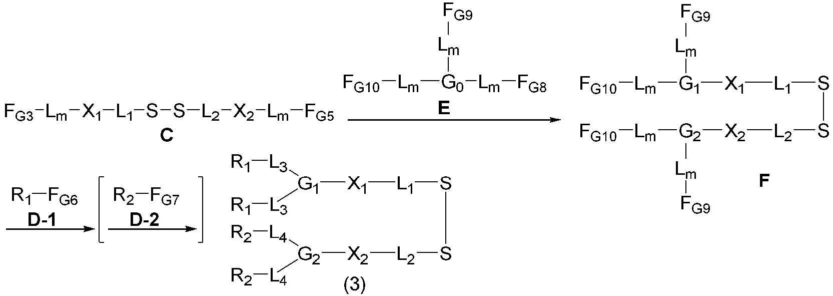

The compound **C** from Method 1 reacts with the trifunctional small molecule compound **E** to yield the intermediate compound **F,** which contains four reactive groups (two F_{G9} and two F_{G10}). Compound **E** includes a trivalent branching core G₀, and its reactive group F_{G8} reacts with F_{G3} of compound **C.** The resulting divalent linking group, together with the adjacent two Lₘ derived respectively from **C** and **E** and the core G₀, jointly constitutes the trivalent branching core G₁. Similarly, the trivalent branching core G₂ is formed through the reaction of F_{G8} of compound **E** with F_{G5} of compound **C.** The F_{G9} and F_{G10} groups in compound **F** may be identical or different reactive groups. Similar to Step 2 of Method 1, compound **F** undergoes a coupling reaction with compound **D-1/D-2.** The resulting divalent linking group, together with the Lₘ in **F,** jointly constitutes L₃/L₄, yielding the cationic lipid represented by general formula (3).

F_{G8}, F_{G9}, and F_{G10} are reactive groups or minorly modified forms thereof, with specific types chosen to ensure the smooth implementation of the present invention. Preferably, F_{G8}, F_{G9}, and F_{G10} are each selected from the group consisting of -OH, -NH₂, -COOH, and protected forms or acid/base addition salts thereof. The definitions of Lₘ, F_{G3}, F_{G5}, F_{G6}, and F_{G7}, as well as other details, are the same as those described in Method 1 and will not be repeated here.

### 4. Lipid Composition, Lipid Pharmaceutical Composition, Lipid Pharmaceutical Composition Formulation, and Preparation Methods Thereof

### An embodiment of the present invention:

A lipid composition, which contains any of the aforementioned cationic lipid containing a disulfide bond.

In a specific embodiment of the present invention, the aforementioned lipid composition further contains phospholipid; or, the aforementioned lipid composition further contains steroid lipid; or, the aforementioned lipid composition further contains PEGylated lipid; or, the aforementioned lipid composition further contains phospholipid and steroid lipid; or, the aforementioned lipid composition further contains phospholipid and PEGylated lipid; or, the aforementioned lipid composition further contains steroid lipid and PEGylated lipid; or, the aforementioned lipid composition further contains phospholipid, steroid lipid, and PEGylated lipid; or, the aforementioned lipid composition further contains phospholipid, steroid lipid, PEGylated lipid, and another type of cationic lipid; or, the aforementioned lipid composition further contains phospholipid, steroid lipid, PEGylated lipid, and anionic lipid.

In a specific embodiment of the present invention, the phospholipid in the lipid composition is selected from the group consisting of 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (4ME 16:0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyl oleoyl phosphatidylethanolamine (POPE), distearoyl phosphatidylethanolamine (DSPE), dipalmitoyl phosphatidylethanolamine (DPPE), dimyristoyl phosphatidylethanolamine (DMPE), 1-stearoyl-2-oleoyl-sn-glycero-3-phosphoethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine (LPE), and combinations thereof.

In a specific embodiment of the present invention, the steroid lipid in the lipid composition is selected from the group consisting of cholesterol, coprostanol, sitosterol, ergosterol, campesterol, stigmasterol, brassicasterol, tomatidine, ursolic acid, α-tocopherol, and combinations thereof.

In a specific embodiment of the present invention, the PEGylated lipid in the lipid composition is selected from the group consisting of non-targeting PEGylated lipid, targeting PEGylated lipid, and combinations thereof; preferably, the non-targeting PEGylated lipid is selected from the group consisting of polyethylene glycol-dipalmitoyl phosphatidylcholine (PEG-DPPC), polyethylene glycol-dimyristoyl glycerol (PEG-DMG), polyethylene glycol-distearoyl phosphatidylethanolamine (PEG-DSPE), polyethylene glycol-dioleoyl phosphatidylethanolamine (PEG-DOPE), polyethylene glycol-cholesterol (PEG-Chol), polyethylene glycol-diacylglycerol (PEG-DAG), polyethylene glycol-dialkoxypropyl (PEG-DAA), and combinations thereof; more preferably, the non-targeting PEGylated lipid is selected from the group consisting of polyethylene glycol 500-dipalmitoyl phosphatidylcholine, polyethylene glycol 2000-dipalmitoyl phosphatidylcholine, polyethylene glycol 500-distearoyl phosphatidylethanolamine, polyethylene glycol 2000-distearoyl phosphatidylethanolamine, polyethylene glycol 500-dioleoyl phosphatidylethanolamine, polyethylene glycol 2000-dioleoyl phosphatidylethanolamine, polyethylene glycol 500-dimyristoyl glycerol, polyethylene glycol 2000-dimyristoyl glycerol (PEG2k-DMG), and combinations thereof; preferably, the targeting PEGylated lipid is PEGylated lipid modified with folic acid or N-acetylgalactosamine.

In a specific embodiment of the present invention, the other type of cationic lipid in the lipid composition is selected from the group consisting of 1,2-dioleoyl-3-trimethylammonium-propane (methyl sulfate salt) (DOTAP), 1,2-dioctadeceneoxy-3-trimethylammonium propane chloride (DOTMA), 1-[2-(oleoyloxy)ethyl]-2-oleyl-3-(2-hydroxyethyl)imidazolinium chloride (DOTIM), 1,2-dioleyloxy-3-dimethylamino-propane (DODMA), 2,3-di(tetradecanoyloxy)propyl-trimethylazanium chloride (DMTAP), didecyldimethylammonium chloride (DDAC), didecyldimethylammonium bromide (DDAB), N,N-dioleyl-N,N-dimethylammonium chloride (DODAC), N-(4-carboxybenzyl)-N,N-dimethyl-2,3-bis(oleoyloxy)propan-1-aminium (DOBAQ), 3,6-bis {4-[bis(2-hydroxydodecyl)amino]butyl}piperazine-2,5-dione (cKK-E12), 1,1'-((2-(4-(2-((2-(bis(2-hydroxydodecyl)amino)ethyl)(2-hydroxydodecyl)amino)ethyl)pipera zin-1-yl)ethyl)azanediyl)bis(dodecan-2-ol) (C12-200), dilinoleylmethyl 4-(N,N-dimethylamino)butanoate (DLin-MC3-DMA), 1,2-dimyristoyl-sn-glycero-3-ethylphosphocholine (EPC), ((4-hydroxybutyl)azanediyl)bis(hexane-6,1-diyl)bis(2-hexyldecanoate) (ALC-0315), heptadecan-9-yl 8-((2-hydroxyethyl) (6-oxo-6-(undecyloxy) hexyl) amino) octanoate) (SM-102), and ((2-(2-hydroxyethoxy)ethyl)azanediyl)bis(hexane-6,1-diyl)bis(2-hexyldecanoate) (DHA-1).

In a specific embodiment of the present invention, the anionic lipid in the lipid composition is selected from the group consisting of 1,2-dioleoyl-sn-glycero-3-phosphate sodium salt (18:1 PA), 1,2-dimyristoyl-sn-glycero-3-phosphate sodium salt (14:0 PA), bis(monooleoylglycero)phosphate ammonium salt (18:1 BMP), and cardiolipin (CL).

In a specific embodiment of the present invention, the molecular weight of the polyethylene glycol (PEG) moiety of any PEGylated lipid is preferably 0.5 to 2 kDa, more preferably 500 Da, 1000 Da, or 2000 Da.

In a specific embodiment of the present invention, the PEGylated lipid in the lipid composition are a combination of a non-targeting PEGylated lipid and a targeting PEGylated lipid.

In a specific embodiment of the present invention, in the lipid composition, the molar percentage of PEGylated lipid in the total lipid is from 0.5% to 5%, preferably from 1% to 3%, more preferably 1.5%, 1.6%, 1.7%, 1.8%, or 1.9%; the molar percentage of cationic lipid in the total lipid is from 30% to 65%, preferably 35%, 40%, 45%, 46%, 47%, 48%, 49%, 50%, or 55%; the molar percentage of phospholipid in the total lipid is from 7.5% to 13%, preferably 8%, 9%, 10%, 11%, or 12%; and the molar percentage of steroid lipid in the total lipid is from 35% to 50%, preferably 38%, 39%, 40%, 41%, 42%, 43%, 44%, 45%, 46%, 47%, 48%, 49%, or 50%.

### An embodiment of the present invention:

A lipid pharmaceutical composition comprising any of the aforementioned lipid composition and a drug, wherein the drug is selected from the group consisting of nucleic acid drugs, small molecule drugs, peptide drugs, and protein drugs.

In a specific embodiment of the present invention, the aforementioned drug is a nucleic acid drug selected from the group consisting of DNA, RNA, antisense nucleic acid, plasmid, interfering nucleic acid, aptamer, antagomir, and ribozyme; wherein, the RNA is selected from the group consisting of mRNA, saRNA, circRNA, miRNA, and siRNA, and is preferably mRNA, miRNA, or siRNA.

In a specific embodiment of the present invention, the lipid pharmaceutical composition is used as a medicine, and the medicine is selected from the group consisting of the following: antineoplastics, anti-infectives, and vaccines; wherein, the anti-infectives are selected from the group consisting of antiparasitics, antibiotics, antifungals, and antivirals.

In a specific embodiment of the present invention, the lipid pharmaceutical composition is an LNP pharmaceutical composition, an LPP pharmaceutical composition, or a PNP pharmaceutical composition, preferably an LNP pharmaceutical composition, more preferably an LNP-nucleic acid pharmaceutical composition, and most preferably an LNP-mRNA pharmaceutical composition. Wherein, the "LNP pharmaceutical composition" refers to a pharmaceutical composition in the lipid nanoparticle form, the "LPP pharmaceutical composition" refers to a pharmaceutical composition in the lipopolyplex form, and the "PNP pharmaceutical composition" refers to a pharmaceutical composition in the polypeptide nanoparticle form; and wherein, the "LNP-nucleic acid pharmaceutical composition" refers to an LNP pharmaceutical composition loaded with nucleic acids, and the "LNP-mRNA pharmaceutical composition" refers to an LNP pharmaceutical composition loaded with mRNA.

In a specific embodiment of the present invention, the drug contained in the lipid pharmaceutical composition includes but is not limited to doxorubicin, mitoxantrone, camptothecin, cisplatin, bleomycin, cyclophosphamide, streptozotocin, actinomycin D, vincristine, vinblastine, cytosine arabinoside, anthracycline, nitrogen mustard, thiotepa, chlorambucil, rachelmycin, melphalan, carmustine, romustine, busulfan, dibromannitol, mitomycin C, cis-diammineplatinum(II) dichloride, methotrexate, 6-mercaptopurine, 6-thioguanine, cytarabine, 5-fluorouracil dacarbazine, dibucaine, chlorpromazine, propranolol, demorol, labetalol, clonidine, hydralazine, imipramine, amitriptyline, doxepin, phenytoin, diphenhydramine, chlorpheniramine, promethazine, gentamicin, ciprofloxacin, cefoxitin, miconazole, terconazole, econazole, isoconazole, butoconazole, clotrimazole, itraconazole, nystatin, naftifine, amphotericin B, antiparasitics, hormones, hormone antagonists, immunomodulators, neurotransmitter antagonists, glaucoma drugs, vitamins, tranquilizers, imaging agents, taxol, cytochalasin B, gramicidin D, ethidium bromide, emetine, mitomycin, etoposide, teniposide, colchicine, daunorubicin, dihydroxyanthracene-dione, mithramycin, 1-dihydrotestosterone, glucocorticoid, procaine, tetracaine, lidocaine, puromycin, and maytansinoid.

In a specific embodiment of the present invention, the drug in the lipid pharmaceutical composition is a nucleic acid drug, and the N/P ratio is (0.1-100):1, preferably (0.2-30):1, and more preferably (0.5-20):1.

### One embodiment of the present invention provides:

A lipid pharmaceutical composition formulation comprising any of the aforementioned lipid pharmaceutical composition and a working solution; wherein, the working solution is a pharmaceutically acceptable diluent or excipient, preferably selected from the group consisting of deionized water, ultrapure water, phosphate buffer, and saline, more preferably phosphate buffer or saline, and most preferably saline.

In a specific embodiment of the present invention, the ratio of the lipid pharmaceutical composition to the working solution in the aforementioned lipid pharmaceutical composition formulation is not particularly limited. Preferably, the ratio of the lipid pharmaceutical composition to the working solution is 0.05-20 g:100 mL, more preferably 0.1-10 g:100 mL, and most preferably 0.2-5 g:100 mL.

In a specific embodiment of the present invention, the preparation of the lipid pharmaceutical composition formulation comprises the following steps:
(1) equilibrating the lipid components in a diluent or excipient;
(2) adding the drug to the equilibrated mixture for complexing;
wherein, the equilibrating time is not particularly limited, preferably 0.1 to 12 h, more preferably 0.2 to 6 h, and most preferably 0.5 to 3 h; the complexing time is not particularly limited, preferably 0.1 to 12 h, more preferably 0.2 to 5 h, and most preferably 0.5to 2 h.

In a specific embodiment of the present invention, the preparation of an LNP-nucleic acid pharmaceutical composition comprises the following steps:
(1) dissolving lipid components in an organic solvent to obtain an organic phase solution;
(2) adding the nucleic acid drug to a buffer solution to obtain an aqueous phase solution;
(3) mixing the organic phase solution and the aqueous phase solution to obtain the LNP-nucleic acid pharmaceutical composition, followed by ultrafiltration and washing to remove the organic solvent and free molecules, and finally passing through a sterile filter for subsequent use;
wherein, the organic solvent is preferably methanol, ethanol, propanol, tert-butanol, acetonitrile, dimethyl sulfoxide, *N,N*-dimethylformamide, *N,N*-dimethylacetamide, *N*-methylpyrrolidone, or any mixture thereof; the buffer solution is preferably a citrate buffer solution, further preferably with a concentration of 5 to 80 mM and a pH of 2 to 6, and more preferably with a concentration of 10 to 50 mM and a pH of 3 to 5; the volume ratio of the organic phase solution to the aqueous phase solution is preferably 1:1-10, and more preferably 1:2 or 1:3.

In a specific embodiment of the present invention, the particle size of lipid nanoparticles is controlled using ultrasonic treatment, extrusion, or microfluidic devices, wherein the particle size ranges from 1 to 1000 nm, preferably from 20 to 500 nm, more preferably from 60 to 200 nm, and most preferably from 60 to 150 nm.

### EXAMPLES

The following specific examples provide a further description of the preparation of cationic lipids containing a disulfide bond, lipid compositions, lipid pharmaceutical compositions, and the bioactivity assays of LNP-nucleic acid pharmaceutical compositions. The specific examples are intended to provide a more detailed explanation of the present invention, without limiting the scope of protection of the present invention.

### Example 1: Preparation of Cationic Lipid N1

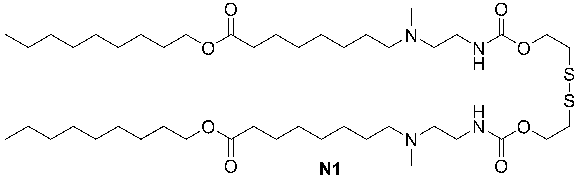

In cationic lipid **N1,** L₁ and L₂ are both -(CH₂)₂-NHC(=O)O-(CH₂)₂-, X₁ and X₂ are both 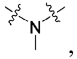 G₁ and G₂ are both linking bonds, L₃ and L₄ are both -OC(=O)-(CH₂)₇-, and R₁ and R₂ are both nonyl groups.

The preparation method is as follows:
Step a: **A1-1** (0.92 g, 6.0 mmol) was dissolved in 50 mL of anhydrous dichloromethane. *N,N*-diisopropylethylamine (DIPEA, 2.32 g, 18.0 mmol) and *N,N*'-disuccinimidyl carbonate (DSC, 3.84 g, 15.0 mmol) were added successively. The reaction was stirred at room temperature overnight. Upon completion of the reaction, the mixture was concentrated under reduced pressure. The residue was dissolved in 50 mL of dichloromethane and washed with saturated sodium bicarbonate solution (20 mL×3). The organic phase was dried over anhydrous sodium sulfate, filtered, and concentrated. The residue was purified by column chromatography to obtain **A1-2** (2.44 g).
Step b: **A1-2** (1.74 g, 4.0 mmol) was dissolved in 30 mL of dichloromethane. The Boc derivative of *N*-methylethylenediamine (**A1-3**, 1.74 g, 10.0 mmol) and triethylamine (TEA, 2.78 mL, 20.0 mmol) were added, and the reaction was carried out at room temperature for 2 h. Upon completion of the reaction, the mixture was washed with saturated saline solution. The organic phase was concentrated under reduced pressure, and the residue was treated with a trifluoroacetic acid/dichloromethane mixed solution (TFA/DCM, 1:1 v/v) to remove the Boc protecting group. The mixture was washed with purified water. The organic phase was dried over anhydrous sodium sulfate, filtered, and concentrated. The residue was purified by column chromatography to yield **A1-4** (1.09 g).
Step c: **A1-4** (0.71 g, 2.0 mmol) and DIPEA (0.77 g, 6.0 mmol) were dissolved in 20 mL of dichloromethane. **A1-5** (1.75 g, 5.0 mmol, obtained by esterification of 8-bromooctanoic acid and 1-nonanol) was added under stirring, and the reaction was carried out at room temperature overnight. Upon completion of the reaction, the mixture was poured into 40 mL of water and extracted three times with dichloromethane (20 mL×3). The organic phases were collected and combined, washed with saturated saline solution, dried over anhydrous sodium sulfate, filtered, and concentrated. The residue was purified by column chromatography to yield cationic lipid **N1** (1.21 g). ¹H NMR (400 MHz, CDCl₃) δ: 4.31 (t, 4H, -NHC(=O)OC*H*₂CH₂S-), 4.06 (t, 4H, -CH₂CH₂C*H*₂OC(=O)-), 3.32-3.15 (m, 4H, >NCH₂C*H*₂NHC(=O)O-), 2.93 (t, 4H, -NHC(=O)OCH₂C*H*₂S-), 2.45 (t, 4H, >NC*H*₂CH₂NHC(=O)O-), 2.38-2.24 (m, 8H, -CH₂CH₂C*H*₂N(CH₃)-, -CH₂CH₂C*H*₂C(=O)O-), 2.19 (s, 6H, >NC*H*₃), 1.67-1.59 (m, 8H, -CH₂C*H*₂CH₂N<, -CH₂C*H*₂CH₂OC(=O)-), 1.48-1.39 (m, 4H, -CH₂C*H*₂CH₂-), 1.35-1.23 (m, 36H, -C*H*₂CH₃, -CH₂C*H*₂CH₂-), 0.88 (t, 6H, -CH₂C*H*₃). MS (ESI): m/z = 891.4 ([M+H]⁺).

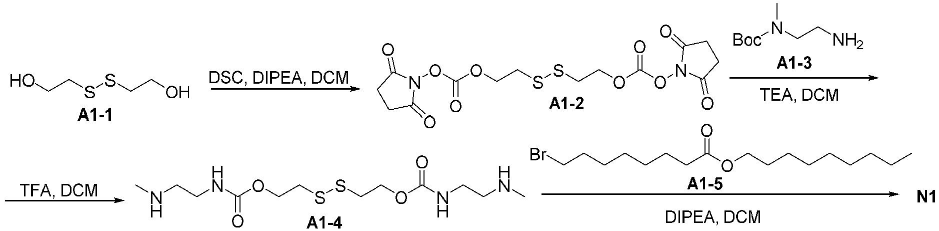

### Example 2: Preparation of Cationic Lipid N2

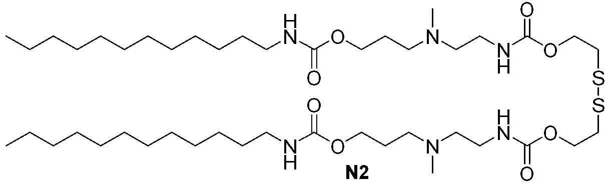

In cationic lipid **N2,** L₁ and L₂ are both -(CH₂)₂-NHC(=O)O-(CH₂)₂-, X₁ and X₂ are both 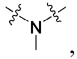 G₁ and G₂ are both linking bonds, L₃ and L₄ are both -NHC(=O)O-(CH₂)₃-, and R₁ and R₂ are both dodecyl groups.

The preparation method is as follows:
Step a: **A1-4** (2.12 g, 6.0 mmol) and DIPEA (2.32 g, 18.0 mmol) were dissolved in 50 mL of dichloromethane. The TBS derivative of 3-bromo-1-propanol (**A2-1**, 3.80 g, 15.0 mmol) was added under stirring, and the reaction was carried out at room temperature overnight. Upon completion of the reaction, the mixture was poured into 50 mL of water and extracted three times with dichloromethane (50 mL×3). The organic phases were collected and combined, washed with saturated saline solution, and the organic phase was concentrated under reduced pressure. The residue was dissolved in 30 mL of anhydrous tetrahydrofuran, and a 1M solution of tetra-n-butylammonium fluoride in tetrahydrofuran (TBAF/THF, 30 mL) was added. The mixture was stirred overnight to remove the TBS protection. After the reaction was completed, the mixture was concentrated under reduced pressure. The residue was dissolved in 50 mL of dichloromethane, washed with saturated ammonium chloride solution, and dried over anhydrous sodium sulfate. The mixture was filtered, concentrated, and the residue was purified by column chromatography to yield **A2-2** (1.96 g).
Step b: **A2-2** (1.88 g, 4.0 mmol) was dissolved in 50 mL of anhydrous dichloromethane. DIPEA (1.55 g, 12.0 mmol) and DSC (2.56 g, 10.0 mmol) were added successively, and the reaction was stirred at room temperature overnight. Upon completion of the reaction, the mixture was washed with saturated sodium bicarbonate solution (20 mL×3). The organic phase was concentrated under reduced pressure, and the residue was dissolved in 50 mL of anhydrous dichloromethane. Dodecylamine (**A2-3**, 1.85 g, 10.0 mmol) and TEA (2.78 mL, 20.0 mmol) were added, and the reaction was carried out at room temperature for 2 h. Upon completion of the reaction, the mixture was washed with saturated saline solution. The organic phase was dried over anhydrous sodium sulfate, filtered, and concentrated. The residue was purified by column chromatography to yield cationic lipid **N2** (3.08 g). ¹H NMR (400 MHz, CDCl₃) δ: 4.31 (t, 4H, -NHC(=O)OC*H*₂CH₂S-), 4.20-4.06 (m, 4H, -NHC(=O)OC*H*₂CH₂CH₂N<), 3.31-3.20 (m, 4H, >NCH₂C*H*₂NHC(=O)O-), 3.20-3.09 (m, 4H, -CH₂CH₂C*H*₂NHC(=O)O-), 2.98-2.88 (m, 4H, -NHC(=O)OCH₂C*H*₂S-), 2.51-2.37 (m, 8H, >NC*H*₂CH₂NHC(=O)O-, -CH₂CH₂C*H*₂N(CH₃)-), 2.20 (s, 6H, >NC*H*₃), 1.84-1.70 (m, 4H, -NHC(=O)OCH₂C*H*₂CH₂N<), 1.53-1.44 (m, 4H, -CH₂C*H*₂CH₂NHC(=O)O-), 1.31-1.23 (m, 36H, -C*H*₂CH₃, -CH₂C*H*₂CH₂-), 0.88 (t, 6H, -CH₂C*H*₃). MS (ESI): m/z = 893.4 ([M+H]⁺).

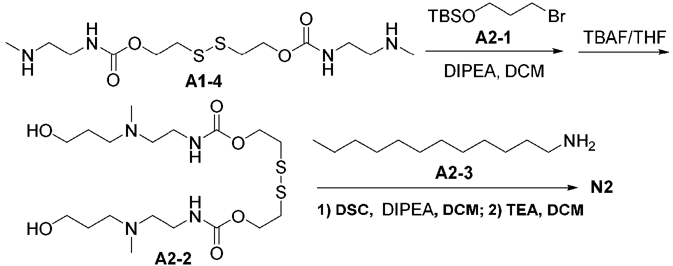

### Example 3: Preparation of Cationic Lipid N3

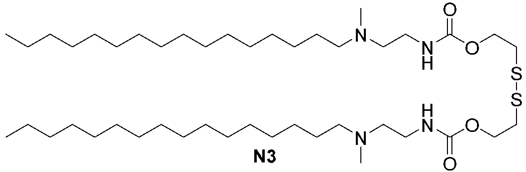

In cationic lipid **N3,** L₁ and L₂ are both -(CH₂)₂-NHC(=O)O-(CH₂)₂-, X₁ and X₂ are both 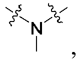 G₁ and G₂ are both linking bonds, L₃ and L₄ are both linking bonds, and R₁ and R₂ are both hexadecyl groups.

The preparation method is as follows:
**A1-4** (0.71 g, 2.0 mmol) and DIPEA (0.77 g, 6.0 mmol) were dissolved in 20 mL of dichloromethane. **A3-1** (1.53 g, 5.0 mmol) was added under stirring, and the reaction was carried out at room temperature overnight. Upon completion of the reaction, the mixture was poured into 40 mL of water and extracted three times with dichloromethane (20 mL×3). The organic phases were collected and combined, washed with saturated saline solution, and dried over anhydrous sodium sulfate. The mixture was filtered, concentrated, and the residue was purified by column chromatography to yield cationic lipid **N3** (1.20 g). ¹H NMR (400 MHz, CDCl₃) δ: 4.34 (t, 4H, -NHC(=O)OC*H*₂CH₂S-), 3.32-3.17 (m, 4H, >NCH₂C*H*₂NHC(=O)O-), 2.93 (t, 4H, -NHC(=O)OCH₂C*H*₂S-), 2.45 (t, 4H, >NC*H*₂CH₂NHC(=O)O-), 2.35 (t, 4H, -CH₂CH₂C*H*₂N(CH₃)-), 2.22 (s, 6H, >NC*H*₃), 1.67-1.60 (m, 4H, -CH₂CH₂C*H*₂N<), 1.40-1.19 (m, 52H, -C*H*₂CH₃, -CH₂C*H*₂CH₂-), 0.87 (t, 6H, -CH₂C*H*₃). MS (ESI): m/z = 803.4 ([M+H]⁺).

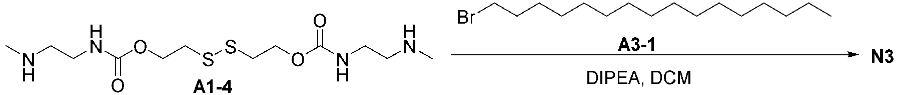

### Example 4: Preparation of Cationic Lipid N4

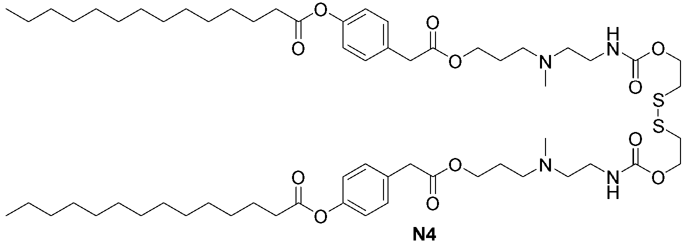

In cationic lipid **N4,** L₁ and L₂ are both -(CH₂)₂-NHC(=O)O-(CH₂)₂-, X₁ and X₂ are both 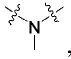 G₁ and G₂ are both linking bonds, L₃ and L₄ are both 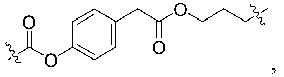 and R₁ and R₂ are both tridecyl groups.

The preparation method is as follows:
Step a: Under an argon atmosphere, a dichloromethane solution (50 mL) containing **A2-2** (2.83 g, 6.0 mmol), the TBS derivative of para-hydroxyphenylacetic acid (**A4-**1, 2.73 g, 15.0 mmol), and 4-dimethylaminopyridine (DMAP, 0.37 g, 3.0 mmol) was placed in an ice bath. A dichloromethane solution (50 mL) of DCC (3.71 g, 18.0 mmol) was slowly added dropwise. The reaction mixture was then warmed to room temperature and stirred for 24 h. Upon completion of the reaction, the precipitate was filtered off, and the filtrate was concentrated under reduced pressure. The residue was dissolved in 30 mL of anhydrous tetrahydrofuran, and 1M TBAF/THF solution (30 mL) was added. The mixture was stirred overnight to remove the TBS protection. After the reaction was completed, the mixture was concentrated under reduced pressure. The residue was dissolved in 50 mL of dichloromethane, washed with saturated ammonium chloride solution, and dried over anhydrous sodium sulfate. The mixture was filtered, concentrated, and the residue was purified by column chromatography to yield **A4-2** (3.39 g).
Step b: **A4-2** (2.96 g, 4.0 mmol) and the anhydride compound **A4-3** (5.27 g, 12.0 mmol, prepared using **S1-4** as the starting material) were dissolved in dichloromethane, and DMAP (0.20 g, 1.6 mmol) was added. The reaction was carried out at room temperature for 9 h. Upon completion of the reaction, the reaction solution was washed sequentially with 10% acetic acid aqueous solution and deionized water. The organic phase was dried over anhydrous sodium sulfate, filtered, and concentrated. The residue was purified by column chromatography to yield cationic lipid **N4** (4.02 g). ¹H NMR (400 MHz, CDCl₃) δ: 7.28 (d, 4H, -Ph-), 7.02 (d, 4H, -Ph-), 4.30 (t, 4H, -NHC(=O)OC*H*₂CH₂S-), 4.13 (t, 4H, -C(=O)OC*H*₂CH₂CH₂N<), 3.60 (s, 4H, -Ph-C*H*₂C(=O)O-), 3.24-3.14 (m, 4H, >NCH₂C*H*₂NHC(=O)O-), 2.91 (t, 4H, -NHC(=O)OCH₂C*H*₂S-), 2.53 (t, 4H, -C*H*₂C(=O)O-Ph-), 2.41 (t, 4H, >NC*H*₂CH₂NHC(=O)O-), 2.35 (t, 4H, -CH₂CH₂C*H*₂N(CH₃)-), 2.16 (s, 6H, >NC*H*₃), 1.79-1.69 (m, 8H, -C(=O)OCH₂C*H*₂CH₂N<, -C*H*₂CH₂C(=O)O-), 1.35-1.21 (m, 40H, -C*H*₂CH₃, -CH₂C*H*₂CH₂-), 0.88 (t, 6H, -CH₂C*H*₃). MS (ESI): m/z = 1159.5 ([M+H]⁺).

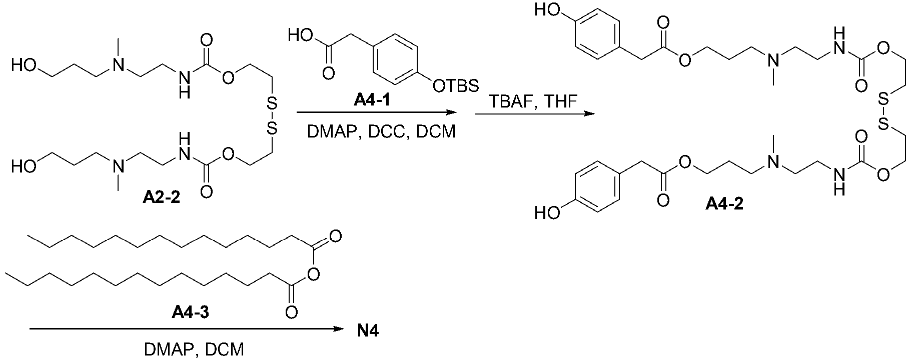

### Example 5: Preparation of Cationic Lipid N5

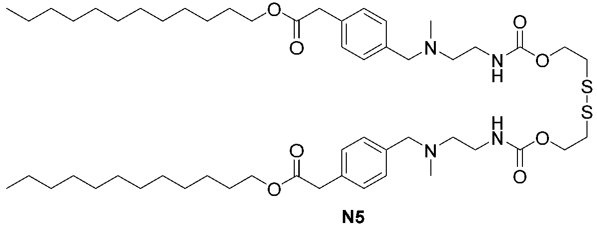

In cationic lipid N5, L₁ and L₂ are both -(CH₂)₂-NHC(=O)O-(CH₂)₂-, X₁ and X₂ are both 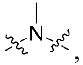 G₁ and G₂ are both linking bonds, L₃ and L₄ are both 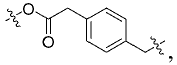 and R₁ and R₂ are both dodecyl groups.

The preparation method is as follows:
Step a: **A1-4** (1.42 g, 4.0 mmol) and DIPEA (1.55 g, 12.0 mmol) were dissolved in 30 mL of dichloromethane. **A5-1** (4-bromomethylphenylacetic acid, 2.29 g, 10.0 mmol) was added under stirring, and the reaction was carried out at room temperature overnight. Upon completion of the reaction, the mixture was poured into 30 mL of water and extracted three times with dichloromethane (30 mL×3). The organic phases were collected and combined, washed with saturated saline solution, and dried over anhydrous sodium sulfate. The mixture was filtered, concentrated, and the residue was purified by column chromatography to yield **A5-2** (2.06 g).
Step b: Under an argon atmosphere, a dichloromethane solution (20 mL) containing **A5-2** (1.30 g, 2.0 mmol), dodecanol (**A5-3**, 0.93 g, 5.0 mmol), and DMAP (0.10 g, 0.8 mmol) was placed in an ice bath. A dichloromethane solution (10 mL) of DCC (0.91 g, 4.4 mmol) was slowly added dropwise. The reaction mixture was then warmed to room temperature and stirred for 24 h. Upon completion of the reaction, the precipitate was filtered off, and the filtrate was concentrated under reduced pressure. The residue was purified by column chromatography to yield cationic lipid **N5** (1.64 g). ¹H NMR (400 MHz, CDCl₃) δ: 7.27 (d, 4H, -Ph-), 7.02 (d, 4H, -Ph-), 4.33 (t, 4H, -NHC(=O)OC*H*₂CH₂S-), 4.04 (t, 4H, -CH₂CH₂C*H*₂OC(=O)-), 3.63 (s, 4H, -OC(=O)C*H*₂-Ph-), 3.49 (s, 4H, -PhC*H*₂N<), 3.36-3.31 (m, 4H, >NCH₂C*H*₂NHC(=O)O-), 2.92 (t, 4H, -NHC(=O)OCH₂C*H*₂S-), 2.55 (t, 4H, >NC*H*₂CH₂NHC(=O)O-), 2.18 (s, 6H, >NC*H*₃), 1.68-1.59 (m, 4H, -CH₂C*H*₂CH₂OC(=O)-), 1.36-1.19 (m, 36H, -C*H*₂CH₃, -CH₂C*H*₂CH₂-), 0.86 (t, 6H, -CH₂C*H*₃). MS (ESI): m/z = 987.4 ([M+H]⁺).

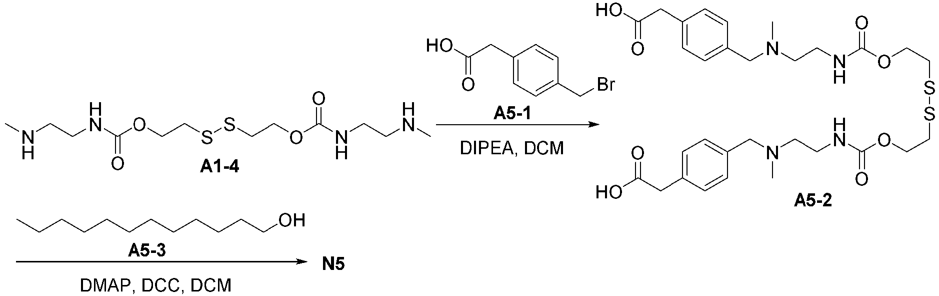

### Example 6: Preparation of Cationic Lipid N6

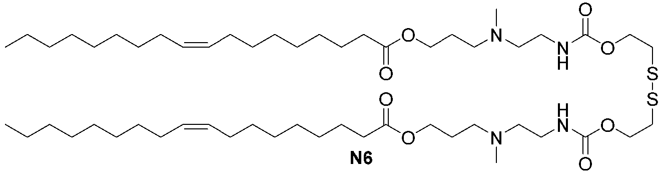

In cationic lipid **N6,** L₁ and L₂ are both -(CH₂)₂-NHC(=O)O-(CH₂)₂-, X₁ and X₂ are both 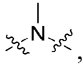 G₁ and G₂ are both linking bonds, L₃ and L₄ are both -C(=O)O-(CH₂)₃-, and R₁ and R₂ are both heptadec-8-enyl groups.

The preparation method is as follows:
Under an argon atmosphere, a dichloromethane solution (20 mL) containing **A2-2** (0.94 g, 2.0 mmol), oleic acid (**A6**-1, 1.41 g, 5.0 mmol), and DMAP (0.12 g, 1.0 mmol) was placed in an ice bath. A dichloromethane solution (15 mL) of DCC (1.24 g, 6.0 mmol) was slowly added dropwise. The reaction mixture was then warmed to room temperature and stirred for 24 h. Upon completion of the reaction, the precipitate was filtered off, and the filtrate was concentrated under reduced pressure. The residue was purified by column chromatography to yield cationic lipid **N6** (1.45 g). ¹H NMR (400 MHz, CDCl₃) δ: 5.38-5.31 (m, 4H, -CH₂-C*H*=C*H*-CH₂-), 4.31 (t, 4H, -NHC(=O)OC*H*₂CH₂S-), 4.12 (t, 4H, -C(=O)OC*H*₂CH₂CH₂N<), 3.29 (s, 4H, >NCH₂C*H*₂NHC(=O)O-), 2.93 (t, 4H, -NHC(=O)OCH₂C*H*₂S-), 2.58-2.41 (m, 8H, >NC*H*₂CH₂NHC(=O)O-, -CH₂CH₂C*H*₂N(CH₃)-), 2.34-2.22 (m, 10H, >NC*H*₃, -C*H*₂C(=O)O-), 2.04-1.98 (m, 8H, -C*H*₂-CH=CH-C*H*₂-), 1.81 (s, 4H, -C(=O)OCH₂C*H*₂CH₂N<), 1.64-1.59 (m, 4H, -C*H*₂CH₂C(=O)O-), 1.33-1.25 (m, 40H, -C*H*₂CH₃, -CH₂C*H*₂CH₂-), 0.88 (t, 6H, -CH₂C*H*₃). MS (ESI): m/z = 999.5 ([M+H]⁺).

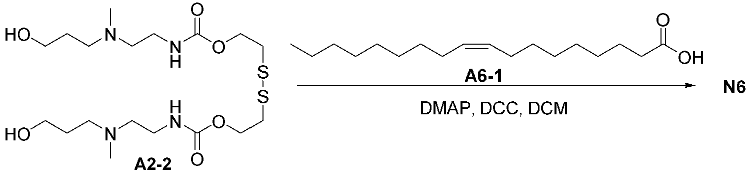

### Example 7: Preparation of Cationic Lipid N7

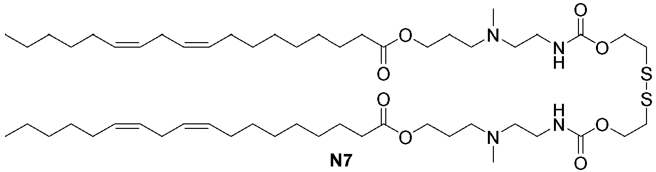

In cationic lipid **N7,** L₁ and L₂ are both -(CH₂)₂-NHC(=O)O-(CH₂)₂-, X₁ and X₂ are both 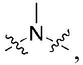 G₁ and G₂ are both linking bonds, L₃ and L₄ are both -C(=O)O-(CH₂)₃-, and R₁ and R₂ are both heptadeca-8,11-dienyl groups.

The preparation method is as follows:
Under an argon atmosphere, a dichloromethane solution (20 mL) containing **A2-2** (0.94 g, 2.0 mmol), linoleic acid (**A7-1**, 1.40 g, 5.0 mmol), and DMAP (0.12 g, 1.0 mmol) was placed in an ice bath. A dichloromethane solution (15 mL) of DCC (1.24 g, 6.0 mmol) was slowly added dropwise. The reaction mixture was then warmed to room temperature and stirred for 24 h. Upon completion of the reaction, the precipitate was filtered off, and the filtrate was concentrated under reduced pressure. The residue was purified by column chromatography to yield cationic lipid **N7** (1.53 g). ¹H NMR (400 MHz, CDCl₃) δ: 5.46-5.33 (m, 8H, -CH₂-C*H*=C*H*-CH₂-), 4.31 (t, 4H, -NHC(=O)OC*H*₂CH₂S-), 4.04 (t, 4H, -C(=O)OC*H*₂CH₂CH₂N<), 3.36-3.31 (m, 4H, >NCH₂C*H*₂NHC(=O)O-), 2.93 (t, 4H, -NHC(=O)OCH₂C*H*₂S-), 2.79-2.73 (m, 4H, -CH=CH-C*H*₂-CH=CH-), 2.60-2.40 (m, 8H, >NC*H*₂CH₂NHC(=O)O-, -CH₂CH₂C*H*₂N(CH₃)-), 2.34 (s, 6H, >NC*H*₃), 2.32 (t, 4H, -C*H*₂C(=O)O-), 2.05-1.99 (m, 8H, -C*H*₂-CH=CH-CH₂-CH=CH-C*H*₂-), 1.74-1.57 (m, 8H, -C(=O)OCH₂C*H*₂CH₂N<, -C*H*₂CH₂C(=O)O-), 1.39-1.20 (m, 28H, -C*H*₂CH₃, -CH₂C*H*₂CH₂-), 0.88 (t, 6H, -CH₂C*H*₃). MS (ESI): m/z = 995.5 ([M+H]⁺).

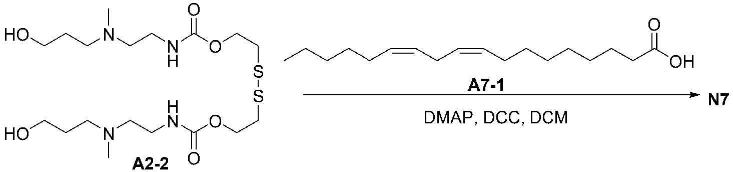

### Example 8: Preparation of Cationic Lipid N8

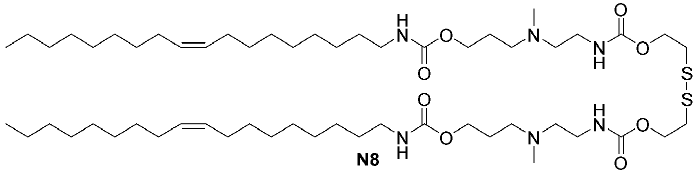

In cationic lipid N8, L₁ and L₂ are both -(CH₂)₂-NHC(=O)O-(CH₂)₂-, X₁ and X₂ are both 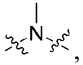 G₁ and G₂ are both linking bonds, L₃ and L₄ are both -NHC(=O)O-(CH₂)₃-, and R₁ and R₂ are both octadec-9-enyl groups.

The preparation method is as follows:
A2-2 (0.94 g, 2.0 mmol) was dissolved in 20 mL of anhydrous dichloromethane, and DIPEA (1.55 g, 12.0 mmol) and DSC (1.23 g, 4.8 mmol) were added successively. The reaction was stirred at room temperature overnight. Upon completion of the reaction, the mixture was washed with saturated sodium bicarbonate (10 mL×3). The organic phase was concentrated under reduced pressure, and the residue was dissolved in 20 mL of anhydrous dichloromethane. Oleylamine (**A8-1**, 1.34 g, 5.0 mmol) and TEA (1.39 mL, 10.0 mmol) were added, and the reaction was carried out at room temperature for 2 h. Upon completion of the reaction, the mixture was washed with saturated saline solution. The organic phase was dried over anhydrous sodium sulfate, filtered, concentrated, and the residue was purified by column chromatography to obtain cationic lipid **N8** (1.47 g). ¹H NMR (400 MHz, CDCl₃) δ: 5.37-5.30 (m, 4H, -CH₂-C*H*=C*H*-CH₂-), 4.31 (t, 4H, -NHC(=O)OC*H*₂CH₂S-), 4.23-4.17 (m, 4H, -NHC(=O)OC*H*₂CH₂CH₂N<), 3.31-3.26 (m, 4H, >NCH₂C*H*₂NHC(=O)O-), 3.12-3.08 (m, 4H, -CH₂CH₂C*H*₂NHC(=O)O-), 2.90-2.85 (m, 4H, -NHC(=O)OCH₂C*H*₂S-), 2.53-2.38 (m, 8H, >NC*H*₂CH₂NHC(=O)O-, -CH₂CH₂C*H*₂N(CH₃)-), 2.32 (s, 6H, >NC*H*₃), 2.01-1.96 (m, 8H, -C*H*₂-CH=CH-C*H*₂-), 1.82-1.69 (m, 4H, -NHC(=O)OCH₂C*H*₂CH₂N<), 1.53-1.43 (m, 4H, -CH₂C*H*₂CH₂NHC(=O)O-), 1.33-1.18 (m, 44H, -C*H*₂CH₃, -CH₂C*H*₂CH₂-), 0.86 (t, 6H, -CH₂C*H*₃). MS (ESI): m/z = 1057.6 ([M+H]⁺).

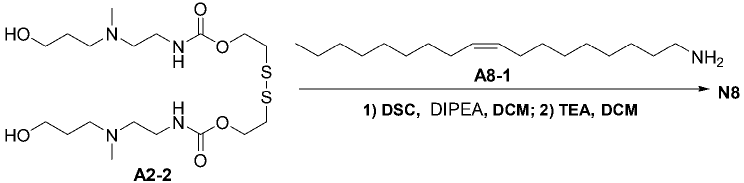

### Example 9: Preparation of Cationic Lipid N9

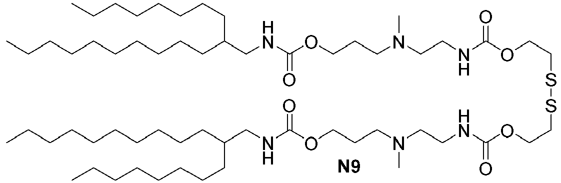

In cationic lipid **N9,** L₁ and L₂ are both -(CH₂)₂-NHC(=O)O-(CH₂)₂-, X₁ and X₂ are both 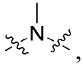 G₁ and G₂ are both linking bonds, L₃ and L₄ are both -NHC(=O)O-(CH₂)₃-, and R₁ and R₂ are both 2-octyldodecyl groups.

The preparation method is as follows:
**A2-2** (0.94 g, 2.0 mmol) was dissolved in 20 mL of anhydrous dichloromethane, and DIPEA (1.55 g, 12.0 mmol) and DSC (1.23 g, 4.8 mmol) were added successively. The reaction was stirred at room temperature overnight. Upon completion of the reaction, the mixture was washed three times with saturated sodium bicarbonate solution (10 mL×3). The organic phase was concentrated under reduced pressure, and the residue was dissolved in 20 mL of anhydrous dichloromethane. 2-Octyldodecylamine (**A9-1**, 1.49 g, 5.0 mmol) and TEA (1.39 mL, 10.0 mmol) were added, and the reaction was carried out at room temperature for 2 h. Upon completion of the reaction, the mixture was washed with saturated saline solution. The organic phase was dried over anhydrous sodium sulfate, filtered, concentrated, and the residue was purified by column chromatography to obtain cationic lipid **N9** (1.50 g). ¹H NMR (400 MHz, CDCl₃) δ: 4.35 (t, 4H, -NHC(=O)OC*H*₂CH₂S-), 4.26-4.20 (m, 4H, -NHC(=O)OC*H*₂CH₂CH₂N<), 3.31-3.18 (m, 4H, >NCH₂C*H*₂NHC(=O)O-), 3.20-3.03 (m, 4H, >CHC*H*₂NHC(=O)O-), 2.94 (t, 4H, -NHC(=O)OCH₂C*H*₂S-), 2.46 (t, 4H, >NC*H*₂CH₂NHC(=O)O-), 2.36 (t, 4H, -CH₂CH₂C*H*₂N(CH₃)-), 2.20 (s, 6H, >NC*H*₃), 2.02-1.95 (m, 2H, >CH-), 1.71-1.20 (m, 68H, -CH₂C*H*₂CH<, -C*H*₂CH₃, -CH₂C*H*₂CH₂-), 0.87 (t, 12H, -CH₂C*H*₃). MS (ESI): m/z = 1117.7 ([M+H]⁺).

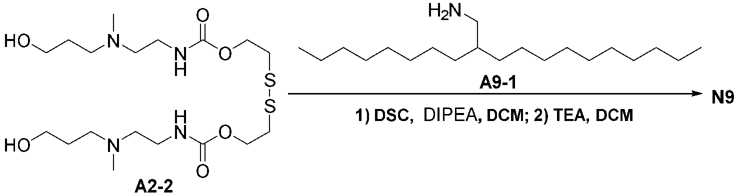

### Example 10: Preparation of Cationic Lipid N10

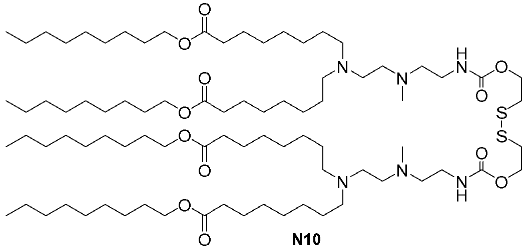

In cationic lipid **N10,** L₁ and L₂ are both -(CH₂)₂-NHC(=O)O-(CH₂)₂-, X₁ and X₂ are both 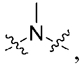 G₁ and G₂ are both 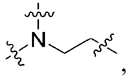 L₃ and L₄ are both -OC(=O)-(CH₂)₇-, and R₁ and R₂ are both nonyl groups.

The preparation method is as follows:
Step a: **A1-2** (1.74 g, 4.0 mmol) was dissolved in 30 mL of dichloromethane, and the Boc derivative of 2,2'-diamino-*N*-methyldiethylamine (**A10-1,** 2.17 g, 10.0 mmol) and TEA (2.78 mL, 20.0 mmol) were added. The reaction was stirred at room temperature for 2 h. After completion of the reaction, the mixture was washed with saturated saline solution. The organic phase was concentrated under reduced pressure, and the residue was treated with a TFA/DCM mixed solution (1:1 v/v) to remove the Boc protecting group, followed by washing with purified water. The organic phase was dried over anhydrous sodium sulfate, filtered, concentrated, and the residue was purified by column chromatography to yield **A10-2** (1.49 g).
Step b: **A10-2** (0.88 g, 2.0 mmol) and DIPEA (1.55 g, 12.0 mmol) were dissolved in 40 mL of dichloromethane. **A1-5** (3.49 g, 10.0 mmol) was added under stirring, and the reaction was carried out at room temperature overnight. After the reaction was completed, the mixture was poured into 80 mL of water, and then extracted three times with dichloromethane (40 mL×3). The organic phases were collected and combined, washed with saturated saline solution, dried over anhydrous sodium sulfate, filtered, concentrated, and the residue was purified by column chromatography to obtain cationic lipid **N10** (1.79 g). ¹H NMR (400 MHz, CDCl₃) δ: 4.32 (t, 4H, -NHC(=O)OC*H*₂CH₂S-), 4.05 (t, 8H, -C*H*₂OC(=O)-), 3.30-3.16 (m, 4H, >NCH₂C*H*₂NHC(=O)O-), 2.90 (t, 4H, -NHC(=O)OCH₂C*H*₂S-), 2.69-2.38 (m, 20H, >NC*H*₂-), 2.31 (t, 8H, -OC(=O)C*H*₂-), 2.23 (s, 6H, >NC*H*₃), 1.71-1.52 (m, 24H, -C*H*₂CH₂OC(=O)CH₂C*H*₂-, -CH₂C*H*₂CH₂N<), 1.43-1.19 (m, 72H, -C*H*₂CH₃, -CH₂C*H*₂CH₂-), 0.87 (t, 12H, -CH₂C*H*₃). MS (ESI): m/z = 1514.0 ([M+H]⁺).

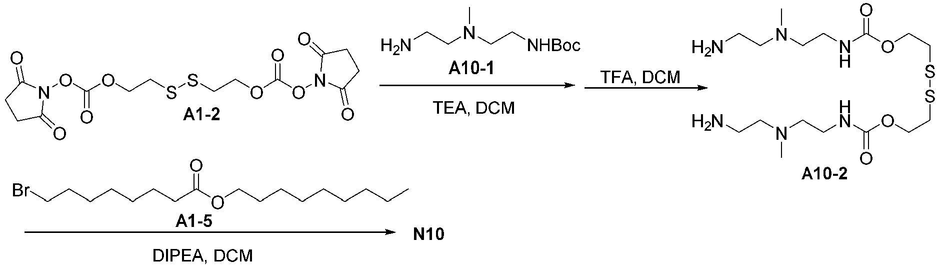

### Example 11: Preparation of Cationic Lipid N11

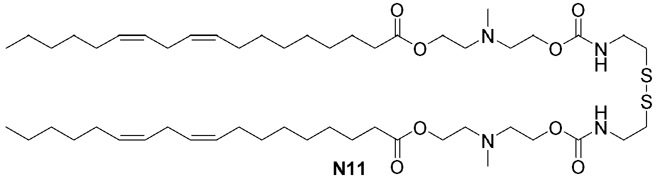

In cationic lipid **N11,** L₁ and L₂ are both -(CH₂)₂-OC(=O)NH-(CH₂)₂-, X₁ and X₂ are both 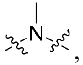 G₁ and G₂ are both linking bonds, L₃ and L₄ are both -C(=O)O-(CH₂)₂-, and R₁ and R₂ are both heptadeca-8,11-dienyl groups.

The preparation method is as follows:
Step a: The TBS derivative of N-methyldiethanolamine (**A11-1,** 1.86 g, 8.0 mmol) was dissolved in 50 mL of anhydrous dichloromethane, followed by the addition of DIPEA (1.55 g, 12.0 mmol) and DSC (2.46 g, 9.6 mmol). The reaction was stirred at room temperature overnight. After the reaction was completed, the mixture was washed with saturated sodium bicarbonate solution (20 mL×3). The organic phase was concentrated under reduced pressure, and the residue was dissolved in 50 mL of anhydrous dichloromethane. Cystamine dihydrochloride (**A11-2,** 0.72 g, 3.2 mmol) and TEA (3.90 mL, 28.0 mmol) were added, and the reaction was stirred at room temperature for 2 h. After completion of the reaction, the mixture was washed with saturated saline solution, and the organic phase was dried over anhydrous sodium sulfate, filtered, and concentrated. The residue was treated with 30 mL of anhydrous tetrahydrofuran, followed by the addition of 1M TBAF/THF solution (30 mL), and stirred overnight to remove the TBS protection. After completion of the reaction, the mixture was concentrated under reduced pressure, and the residue was dissolved in 50 mL of dichloromethane. The solution was washed with saturated ammonium chloride solution, dried over anhydrous sodium sulfate, filtered, and concentrated. The residue was purified by column chromatography to yield **A11-3** (1.08 g).
Step b: Under an argon atmosphere, a solution containing **A11-3** (0.89 g, 2.0 mmol), linoleic acid (**A7-1**, 1.40 g, 5.0 mmol), and DMAP (0.12 g, 1.0 mmol) in dichloromethane (20 mL) was placed in an ice bath, and a solution of DCC (1.24 g, 6.0 mmol) in dichloromethane (15 mL) was added dropwise. Subsequently, the reaction mixture was warmed to room temperature and stirred for 24 h. After the reaction was completed, the mixture was filtered to remove the precipitate, and the filtrate was concentrated under reduced pressure. The residue was purified by column chromatography to obtain cationic lipid **N11** (1.61 g). ¹H NMR (400 MHz, CDCl₃) δ: 5.40-5.27 (m, 8H, -CH₂-C*H*=C*H*-CH₂-), 4.23-4.09 (m, 8H, -C*H*₂CH₂N(CH₃)CH₂C*H*₂-), 3.55-3.45 (m, 4H, -OC(=O)NHC*H*₂CH₂SS-), 2.87-2.74 (m, 8H, -OC(=O)NHCH₂C*H*₂SS-, -CH=CH-C*H*₂-CH=CH-), 2.74-2.65 (m, 8H, -CH₂C(=O)OCH₂C*H*₂N<, >NC*H*₂CH₂OC(=O)NH-), 2.35 (s, 6H, -N(C*H*₃)-), 2.31 (t, 4H, -CH₂C*H*₂C(=O)O-), 2.10-1.96 (m, 8H, -C*H*₂-CH=CH-C*H*₂-), 1.63-1.56 (m, 4H, -C*H*₂CH₂C(=O)O-), 1.37-1.25 (m, 28H, -C*H*₂CH₃, -CH₂C*H*₂CH₂-), 0.89 (t, 6H, -CH₂C*H*₃). MS (ESI): m/z = 967.4 ([M+H]⁺).

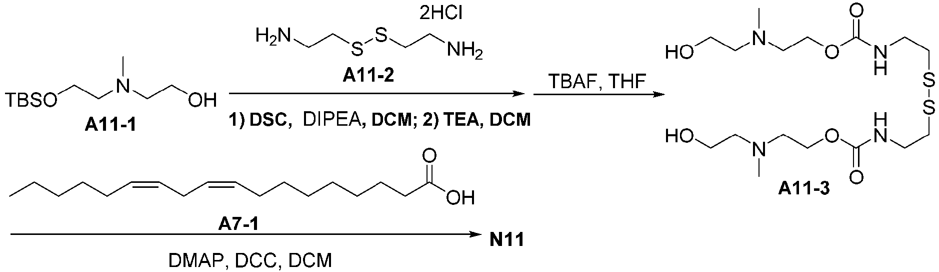

### Example 12: Preparation of Cationic Lipid N12

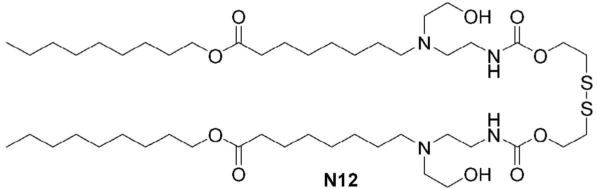

In cationic lipid **N12,** L₁ and L₂ are both -(CH₂)₂-NHC(=O)O-(CH₂)₂-, X₁ and X₂ are both 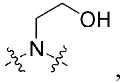 G₁ and G₂ are both linking bonds, L₃ and L₄ are both -OC(=O)-(CH₂)₇-, and R₁ and R₂ are both nonyl groups.

The preparation method is as follows:
Step a: **A1-2** (1.74 g, 4.0 mmol) in was dissolved in 30 mL of dichloromethane. The derivative of diethanolamine containing one Boc-protected amino group and one TBS-protected hydroxyl group (**A12-1,** 3.19 g, 10.0 mmol) and TEA (2.78 mL, 20.0 mmol) were added. The reaction was stirred at room temperature for 2 h. After completion of the reaction, the mixture was washed with saturated saline solution, and the organic phase was concentrated under reduced pressure. The residue was treated with a TFA/DCM mixed solution (1:1 v/v) to remove the Boc protecting group, followed by washing with purified water. The organic phase was dried over anhydrous sodium sulfate, filtered, and concentrated. The residue was purified by column chromatography to obtain **A12-2** (1.88 g).
Step b: **A12-2** (1.29 g, 2.0 mmol) and DIPEA (0.77 g, 6.0 mmol) were dissolved in 20 mL of dichloromethane. **A1-5** (1.75 g, 5.0 mmol) was added under stirring, and the reaction was carried out at room temperature overnight. After the reaction was completed, the mixture was poured into 40 mL of water and extracted three times with dichloromethane (20 mL×3). The organic phases were collected and combined, washed with saturated saline solution, dried over anhydrous sodium sulfate, filtered, and concentrated. The residue was dissolved in 30 mL of anhydrous tetrahydrofuran, and 1M TBAF/THF solution (30 mL) was added. The reaction was stirred overnight to remove the TBS protection. After completion of the reaction, the mixture was concentrated under reduced pressure, and the residue was dissolved in 50 mL of dichloromethane. The solution was washed with saturated ammonium chloride solution, dried over anhydrous sodium sulfate, filtered, and concentrated. The residue was purified by column chromatography to obtain cationic lipid **N12** (1.34 g). ¹H NMR (400 MHz, CDCl₃) δ: 4.36 (t, 4H, -NHC(=O)OC*H*₂CH₂S-), 4.07 (t, 4H, -C*H*₂OC(=O)-), 3.62 (t, 4H, >NCH₂C*H*₂OH), 3.34-3.29 (m, 4H, >NCH₂C*H*₂NHC(=O)O-), 2.93 (t, 4H, -NHC(=O)OCH₂C*H*₂S-), 2.66-2.48 (m, 12H, >NC*H*₂-), 2.30 (t, 4H, -OC(=O)C*H*₂-), 1.69-1.52 (m, 12H, -C*H*₂CH₂OC(=O)CH₂C*H*₂-, -CH₂C*H*₂CH₂N<), 1.40-1.17 (m, 36H, -C*H*₂CH₃, -CH₂C*H*₂CH₂-), 0.86 (t, 6H, -CH₂C*H*₃). MS (ESI): m/z = 951.5 ([M+H]⁺).

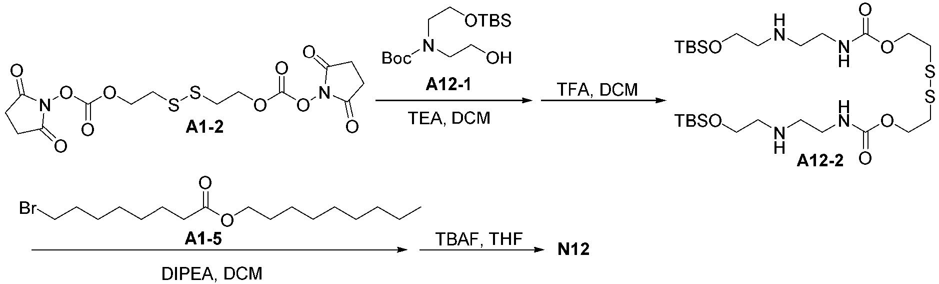

### Example 13: Preparation of Cationic Lipid N13

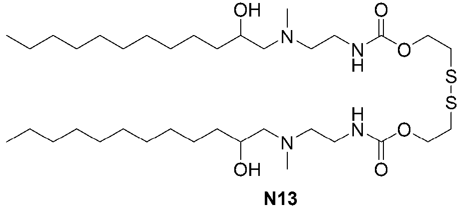

In cationic lipid **N13,** L₁ and L₂ are both -(CH₂)₂-NHC(=O)O-(CH₂)₂-, X₁ and X₂ are both 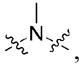 G₁ and G₂ are both linking bonds, L₃ and L₄ are both -CH(OH)CH₂-, and R₁ and R₂ are both decyl groups.

The preparation method is as follows:
Epoxide compound **A13-1** (4.4 mmol, 0.81 g) was dissolved in ethanol, and **A1-4** (2.0 mmol, 0.71 g) was added. The reaction was stirred at 60°C for 10 h. Upon completion of the reaction, the mixture was diluted with dichloromethane and washed with saturated sodium chloride solution. The organic phase was separated, dried over anhydrous sodium sulfate, filtered, and concentrated. The residue was purified by column chromatography to obtain cationic lipid **N13** (1.11 g). ¹H NMR (400 MHz, CDCl₃) δ: 4.34 (t, 4H, -NHC(=O)OC*H*₂CH₂S-), 3.65-3.55 (m, 2H, >C*H*OH), 3.36-3.23 (m, 4H, >NCH₂C*H*₂NHC(=O)O-), 2.95 (t, 4H, -NHC(=O)OCH₂C*H*₂S-), 2.56-2.43 (m, 8H, -C*H*₂N(CH₃)C*H*₂-), 2.32 (s, 6H, >NC*H*₃), 1.58-1.19 (m, 36H, -C*H*₂CH₃, -CH₂C*H*₂CH₂-), 0.86 (t, 6H, -CH₂C*H*₃). MS (ESI): m/z = 723.3 ([M+H]⁺).

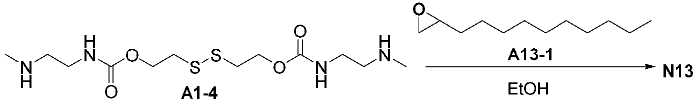

### Example 14: Preparation of Cationic Lipid N14

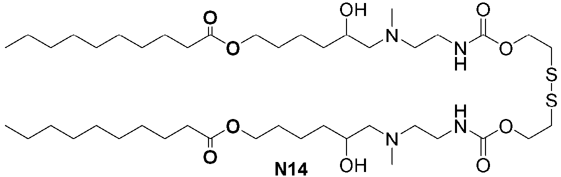

In cationic lipid **N14,** L₁ and L₂ are both -(CH₂)₂-NHC(=O)O-(CH₂)₂-, X₁ and X₂ are both 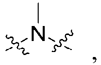 G₁ and G₂ are both linking bonds, L₃ and L₄ are both -C(=O)O-(CH₂)₄-CH(OH)CH₂-, and R₁ and R₂ are both nonyl groups.

The preparation method is as follows:
Epoxide compound **A14-1** (4.4 mmol, 1.19 g, synthesized according to the method disclosed in WO2023126006A1) was dissolved in ethanol, and **A1-4** (2.0 mmol, 0.71 g) was added. The reaction was stirred at 60°C for 10 h. Upon completion of the reaction, the mixture was diluted with dichloromethane and washed with saturated sodium chloride solution. The organic phase was separated, dried over anhydrous sodium sulfate, filtered, and concentrated. The residue was purified by column chromatography to obtain cationic lipid **N14** (1.42 g). ¹H NMR (400 MHz, CDCl₃) δ: 4.35 (t, 4H, -NHC(=O)OC*H*₂CH₂S-), 4.04 (t, 4H, -C(=O)OC*H*₂CH₂-), 3.67-3.58 (m, 2H, >CHOH), 3.36-3.21 (m, 4H, >NCH₂C*H*₂NHC(=O)O-), 2.94 (t, 4H, -NHC(=O)OCH₂C*H*₂S-), 2.58-2.42 (m, 8H, -C*H*₂N(CH₃)C*H*₂-), 2.33 (s, 6H, >NC*H*₃), 2.30 (t, 4H, -CH₂C*H*₂C(=O)O-), 1.67-1.17 (m, 40H, -C*H*₂CH₃, -CH₂C*H*₂CH₂-), 0.87 (t, 6H, -CH₂C*H*₃). MS (ESI): m/z = 895.4 ([M+H]⁺).

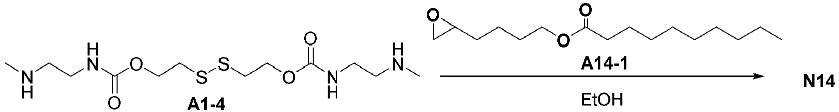

### Example 15: Preparation of Cationic Lipid N15

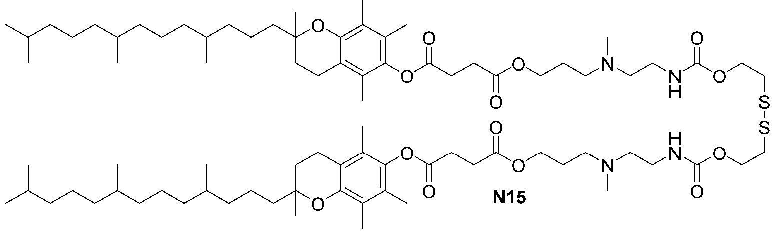

In cationic lipid **N15,** L₁ and L₂ are both -(CH₂)₂-NHC(=O)O-(CH₂)₂-, X₁ and X₂ are both 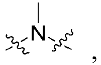 G₁ and G₂ are both linking bonds, L₃ and L₄ are both -OC(=O)-(CH₂)₂-C(=O)O-(CH₂)₃-, and R₁ and R₂ are both

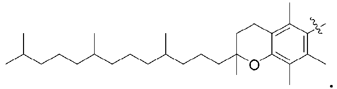

The preparation method is as follows:
Under an argon atmosphere, a dichloromethane solution (30 mL) containing **A2-2** (0.94 g, 2.0 mmol), D-α-tocopheryl succinate (**A15-1,** 2.66 g, 5.0 mmol), and DMAP (0.12 g, 1.0 mmol) was placed in an ice bath. A dichloromethane solution (15 mL) of DCC (1.13 g, 5.5 mmol) was then slowly added dropwise. Subsequently, the reaction mixture was warmed to room temperature and stirred for 24 h. Upon completion of the reaction, the mixture was filtered to remove the precipitate, and the filtrate was concentrated under reduced pressure. The residue was purified by column chromatography to obtain cationic lipid **N15** (2.09 g). ¹H NMR (400 MHz, CDCl₃) δ: 4.36 (t, 4H, -NHC(=O)OC*H*₂CH₂S-), 4.09 (t, 4H, -CH₂C(=O)OC*H*₂-), 3.36-3.30 (m, 4H, >NCH₂C*H*₂NHC(=O)O-), 2.93 (t, 4H, -NHC(=O)OCH₂C*H*₂S-), 2.67-2.53 (m, 16H, -N(CH₃)C*H*₂CH₂NHC(=O)O-, -OC(=O)C*H*₂C*H*₂C(=O)O-, -CH₂C*H*₂-Ar-), 2.37 (t, 4H, -CH₂CH₂C*H*₂N(CH₃)-), 2.24 (s, 6H, >NC*H*₃), 2.09-2.00 (m, 18H, -Ar-C*H*₃), 1.88-1.74 (m, 4H, -C*H*₂CH₂Ar-), 1.66-1.01 (m, 52H, >C*H*(C*H*₂)₃C*H*<, >C*H*(C*H*₂)₃C<, >C(C*H*₃)O-), 0.89-0.86 (m, 24H, (C*H*₃)₂CH-, -CH₂C(C*H*₃)CH₂-). MS (ESI): m/z = 1495.8 ([M+H]⁺).

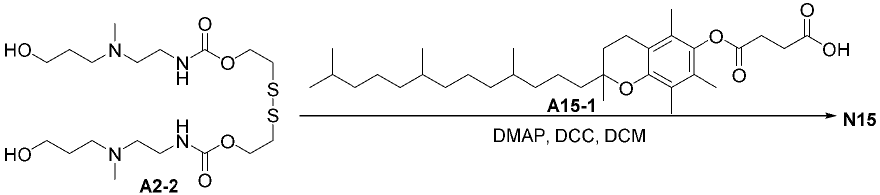

### Example 16: Preparation of Cationic Lipid N16

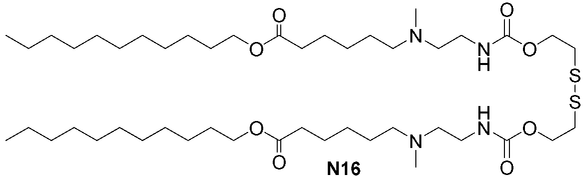

In cationic lipid **N16,** L₁ and L₂ are both -(CH₂)₂-NHC(=O)O-(CH₂)₂-, X₁ and X₂ are both 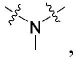 G₁ and G₂ are both linking bonds, L₃ and L₄ are both -OC(=O)-(CH₂)₅-, and R₁ and R₂ are both undecyl groups.

Referring to the preparation method of cationic lipid **N1** in Example 1, compound **A1-5** was replaced with compound **A16-1** ( 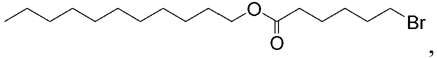 obtained via an esterification reaction between undecanol and 6-bromohexanoic acid), with the rest conditions unchanged, to obtain cationic lipid **N16** (1.30 g). ¹H NMR (400 MHz, CDCl₃) δ: 4.32 (t, 4H, -NHC(=O)OC*H*₂CH₂S-), 4.05 (t, 4H, -CH₂CH₂C*H*₂OC(=O)-), 3.33-3.25 (m, 4H, >NCH₂C*H*₂NHC(=O)O-), 2.93 (t, 4H, -NHC(=O)OCH₂C*H*₂S-), 2.54 (t, 4H, >NC*H*₂CH₂NHC(=O)O-), 2.36 (t, 4H, -CH₂CH₂C*H*₂N(CH₃)-), 2.27 (t, 4H, -CH₂CH₂C*H*₂C(=O)O-), 2.23 (s, 6H, >NC*H*₃), 1.69-1.52 (m, 12H, -CH₂C*H*₂CH₂N<, -C*H*₂CH₂OC(=O)CH₂C*H*₂-), 1.35-1.19 (m, 36H, -C*H*₂CH₃, -CH₂C*H*₂CH₂-), 0.86 (t, 6H, -CH₂C*H*₃). MS (ESI): m/z = 891.4 ([M+H]⁺).

### Example 17: Preparation of Cationic Lipid N17

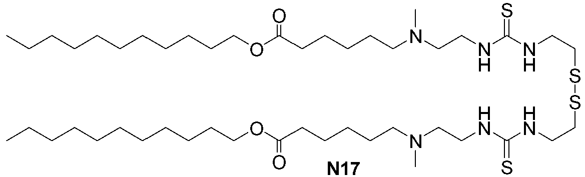

In cationic lipid **N17,** L₁ and L₂ are both -(CH₂)₂-NHC(=S)NH-(CH₂)₂-, X₁ and X₂ are both 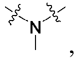 G₁ and G₂ are both linking bonds, L₃ and L₄ are both -OC(=O)-(CH₂)₅-, and R₁ and R₂ are both undecyl groups.

The preparation method is as follows:
Step a: **A1-3** (2.09 g, 12.0 mmol) was dissolved in 50 mL of tetrahydrofuran, followed by the addition of TEA (2.50 mL, 18.0 mmol). The solution was placed in an ice bath, and carbon disulfide (CS₂, 1.19 g, 15.6 mmol) was added dropwise. Subsequently, the reaction solution was warmed to room temperature. The reaction was stirred for 12 h, and then DMAP (0.44 g, 3.6 mmol) was added. The mixture was placed in an ice bath, and di-tert-butyl dicarbonate ((Boc)₂O, 3.40 g, 15.6 mmol) was added. The reaction was stirred at room temperature for an additional 3 h. Upon completion of the reaction, the mixture was washed with saturated sodium chloride solution (20 mL×3), dried over anhydrous sodium sulfate, filtered, and concentrated. The residue was purified by column chromatography to obtain **A17-1** (2.09 g).
Step b: In an ice bath, **A11-2** (0.90 g, 4.0 mmol) was added to a dichloromethane solution (30 mL) containing **A17-1** (1.73 g, 8.0 mmol). A dichloromethane solution (20 mL) of TEA (1.11 mL, 8.0 mmol) was then added dropwise over 15 minutes. After removing the ice bath, the reaction was stirred at room temperature for 5 h. Upon completion of the reaction, the mixture was washed sequentially with dilute hydrochloric acid (15 mL), water (15 mL), and saturated saline solution (15 mL). The organic phase was concentrated under reduced pressure, treated with a TFA/DCM mixed solution (1:1 v/v) to remove the Boc protecting group, and washed with purified water. The organic phase was dried over anhydrous sodium sulfate, filtered, concentrated, and the residue was purified by column chromatography to obtain **A17-2** (1.10 g).
Step c: **A17-2** (0.77 g, 2.0 mmol) and DIPEA (0.77 g, 6.0 mmol) were dissolved in 20 mL of dichloromethane, and **A16-1** (1.75 g, 5.0 mmol) was added with stirring. The reaction was carried out overnight at room temperature. Upon completion of the reaction, the mixture was poured into 40 mL of water and extracted three times with dichloromethane (20 mL×3). The organic phases were collected and combined, washed with saturated saline solution, dried over anhydrous sodium sulfate, filtered, and concentrated. The residue was purified by column chromatography to obtain cationic lipid **N17** (1.55 g). ¹H NMR (400 MHz, CDCl₃) δ: 4.07 (t, 4H, -C*H*₂OC(=O)-), 3.94 (q, 4H, -NHC(=S)NHC*H*₂CH₂SS-), 3.39 (br, 4H, >NCH₂C*H*₂NHC(=S)NH-), 2.98 (t, 4H, -NHC(=S)NHCH₂C*H*₂S-), 2.42 (t, 4H, >NC*H*₂CH₂NHC(=S)NH-), 2.35 (t, 4H, -CH₂CH₂C*H*₂N<), 2.29 (t, 4H, -OC(=O)C*H*₂-), 2.22 (s, 6H, >NC*H*₃), 1.65-1.53 (m, 12H, -CH₂C*H*₂CH₂N<, -C*H*₂CH₂OC(=O)CH₂C*H*₂-), 1.38-1.18 (m, 36H, -C*H*₂CH₃, -CH₂C*H*₂CH₂-), 0.88 (t, 6H, -CH₂C*H*₃). MS (ESI): m/z = 921.4 ([M+H]⁺).

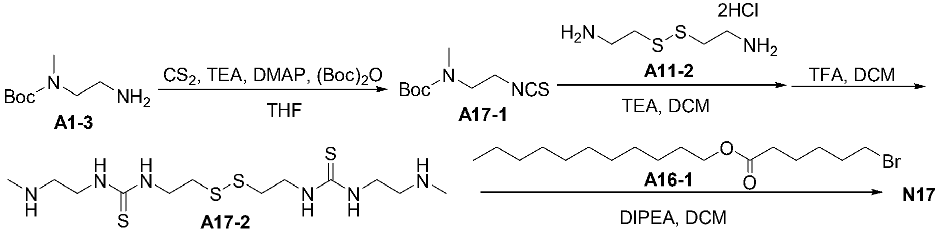

### Example 18: Preparation of Cationic Lipid N18

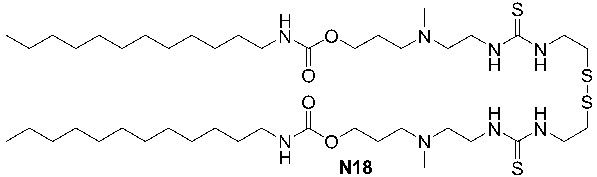

In cationic lipid **N18,** L₁ and L₂ are both -(CH₂)₂-NHC(=S)NH-(CH₂)₂-, X₁ and X₂ are both 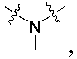 G₁ and G₂ are both linking bonds, L₃ and L₄ are both -NHC(=O)O-(CH₂)₃-, and R₁ and R₂ are both dodecyl groups.

Referring to the preparation method of cationic lipid **N2** in Example 2, compound **A1-4** was replaced with compound **A17-2,** with the rest conditions unchanged, to obtain cationic lipid **N18** (2.97 g). ¹H NMR (400 MHz, CDCl₃) δ: 4.22-4.18 (m, 4H, -NHC(=O)OC*H*₂CH₂CH₂N<), 3.92 (q, 4H, -NHC(=S)NHC*H*₂CH₂SS-), 3.36 (br, 4H, >NCH₂C*H*₂NHC(=S)NH-), 3.15-3.09 (m, 4H, -CH₂CH₂C*H*₂NHC(=O)O-), 2.96 (t, 4H, -NHC(=S)NHCH₂C*H*₂S-), 2.41 (t, 4H, >NC*H*₂CH₂NHC(=S)NH-), 2.35 (t, 4H, -CH₂CH₂C*H*₂N<), 2.23 (s, 6H, >NC*H*₃), 1.83-1.70 (m, 4H, -NHC(=O)OCH₂C*H*₂CH₂N<), 1.54-1.42 (m, 4H, -C*H*₂CH₂NHC(=O)O-), 1.37-1.19 (m, 36H, -C*H*₂CH₃, -CH₂C*H*₂CH₂-), 0.86 (t, 6H, -CH₂C*H*₃). MS (ESI): m/z = 923.4 ([M+H]⁺).

### Example 19: Preparation of Cationic Lipid N19

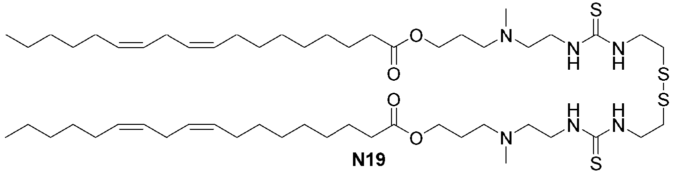

In cationic lipid **N19,** L₁ and L₂ are both -(CH₂)₂-NHC(=S)NH-(CH₂)₂-, X₁ and X₂ are both 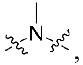 G₁ and G₂ are both linking bonds, L₃ and L₄ are both -C(=O)O-(CH₂)₃-, and R₁ and R₂ are both heptadeca-8,11-dienyl groups.

The preparation method is as follows:
Step a: **A17-2** (2.30 g, 6.0 mmol) and DIPEA (2.32 g, 18.0 mmol) were dissolved in 50 mL of dichloromethane, and **A2-1** (3.80 g, 15.0 mmol) was added under stirring. The reaction was carried out at room temperature overnight. After completion of the reaction, the mixture was poured into 50 mL of water and extracted three times with dichloromethane (50 mL×3). The organic phases were collected and combined, washed with saturated saline solution, and concentrated under reduced pressure. The residue was dissolved in 30 mL of anhydrous tetrahydrofuran, followed by the addition 1M TBAF/THF solution (30 mL). The reaction was stirred overnight to remove the TBS protection. Upon completion of the reaction, the mixture was concentrated under reduced pressure, and the residue was dissolved in 50 mL of dichloromethane. The solution was washed with saturated ammonium chloride solution, dried over anhydrous sodium sulfate, filtered, and concentrated. The residue was purified by column chromatography to obtain **A19-1** (2.14 g).
Step b: Under an argon atmosphere, a solution of **A19-1** (1.00 g, 2.0 mmol), **A7-1** (1.40 g, 5.0 mmol), and DMAP (0.12 g, 1.0 mmol) in 20 mL of dichloromethane was placed in an ice bath. A solution of DCC (1.24 g, 6.0 mmol) in 15 mL of dichloromethane was slowly added dropwise. The reaction mixture was then warmed to room temperature and stirred for 24 h. After completion of the reaction, the precipitate was removed by filtration, and the filtrate was concentrated under reduced pressure. The residue was purified by column chromatography to obtain cationic lipid **N19** (1.57 g). ¹H NMR (400 MHz, CDCl₃) δ: 5.44-5.31 (m, 8H, -CH₂-C*H*=C*H*-CH₂-), 4.06 (t, 4H, -C(=O)OC*H*₂CH₂CH₂N<), 3.94 (q, 4H, -NHC(=S)NHC*H*₂CH₂SS-), 3.38 (br, 4H, >NCH₂C*H*₂NHC(=S)NH-), 2.95 (t, 4H, -NHC(=S)NHCH₂C*H*₂S-), 2.80-2.73 (m, 4H, -CH=CH-C*H*₂-CH=CH-), 2.44 (t, 4H, >NCH₂C*H*₂NHC(=S)NH-), 2.36 (t, 4H, -CH₂CH₂C*H*₂N<), 2.30 (t, 4H, -C*H*₂C(=O)O-), 2.20 (s, 6H, >NC*H*₃), 2.04-1.97 (m, 8H, -C*H*₂-CH=CH-CH₂-CH=CH-C*H*₂-), 1.72-1.58 (m, 8H, -C(=O)OCH₂C*H*₂CH₂N<, -CH₂C*H*₂CH₂OC(=O)-), 1.36-1.18 (m, 28H, -C*H*₂CH₃, -CH₂C*H*₂CH₂-), 0.87 (t, 6H, -CH₂C*H*₃). MS (ESI): m/z = 1025.5 ([M+H]⁺).

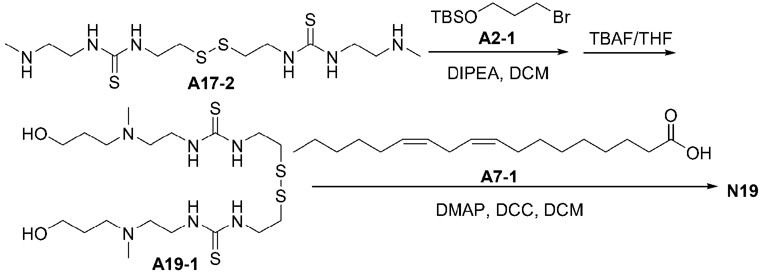

### Example 20: Preparation of Cationic Lipid N20

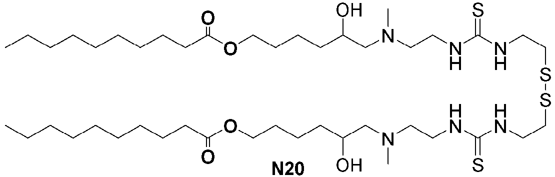

In cationic lipid **N20,** L₁ and L₂ are both -(CH₂)₂-NHC(=S)NH-(CH₂)₂-, X₁ and X₂ are both 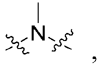 G₁ and G₂ are both linking bonds, L₃ and L₄ are both -C(=O)O-(CH₂)₄-CH(OH)CH₂-, and R₁ and R₂ are both nonyl groups.

Referring to the preparation method of cationic lipid **N14** in Example 14, compound **A1-4** was replaced with compound **A17-2,** with the rest conditions unchanged, to obtain cationic lipid **N20** (1.39 g). ¹H NMR (400 MHz, CDCl₃) δ: 4.05 (t, 4H, -C(=O)OC*H*₂-), 3.93 (q, 4H, -NHC(=S)NHC*H*₂CH₂SS-), 3.66-3.58 (m, 2H, -C*H*(OH)-), 3.37 (br, 4H, >NCH₂C*H*₂NHC(=S)NH-), 2.97 (t, 4H, -NHC(=S)NHCH₂C*H*₂S-), 2.53-2.48 (m, 4H, >CHC*H*₂N<), 2.40 (t, 4H, >NCH₂C*H*₂NHC(=S)NH-), 2.30 (t, 4H, -C*H*₂C(=O)O-), 2.20 (s, 6H, >NC*H*₃), 1.66-1.19 (m, 40H, -C*H*₂CH₃, -CH₂C*H*₂CH₂-), 0.86 (t, 6H, -CH₂C*H*₃). MS (ESI): m/z = 925.3 ([M+H]⁺).

### Example 21: Preparation of Cationic Lipid N21

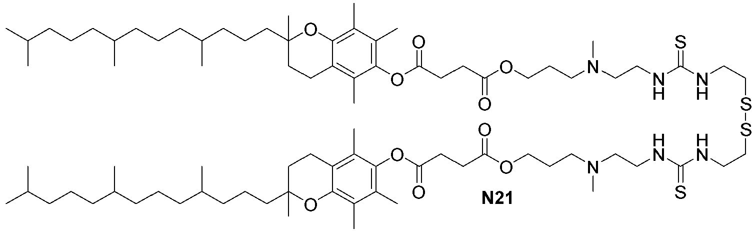

In cationic lipid **N21,** L₁ and L₂ are both -(CH₂)₂-NHC(=S)NH-(CH₂)₂-, X₁ and X₂ are both 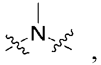 G₁ and G₂ are both linking bonds, L₃ and L₄ are both -OC(=O)-(CH₂)₂-C(=O)O-(CH₂)₃-, and R₁ and R₂ are both

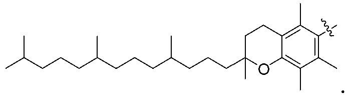

Referring to the preparation method of cationic lipid **N15** in Example 15, compound **A2-2** was replaced with compound **A19-1,** with the rest conditions unchanged, to obtain cationic lipid **N21** (1.94 g). ¹H NMR (400 MHz, CDCl₃) δ: 4.07 (t, 4H, -CH₂C(=O)OC*H*₂-), 3.92 (q, 4H, -NHC(=S)NHC*H*₂CH₂SS-), 3.41 (br, 4H, >NCH₂C*H*₂NHC(=S)NH-), 2.99 (t, 4H, -NHC(=S)NHCH₂C*H*₂S-), 2.66-2.59 (m, 12H, -OC(=O)C*H*₂C*H*₂C(=O)O-, -CH₂C*H*₂-Ar-), 2.41 (t, 4H, >NC*H*₂CH₂NHC(=S)NH-), 2.37 (t, 4H, -CH₂CH₂C*H*₂N<), 2.23 (s, 6H, >NC*H*₃), 2.07-1.99 (m, 18H, -Ar-C*H*₃), 1.87-1.73 (m, 4H, -C*H*₂CH₂Ar-), 1.71-1.00 (m, 52H, >C*H*(C*H*₂)₃C*H*<, >C*H*(C*H*₂)₃C<, >C(C*H*₃)O-), 0.89-0.86 (m, 24H, (C*H*₃)₂CH-, -CH₂CH(C*H*₃)CH₂-). MS (ESI): m/z = 1525.8 ([M+H]⁺).

### Example 22: Preparation of LNP-mRNA Pharmaceutical Compositions

Herein, LNP-mRNA pharmaceutical compositions containing Fluc-mRNA (LNP/Fluc-mRNA) were prepared, each comprising DSPC as the phospholipid, cholesterol as the steroid lipid, and PEG2k-DMG as the PEGylated lipid, with different cationic lipids.

The preparation method of LNP/Fluc-mRNA is as follows:
Step (1): A certain amount of cationic lipid, DSPC, cholesterol, and PEGylated lipid stock solutions were transferred, and the cationic lipid, DSPC, cholesterol, and PEGylated lipid were dissolved in ethanol at a molar ratio of 50:10:38:1.5 to obtain an ethanol phase solution (the specific lipid formulations for each group are shown in Table 1 of **Example 23;** the cationic lipid used in the control group **L0** is **R1,** while the cationic lipids used in experimental groups **L1-L21** are the disulfide-containing cationic lipids **N1-N21** of the present invention).

Wherein, cationic lipid **R1** was synthesized according to the method in CN103930398A. Compared with cationic lipid **N1** of the present invention, the difference lies in the absence of a degradable group between the ionizable tertiary amine group and the disulfide bond in **R1.** The structure of **R1** is as follows:

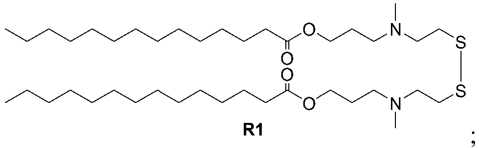

Step (2): Fluc-mRNA was added to a 10-50 mM citrate buffer (pH=4) to obtain an aqueous phase solution.

Step (3): The ethanol phase solution and the aqueous phase solution (1:3 v/v) were mixed to prepare LNP/Fluc-mRNA, and washed multiple times by ultrafiltration with DPBS to remove ethanol and free molecules. Finally, the solution was filtered through a 0.2 µm sterile filter for later use.

Referring to the above Steps (1-3), the molar ratio of the lipids was changed to cationic lipid : DSPC : cholesterol : PEGylated lipid = 48:9:42:1.5, with cationic lipid **N4** as the cationic lipid and the rest conditions unchanged, to prepare LNP/Fluc-mRNA **(L4-d).**

### Example 23: Bioactivity Assays of Lipid Pharmaceutical Compositions

### (1) Evaluation of Nanoparticle Size and Nucleic Acid Complexation Ability

Evaluation of nucleic acid complexation ability: Gel electrophoresis was used to examine the nucleic acid complexation ability of LNP/Fluc-mRNA. 0.8 g of agarose was dissolved in 40 mL of TAE solution, and heated in a microwave to completely dissolve the agarose particles. After cooling, 5 µL of the nucleic acid stain GelGreen was added to the cooled agarose gel, and the gel was placed into the gel tank and air-dried. After thoroughly mixing LNP/Fluc-mRNA with 2 µL of loading buffer, the mixture was loaded into the agarose gel wells. Electrophoresis was performed at 90 V for 10 min at room temperature. No free Fluc-mRNA was observed in any of the experimental and control groups, indicating that the cationic lipids containing a disulfide bond of the present invention exhibit excellent nucleic acid complexation ability.

Evaluation of encapsulation efficiency: LNP/Fluc-mRNA was subjected to ultracentrifugation (4°C, 60,000 rpm, 1 h) using an ultracentrifuge. The concentration of unencapsulated Fluc-mRNA in the supernatant was measured using a nucleic acid quantifier, and the encapsulation efficiency of LNP for Fluc-mRNA was calculated. The results are summarized in **Table 1.** The encapsulation efficiencies of the experimental groups were all above 86%, indicating that the LNPs of the present invention demonstrate relatively high encapsulation efficiencies for nucleic acid drugs.

Evaluation of particle size: According to the literature (Hassett et al., J. Controlled Release 2021, 335, 237-246), LNP formulations loaded with nucleic acid drugs exhibit better efficacy when the particle size ranges from 60 to 150 nm. Herein, the particle size of LNP/Fluc-mRNA was measured using dynamic light scattering (DLS). The measured sizes of LNP/Fluc-mRNA demonstrated relatively high uniformity, with PDI values all being less than 0.3. The experimental results showed that the particle size of the LNP/Fluc-mRNA prepared using the cationic lipid of the present invention ranged from 59 nm to 85 nm, which falls within the particle size range for achieving better drug efficacy.

**Table 1: Evaluation of Encapsulation Efficiency and Particle Size**

| **LNP/Fluc-mRNA** | **L0** | | | |
|---|---|---|---|---|
| Cationic Lipid | **R1** | | | |
| Encapsulation Efficiency /% | 90.0 | | | |
| Particle Size /nm | 66 | | | |

| **LNP/Fluc-mRNA** | **L1** | **L2** | **L3** | **L4** |
|---|---|---|---|---|
| Cationic Lipid | **N1** | **N2** | **N3** | **N4** |
| Encapsulation Efficiency /% | 91.7 | 96.6 | 92.0 | 97.6 |
| Particle Size /nm | 69 | 74 | 79 | 83 |

| **LNP/Fluc-mRNA** | **L4-d** | **L5** | **L6** | **L7** |
|---|---|---|---|---|
| Cationic Lipid | **N4** | **N5** | **N6** | **N7** |
| Encapsulation Efficiency /% | 96.5 | 88.6 | 89.1 | 93.3 |
| Particle Size /nm | 87 | 77 | 62 | 80 |

| **LNP/Fluc-mRNA** | **L8** | **L9** | **L10** | **L11** |
|---|---|---|---|---|
| Cationic Lipid | **N8** | **N9** | **N10** | **N11** |
| Encapsulation Efficiency /% | 91.9 | 94.0 | 98.5 | 91.1 |
| Particle Size /nm | 68 | 80 | 71 | 85 |

| **LNP/Fluc-mRNA** | **L12** | **L13** | **L14** | **L15** |
|---|---|---|---|---|
| Cationic Lipid | **N12** | **N13** | **N14** | **N15** |
| Encapsulation Efficiency /% | 88.1 | 92.8 | 88.3 | 91.9 |
| Particle Size /nm | 59 | 67 | 70 | 74 |

| **LNP/Fluc-mRNA** | **L16** | **L17** | **L18** | **L19** |
|---|---|---|---|---|
| Cationic Lipid | **N16** | **N17** | **N18** | **N19** |
| Encapsulation Efficiency /% | 94.6 | 90.0 | 91.3 | 95.2 |
| Particle Size /nm | 67 | 64 | 76 | 79 |

| **LNP/Fluc-mRNA** | **L20** | **L21** | | |
|---|---|---|---|---|
| Cationic Lipid | **N20** | **N21** | | |
| Encapsulation Efficiency /% | 92.0 | 89.9 | | |
| Particle Size /nm | 69 | 78 | | |

### (2) Evaluation of Stability in Serum

The above LNP/Fluc-mRNA formulations (control group **L0**, experimental groups **L1-L21** and **L1-d)** were respectively added to a culture medium containing 10% fetal bovine serum (FBS) and stirred at 37°C. Samples were taken at designated intervals to measure changes in the particle size of LNP/Fluc-mRNA. The stability in serum of the nucleic acid pharmaceutical formulations was analyzed based on the change in particle size. The experimental results showed that over a period of 7 days, the change in the particle size of the control group was 3%, while the change in the particle size of the experimental groups ranged from 2% to 8%. These results indicate that, in the present invention, the appropriate introduction of more degradable groups into cationic lipids containing a disulfide bond does not significantly affect the stability of the prepared LNP-nucleic acid pharmaceutical compositions in serum.

### (3) Evaluation of Cytotoxicity

DMEM high glucose complete medium (containing 10% FBS) was prepared. The samples **(L1-L21** and **L1-d)** were each prepared into working solutions at concentrations of 0.1, 0.15, 0.2, 0.25, and 0.3 µg/100 µL using the complete medium, and stored for later use. Log-phase 293T cells were seeded into a 96-well plate at a density of 7×10³ cells/well, 100 µL/well. Three replicate wells were set for each of the control group and the sample groups. After incubating at 5% CO₂, 37°C in a temperature-controlled incubator for 24 h, the original medium was retained, the complete medium was added to the control group at 100 µL/well, and the working solutions were added to the sample groups at 100 µL/well. After incubation for another 24 h, the medium containing 10% CCK-8 was added at 100 µL/well, followed by incubation for 2 h at 5% CO₂, 37°C in a temperature-controlled incubator. The absorbance at 450 nm was measured using a microplate reader. Relative cell viability was calculated using the following formula: 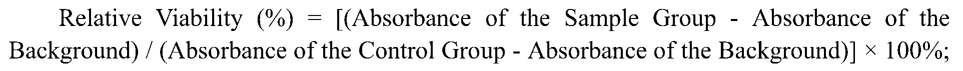 Relative Viability (%) = [(Absorbance of the Sample Group - Absorbance of the Background) / (Absorbance of the Control Group - Absorbance of the Background)] × 100%;wherein, the absorbance of the background refers to the absorbance with only CCK-8 reagent and medium.

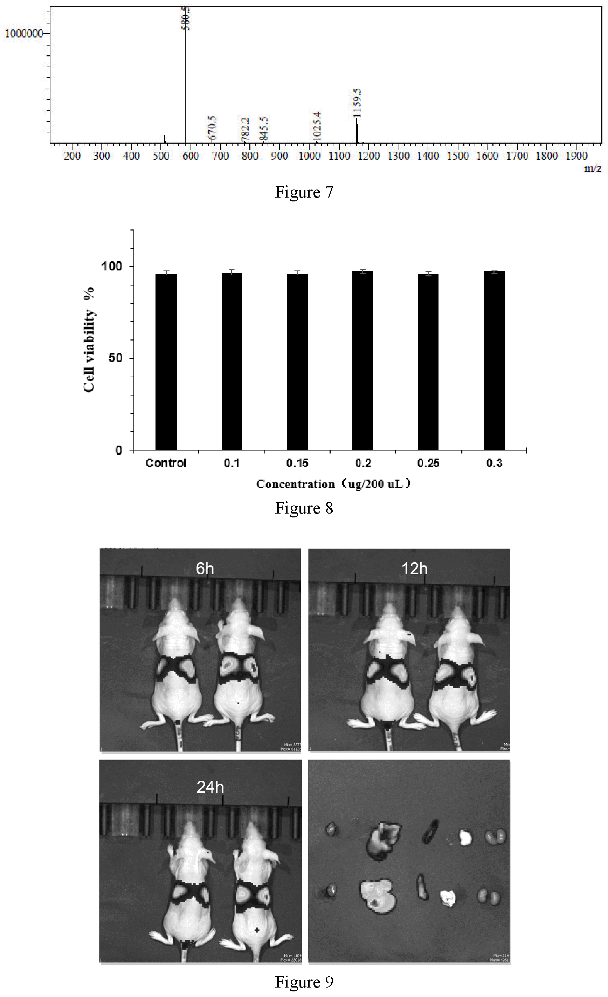

The experimental results demonstrated that the lipid nanoparticles prepared using the cationic lipids of the present invention exhibited no significant cytotoxicity across five concentration gradients, with cell viability exceeding 95% in all cases. Specifically, the results for sample group **L4** are shown in FIG. 8.

### (4) Evaluation of Transfection Efficiency

In the structural design of the present invention, additional degradable groups are introduced at suitable positions within the cationic lipids containing a disulfide bond; specifically, carbamate, thiocarbamate, urea, or thiourea groups are introduced at the position between the ionizable tertiary amine group and the disulfide bond, which significantly enhances the delivery efficiency of LNPs for nucleic acid drugs, achieving more efficient transfection.

To evaluate the mRNA transfection efficiency of various LNP/Fluc-mRNA compositions prepared in **Example 22** of the present invention at the cellular level, luciferase bioluminescence was used for testing. The LNP/Fluc-mRNA composition formulations were dissolved in culture media to prepare the required doses. HeLa cells were used as the cell model. Cell suspensions of 100 µL/well were seeded into a black-edged, clear-bottom 96-well plate at a density of 6000 cells/well. After seeding, the plates were incubated in a cell incubator for 24 h. Then, the formulations were administered at a dose of 0.2 µg mRNA per well. Group L0 was set as the positive control group, and groups L1-L21 were set as the experimental groups. A blank control group was also included, with an equivalent dose of free Fluc-mRNA added. After 24 h of transfection, the old culture media were removed and replaced with fresh culture media containing D-luciferin sodium (1.5 mg/mL). After 5 minutes of incubation, bioluminescence was measured using a microplate reader. A stronger luminescence indicated a higher amount of Fluc-mRNA transported into the cytoplasm and translated into the corresponding fluorescent protein. The results showed that the transfection efficiencies of the experimental groups were all superior to that of the positive control group.

**Table 2: Results of Cell Transfection Assay**

| **Entry** | **Relative Fluorescence Intensity** | **Entry** | **Relative Fluorescence Intensity** | **Entry** | **Relative Fluorescence Intensity** |
|---|---|---|---|---|---|
| Blank | 1 | L1 | 3.1 | L11 | 2.8 |
| L0 | 2.4 | L2 | 3.3 | L12 | 3.5 |
| | | L3 | 3.1 | L13 | 3.0 |
| | | L4 | 4.4 | L14 | 3.0 |
| | | L4-d | 4.0 | L15 | 3.8 |
| | | L5 | 4.1 | L16 | 2.7 |
| | | L6 | 3.4 | L17 | 3.1 |
| | | L7 | 2.9 | L18 | 3.5 |
| | | L8 | 3.4 | L19 | 3.2 |
| | | L9 | 3.0 | L20 | 3.3 |
| | | L10 | 3.7 | L21 | 3.8 |

### (5) Evaluation of In Vivo Transfection Efficiency

Lipid nanoparticle **L4** was delivered to 6-8-week-old female BALB/c mice via tail vein injection at a dose of 10 µg per mouse. Small animal in vivo fluorescence imaging was performed at 6, 12, and 24 h post-administration. After imaging at the final time point, the mice were euthanized, and the major organs (heart, liver, spleen, lungs, and kidneys) were imaged. Ten to fifteen minutes prior to imaging, 0.2 mL of D-luciferin sodium (15 mg/mL) was injected intraperitoneally. The experimental results (FIG. 9) demonstrated that the pharmaceutical lipid nanoparticle prepared with the cationic lipid of the present invention enables highly efficient in vivo delivery of nucleic acid drugs, and the delivered LNP-mRNA pharmaceutical composition was primarily distributed in the liver and spleen.

## Claims

1. A cationic lipid containing a disulfide bond, the structure of which is represented by general formula (1): 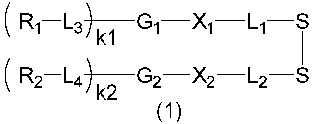
or salt, tautomer, stereoisomer, isotopically substituted derivative, or solvate thereof;
wherein, -S-S- is a disulfide bond;
L₁ and L₂ are each independently -(CH₂)ₜ-L_{d}-(CH₂)ₜ- or -(CH₂)ₜ-L_{d}-(CH₂)ₜ-L_{d}-(CH₂)ₜ-, wherein each t is independently an integer from 1 to 4;
each L_{d} is independently -NHC(=Y₁)Y₂- or -Y₂C(=Y₁)NH-, wherein Y₁ and Y₂ are each independently O, S, or NH;
X₁ and X₂ are each independently an acyclic divalent linking group containing a tertiary amine group;
G₁ and G₂ are each independently a linking bond or a trivalent branching group;
when G₁ is a linking bond, k1 is 1; when G₁ is a trivalent branching group, k1 is 2;
when G₂ is a linking bond, k1 is 1; when G₂ is a trivalent branching group, k2 is 2;
L₃ and L₄ are, at each occurrence, each independently selected from the group consisting of a linking bond, -(CH₂)_{q}-, Z, P₀, 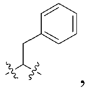 and combinations thereof; wherein, q is an integer from 1 to 10, Z is selected from the group consisting of -O-, -S-, -OC(=O)-, -C(=O)O-, -OC(=O)O-, -C(=O)-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, -C(R_{c})=N-NR_{c}-, -NR_{c}-N=C(R_{c})-, and -CH(OH)-, R_{c} is, at each occurrence, independently H or a methyl group; P₀ is 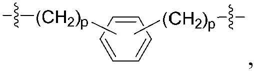 and p is, at each occurrence, independently 0 or 1;
R₁ and R₂ are each independently a substituted or unsubstituted C₅₋₃₀ hydrocarbon group or C₅₋₃₀ hydrocarbon derivative residue.

2. The cationic lipid containing a disulfide bond according to claim **1,** wherein L₁ and L₂ are identical or different, each independently being -(CH₂)ₜ-L_{d}-(CH₂)ₜ-;
preferably, L₁ and L₂ are identical and selected from the group consisting of -(CH₂)ₜ-OC(=O)NH-(CH₂)ₜ-, -(CH₂)ₜ-NHC(=O)O-(CH₂)ₜ-, and -(CH₂)ₜ-NHC(=S)NH-(CH₂)ₜ-;
more preferably, L₁ and L₂ are identical and selected from the group consisting of -(CH₂)₂-NHC(=O)O-(CH₂)₂-, -(CH₂)₂-OC(=O)NH-(CH₂)₂-, and -(CH₂)₂-NHC(=S)NH-(CH₂)₂-.

3. The cationic lipid containing a disulfide bond according to claim **1,** wherein X₁ and X₂ are each independently 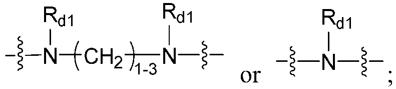 wherein, R_{d1} is, at each occurrence, independently a substituted or unsubstituted C₁₋₆ alkyl group, preferably a hydroxyl-substituted or unsubstituted C₁₋₆ alkyl group; preferably, X₁ and X₂ are identical and selected from the group consisting of

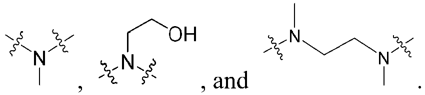

4. The cationic lipid containing a disulfide bond according to claim **1,** wherein R₁ and R₂ are identical or different, each independently being a C₅₋₃₀ aliphatic hydrocarbon group, a C₅₋₃₀ aliphatic hydrocarbon derivative residue, a C₇₋₃₀ aromatic hydrocarbon group, or a C₇₋₃₀ aromatic hydrocarbon derivative residue; preferably, R₁ and R₂ are each independently selected from the group consisting of R_{L}, R_{B}, Rᵣ, and R_{A}; more preferably, R₁ and R₂ are each independently R_{L}, R_{B}, or R_{A};
R_{L} is a linear C₅₋₃₀ aliphatic hydrocarbon group containing 0 to 4 carbon-carbon double bonds or carbon-carbon triple bonds; preferably, R_{L} is any one or a substituted form of 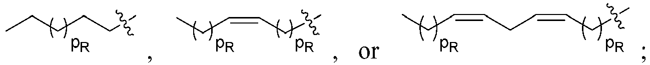 wherein, p_{R} is, at each occurrence, independently an integer from 1 to 15; the substituted form contains 1 to 4 substituents; the substituent is, at each occurrence, a linear or branched C₁₋₄ alkyl group, a hydroxyl group, or a halogen atom, preferably a methyl group or a hydroxyl group; more preferably, R_{L} is selected from the group consisting of the following structures: 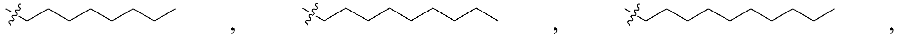 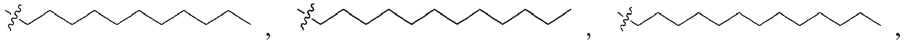 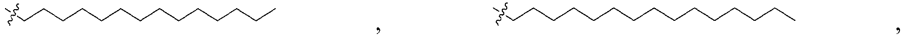 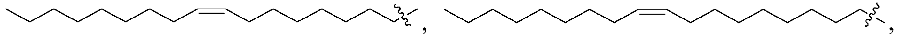 and 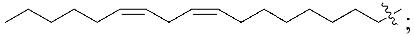
R_{B} is a branched C₅₋₃₀ aliphatic hydrocarbon group or C₅₋₃₀ aliphatic hydrocarbon derivative residue, selected from the group consisting of the following structures: 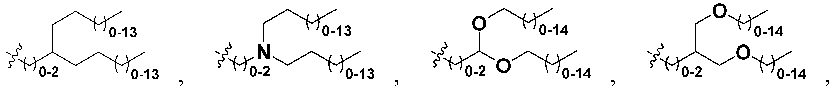 and 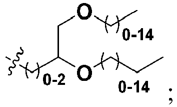
preferably, R_{B} is selected from the group consisting of the following structures: 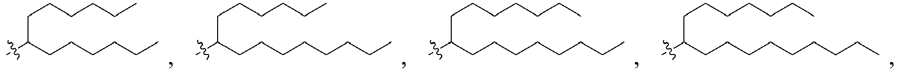 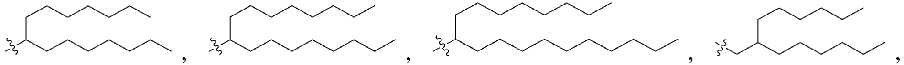 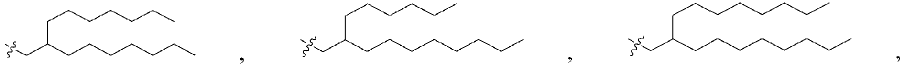 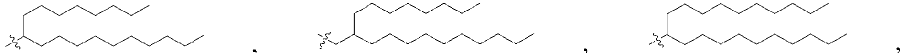 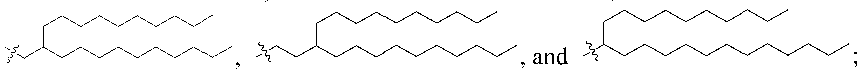
Rᵣ is a ring-containing C₅₋₃₀ aliphatic hydrocarbon group, preferably 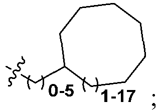
R_{A} is a C₇₋₃₀ aromatic hydrocarbon group or C₇₋₃₀ aromatic hydrocarbon derivative residue, preferably

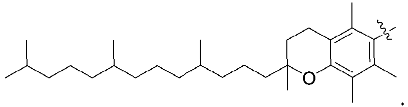

5. The cationic lipid containing a disulfide bond according to claim **1,** wherein L₃ and L₄ are each independently selected from the group consisting of a linking bond, -Z-(CH₂)_{q}-, -Z-(CH₂)_{q}-Z-(CH₂)_{q}-, -Z-P₀-(CH₂)_{q}-, -Z-P₀-Z-(CH₂)_{q}-, and -P₀-(CH₂)_{q}-, and the left end is connected to R₁ or R₂; wherein, the structures of any two Z are identical or different from each other.

6. The cationic lipid containing a disulfide bond according to claim **5,** wherein Z is, at each occurrence, independently selected from the group consisting of -O-, -C(=O)-, -OC(=O)-, -C(=O)O-, -NHC(=O)-, -C(=O)NH-, -OC(=O)O-, -OC(=O)NH-, -NHC(=O)O-, and -CH(OH)-.

7. The cationic lipid containing a disulfide bond according to claim **5,** wherein, at least one of L₃ and L₄ comprises P₀ and is preferably -Z-P₀-(CH₂)_{q}-*, -Z-P₀-Z-(CH₂)_{q}-*, or -P₀-(CH₂)_{q}-*, wherein the * end is connected to G₁ or G₂; preferably, P₀ is

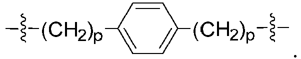

8. The cationic lipid containing a disulfide bond according to claim **1,** wherein L₃ and L₄ are identical and belong to any of the following cases:
Case (1): L₃ and L₄ are both linking bonds;
Case (2): L₃ and L₄ are both -C(=O)O-(CH₂)_{q}- or -OC(=O)-(CH₂)_{q}-;
Case (3): L₃ and L₄ are both -C(=O)NH-(CH₂)_{q}- or -NHC(=O)-(CH₂)_{q}-;
Case (4): L₃ and L₄ are both -OC(=O)NH-(CH₂)_{q}- or -NHC(=O)O-(CH₂)_{q}-;
Case (5): L₃ and L₄ are both -OC(=O)-(CH₂)_{q}-C(=O)O-(CH₂)_{q}-, -OC(=O)-(CH₂)_{q}-OC(=O)-(CH₂)_{q}-, -C(=O)O-(CH₂)_{q}-C(=O)O-(CH₂)_{q}-, -C(=O)O-(CH₂)_{q}-OC(=O)-(CH₂)_{q}-, -OC(=O)-(CH₂)_{q}-C(=O)NH-(CH₂)_{q}-, -OC(=O)-(CH₂)_{q}-NHC(=O)-(CH₂)_{q}-, -C(=O)O-(CH₂)_{q}-C(=O)NH-(CH₂)_{q}-, -C(=O)O-(CH₂)_{q}-NHC(=O)-(CH₂)_{q}-, -OC(=O)-(CH₂)_{q}-CH(OH)-(CH₂)_{q}-, -C(=O)O-(CH₂)_{q}-CH(OH)-(CH₂)_{q}-, -NHC(=O)-(CH₂)_{q}-CH(OH)-(CH₂)_{q}-, or -C(=O)NH-(CH₂)_{q}-CH(OH)-(CH₂)_{q}-;
Case (6): L₃ and L₄ are both -OC(=O)-P₀-(CH₂)_{q}- or -C(=O)O-P₀-C(=O)O-(CH₂)_{q}-, preferably 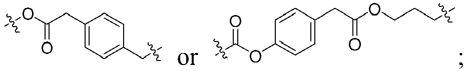
in any of the above cases, the left end of L₃ is connected to R₁, and the left end of L₄ is connected to R₂.

9. The cationic lipid containing a disulfide bond according to claim **1,** wherein the structure of the cationic lipid is represented by general formula (2):

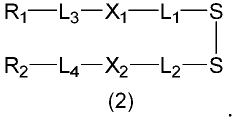

10. The cationic lipid containing a disulfide bond according to claim **1,** wherein the structure of the cationic lipid is represented by general formula (3): 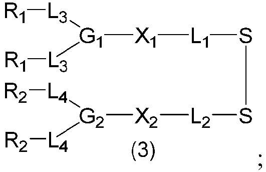
wherein, two L₃ are identical, two R₁ are identical, two L₄ are identical, and two R₂ are identical; G₁ and G₂ are each independently represented by 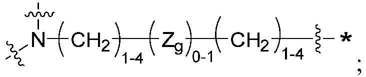 wherein, the * end is connected to X₁ or X₂, the other two ends are connected to L₃ or L₄, and Z_{g} is selected from the group consisting of -O-, -C(=O)-, -OC(=O)-, -C(=O)O-, -NHC(=O)-, -C(=O)NH-, -OC(=O)O-, -OC(=O)NH-, and -NHC(=O)O-;
preferably, G₁ and G₂ are each independently selected from the group consisting of the following structures: 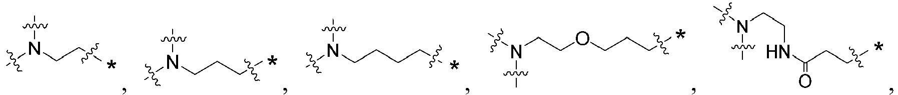 and 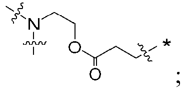 more preferably, G₁ and G₂ are both 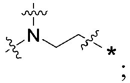
preferably, all L₃ and L₄ are -Z-(CH₂)_{q}-.

11. The cationic lipid containing a disulfide bond according to claim **1,** wherein the structure of the cationic lipid is represented by any of the following general formulas: 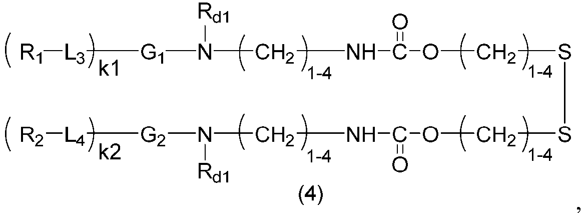 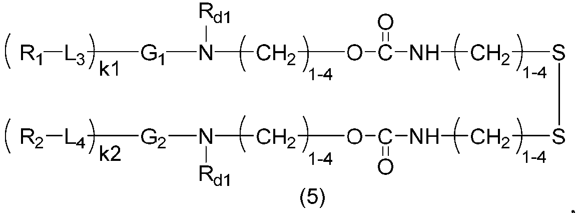 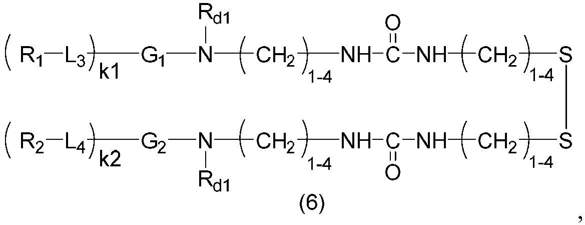 and

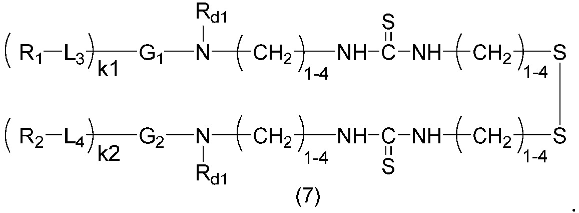

12. The cationic lipid containing a disulfide bond according to claim **1,** wherein the structure of the cationic lipid is selected from the group consisting of **N1** to **N21:** 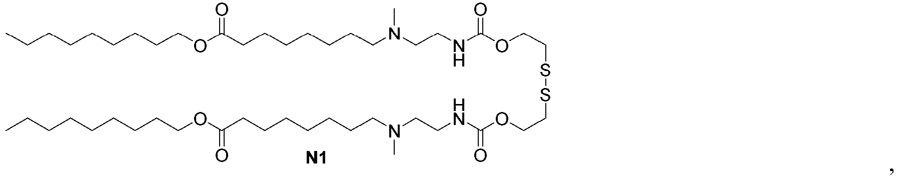 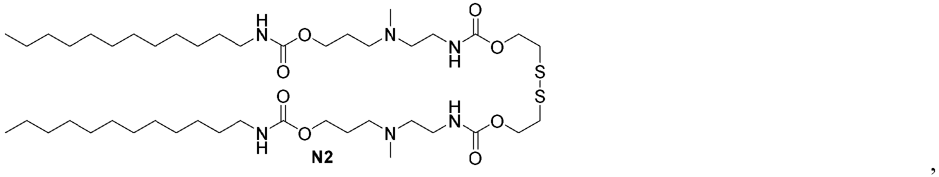 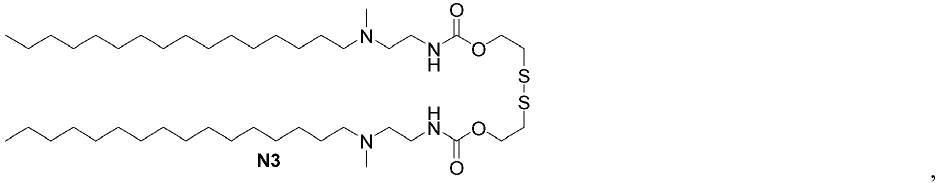 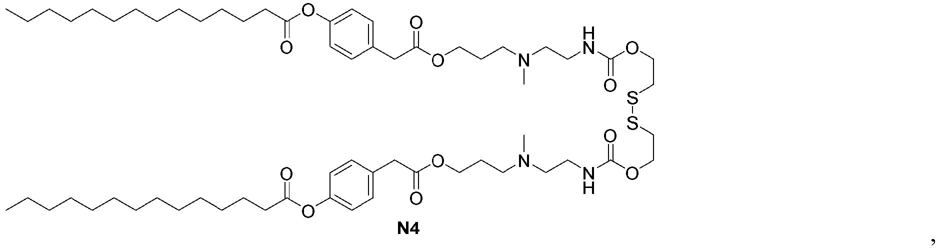 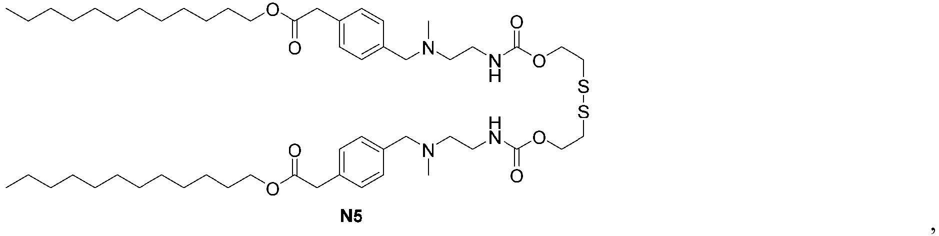 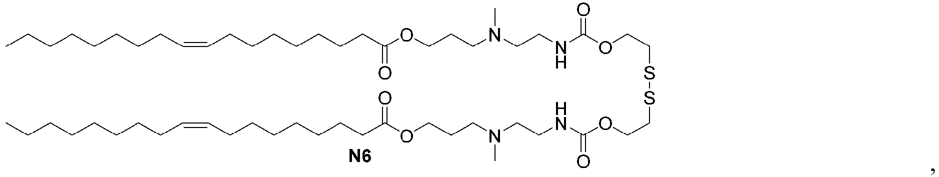 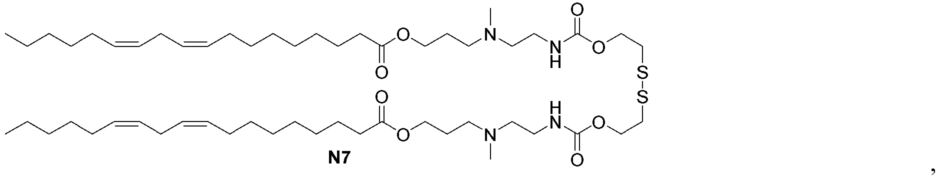 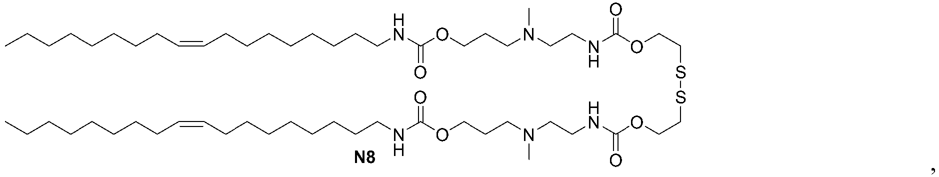 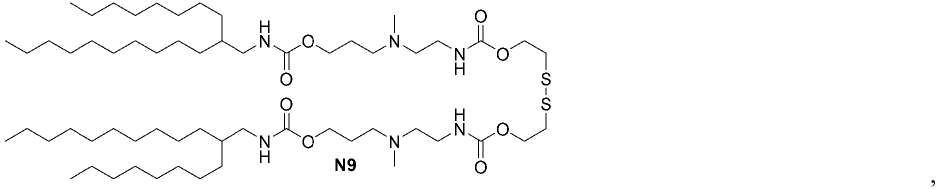 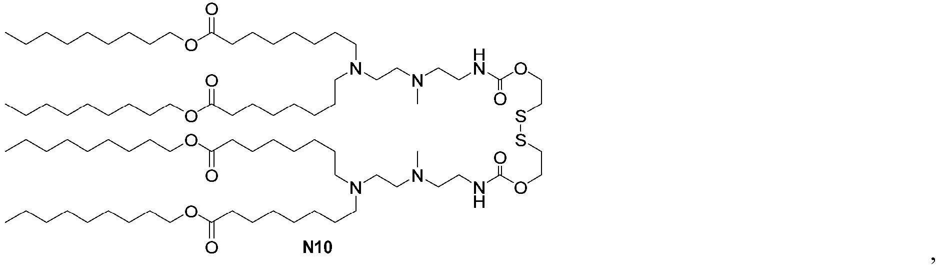 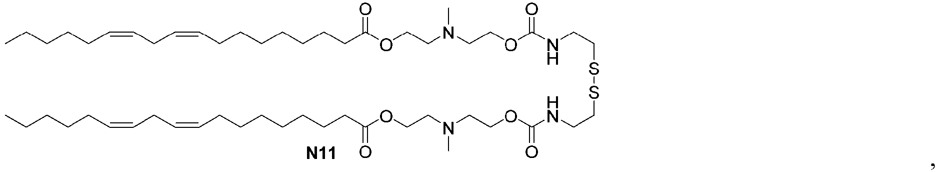 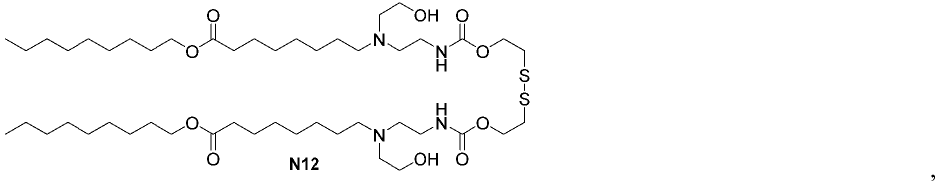 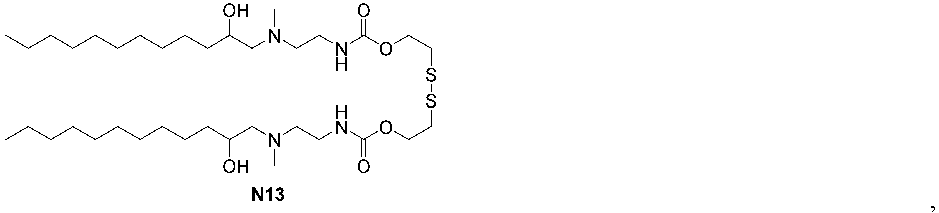 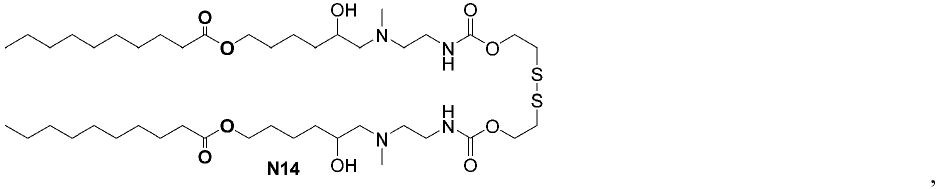 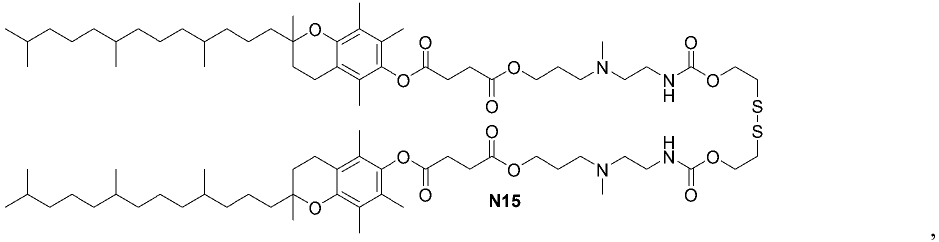 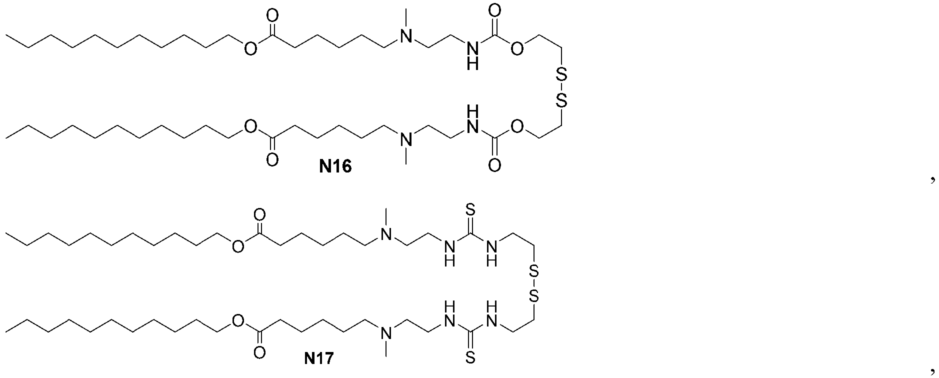 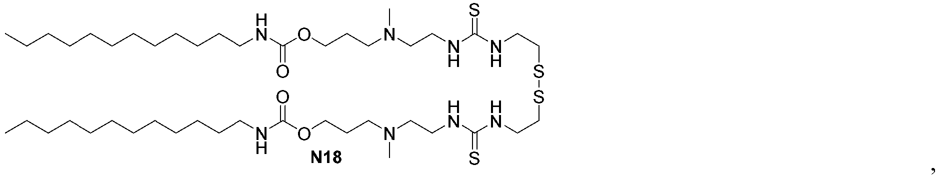 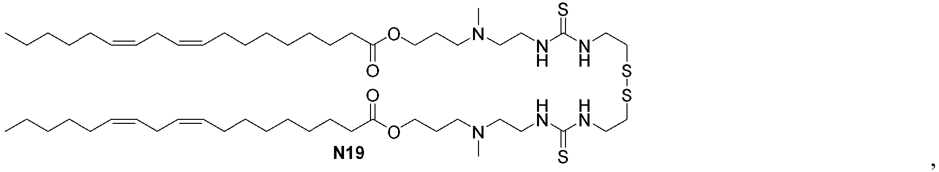 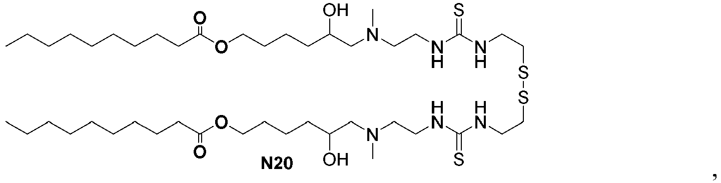 and

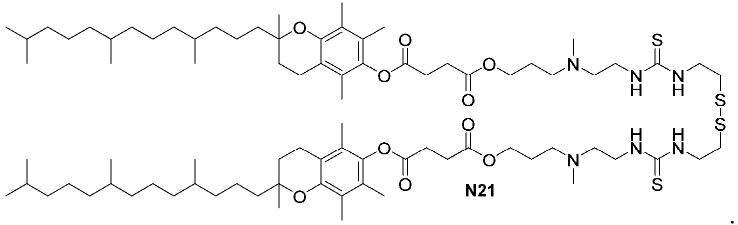

13. A lipid composition, which contains a cationic lipid containing a disulfide bond of any one of claims **1** to **12.**

14. The lipid composition according to claim **13,** wherein the lipid composition further contains phospholipid; or, the lipid composition further contains steroid lipid; or, the lipid composition further contains PEGylated lipid; or, the lipid composition further contains phospholipid and steroid lipid; or, the lipid composition further contains phospholipid and PEGylated lipid; or, the lipid composition further contains steroid lipid and PEGylated lipid; or, the lipid composition further contains phospholipid, steroid lipid, and PEGylated lipid; or, the lipid composition further contains phospholipid, steroid lipid, PEGylated lipid, and another type of cationic lipid; or, the lipid composition further contains phospholipid, steroid lipid, PEGylated lipid, and anionic lipid.

15. The lipid composition according to claim **14,** wherein the phospholipid is selected from the group consisting of 1,2-dilinoleoyl-sn-glycero-3-phosphocholine, 1,2-dimyristoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-palmitoyl-2-oleoyl-sn-glycero-3-phosphocholine, 1,2-di-O-octadecyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyl oleoyl phosphatidylethanolamine, distearoyl phosphatidylethanolamine, dipalmitoyl phosphatidylethanolamine, dimyristoyl phosphatidylethanolamine, 1-stearoyl-2-oleoyl-sn-glycero-3-phosphoethanolamin, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyl oleoyl phosphatidylcholine, lysophosphatidylcholine, lysophosphatidylethanolamine, and combinations thereof.

16. The lipid composition according to claim **14,** wherein the steroid lipid is selected from the group consisting of cholesterol, coprostanol, sitosterol, ergosterol, campesterol, stigmasterol, brassicasterol, tomatidine, ursolic acid, α-tocopherol, and combinations thereof.

17. The lipid composition according to claim **14,** wherein the PEGylated lipid is selected from the group consisting of non-targeting PEGylated lipid, targeting PEGylated lipid, and combinations thereof; preferably, the non-targeting PEGylated lipid is selected from the group consisting of polyethylene glycol-dipalmitoyl phosphatidylcholine, polyethylene glycol-dimyristoyl glycerol, polyethylene glycol-distearoyl phosphatidylethanolamine, polyethylene glycol-dioleoyl phosphatidylethanolamine, polyethylene glycol-cholesterol, polyethylene glycol-diacylglycerol, polyethylene glycol-dialkoxypropyl, and combinations thereof; more preferably, the non-targeting PEGylated lipid is selected from the group consisting of polyethylene glycol 500-dipalmitoyl phosphatidylcholine, polyethylene glycol 2000-dipalmitoyl phosphatidylcholine, polyethylene glycol 500-distearoyl phosphatidylethanolamine, polyethylene glycol 2000-distearoyl phosphatidylethanolamine, polyethylene glycol 500-dioleoyl phosphatidylethanolamine, polyethylene glycol 2000-dioleoyl phosphatidylethanolamine, polyethylene glycol 500-dimyristoyl glycerol, polyethylene glycol 2000-dimyristoyl glycerol, and combinations thereof; preferably, the targeting PEGylated lipid is PEGylated lipid modified with folic acid or N-acetylgalactosamine.

18. The lipid composition according to claim **14,** wherein the other type of cationic lipid is selected from the group consisting of 1,2-dioleoyl-3-trimethylammonium-propane (methyl sulfate salt), 1,2-dioctadeceneoxy-3-trimethylammonium propane chloride, 1-[2-(oleoyloxy)ethyl]-2-oleyl-3-(2-hydroxyethyl)imidazolinium chloride, 1,2-dioleyloxy-3-dimethylamino-propane, 2,3-di(tetradecanoyloxy)propyl-trimethylazanium chloride, didecyldimethylammonium chloride, didecyldimethylammonium bromide, *N,N-d*ioleyl-*N,N*-dimethylammonium chloride, N-(4-carboxybenzyl)-N,N-dimethyl-2,3-bis(oleoyloxy)propan-1-aminium, 3,6-bis{4-[bis(2-hydroxydodecyl)amino]butyl}piperazine-2,5-dione, 1,1'-((2-(4-(2-((2-(bis(2-hydroxydodecyl)amino)ethyl)(2-hydroxydodecyl)amino)ethyl)pipera zin-1-yl)ethyl)azanediyl)bis(dodecan-2-ol), dilinoleylmethyl 4-(*N,N*-dimethylamino)butanoate, 1,2-dimyristoyl-sn-glycero-3-ethylphosphocholine, ((4-hydroxybutyl)azanediyl)bis(hexane-6,1-diyl)bis(2-hexyldecanoate), heptadecan-9-yl 8-((2-hydroxyethyl) (6-oxo-6-(undecyloxy) hexyl) amino) octanoate), and ((2-(2-hydroxyethoxy)ethyl)azanediyl)bis(hexane-6,1-diyl)bis(2-hexyldecanoate).

19. The lipid composition according to claim **14,** wherein the anionic lipid is selected from the group consisting of 1,2-dioleoyl-sn-glycero-3-phosphate sodium salt, 1,2-dimyristoyl-sn-glycero-3-phosphate sodium salt, bis(monooleoylglycero)phosphate ammonium salt, and cardiolipin.

20. The lipid composition according to claim **14,** wherein,
the molar percentage of PEGylated lipid in the total lipid is from 0.5% to 5%, preferably from 1% to 3%, more preferably 1.5%, 1.6%, 1.7%, 1.8%, or 1.9%;
the molar percentage of cationic lipid in the total lipid is from 30% to 65%, preferably 35%, 40%, 45%, 46%, 47%, 48%, 49%, 50%, or 55%;
the molar percentage of phospholipid in the total lipid is from 7.5% to 13%, preferably 8%, 9%, 10%, 11%, or 12%;
and the molar percentage of steroid lipid in the total lipid is from 35% to 50%, preferably 38%, 39%, 40%, 41%, 42%, 43%, 44%, 45%, 46%, 47%, 48%, 49%, or 50%.

21. A lipid pharmaceutical composition containing a lipid composition of any one of claims **13** to **20** and a drug, wherein the drug is selected from the group consisting of nucleic acid drugs, small molecule drugs, peptide drugs, and protein drugs.

22. The lipid pharmaceutical composition according to claim **21,** wherein the drug is a nucleic acid drug selected from the group consisting of DNA, RNA, antisense nucleic acid, plasmid, interfering nucleic acid, aptamer, antagomir, and ribozyme; wherein, the RNA is selected from the group consisting of mRNA, saRNA, circRNA, miRNA, and siRNA, and is preferably mRNA, miRNA, or siRNA.

23. The lipid pharmaceutical composition according to claim **21,** wherein the lipid pharmaceutical composition is used as a medicine, selected from the group consisting of the following: antineoplastics, anti-infectives, and vaccines; wherein, the anti-infectives are selected from the group consisting of antiparasitics, antibiotics, antifungals, and antivirals.

24. The lipid pharmaceutical composition according to claim **21,** wherein the lipid pharmaceutical composition is an LNP pharmaceutical composition, an LPP pharmaceutical composition, or a PNP pharmaceutical composition, preferably an LNP pharmaceutical composition, more preferably an LNP-nucleic acid pharmaceutical composition, and most preferably an LNP-mRNA pharmaceutical composition.

25. A lipid pharmaceutical composition formulation which contains a lipid pharmaceutical composition of any one of claims **21** to **24** and a working solution; wherein, the working solution is a pharmaceutically acceptable diluent or excipient, preferably selected from the group consisting of deionized water, ultrapure water, phosphate buffer, and saline, more preferably phosphate buffer or saline, and most preferably saline.
